# EUROPEAN PATENT SPECIFICATION

(11) **EP 2 941 488 B1**
(45) Date of publication and mention of the grant of the patent: **22.03.2023**
(21) Application number: 13828994.7
(22) Date of filing: 30.12.2013
(51) Int. Cl.: C12N 15/82, A01H 3/04, A01H 5/10

(54) **METHODS OF INTRODUCING DSRNA TO PLANT SEEDS FOR MODULATING GENE EXPRESSION**
VERFAHREN ZUR EINFÜGUNG VON DSRNA IN PFLANZENSAMEN ZUR MODULATION DER GENEXPRESSION
PROCÉDÉS D'INTRODUCTION D'ARNDS DANS DES GRAINES DE PLANTE POUR MODULER L'EXPRESSION DES GÈNES

(30) Priority: 01.01.2013 US 201361748095 P; 01.01.2013 US 201361748094 P; 01.01.2013 US 201361748101 P; 01.01.2013 US 201361748099 P; 23.04.2013 US 201361814888 P; 23.04.2013 US 201361814892 P; 23.04.2013 US 201361814899 P; 23.04.2013 US 201361814890 P; 26.11.2013 US 201361908965 P; 26.11.2013 US 201361908855 P
(43) Date of publication of application: 11.11.2015
(73) Proprietor: Monsanto Technology LLC, Saint Louis, MO 63167 (US)
(72) Inventor: AVNIEL, Amir, 6219207 Tel-Aviv (IL); LIDOR-NILI, Efrat, 7405492 Nes Ziona (IL); MAOR, Rudy, 7666206 Rechovot (IL); MEIR, Ofir, 7688500 Doar-Na Emek Soreq (IL); NOIVIRT-BRIK, Orly, 5339711 Givataim (IL)
(74) Representative: BIP Patents
(86) International application number: PCT/IL2013/051085
(87) International publication number: WO 2014/106838

(56) References cited:
- EP-A1- 1 416 049
- WO-A1-99/32619
- WO-A1-2007/039454
- WO-A1-2008/148223
- WO-A1-2011/001434
- WO-A1-2011/112570
- WO-A1-2013/025670
- WO-A1-2013/175480
- WO-A2-2005/110068
- WO-A2-2008/007100
- WO-A2-2012/092580
- UNNAMALAI N ET AL: "Cationic oligopeptide-mediated delivery of dsRNA for post-transcriptional gene silencing in plant cells", FEBS LETTERS, ELSEVIER, AMSTERDAM, NL, vol. 566, no. 1-3, 21 May 2004 (2004-05-21), pages 307-310, XP004509368, ISSN: 0014-5793, DOI: 10.1016/J.FEBSLET.2004.04.018

## Description

### FIELD OF THE DISCLOSURE

Methods and compositions for improving plant resistance to insect pests are provided.

### BACKGROUND

With a growing world population, increasing demand for food, fuel and fiber, and a changing climate, agriculture faces unprecedented challenges. Development of plants with improved traits is highly desirable, with some of the major traits that are of major interest to farmers and seed companies include improved abiotic stress tolerance, fertilizer use efficiency, disease resistance, yield and more.

Plant trait improvement is typically performed by either genetic engineering or classical breeding. New methods for trait improvement through specific gene alteration are highly desirable. These include methods for over-expression of genes or gene silencing. A powerful technique for sequence-specific gene silencing is through RNA interference (RNAi). First discovered in the nematode C. *elegans* (Fire et al. 1998, Nature, 391:806-811), RNAi is a mechanism in which expression of an individual gene can be specifically silenced by introducing a double-stranded RNA (dsRNA) that is homologous to the selected gene into cells. Inside the cell, dsRNA molecules are cut into shorter fragments of 21-27 nucleotides by an RNase III-related enzyme (Dicer). These fragments, called small interfering RNAs (siRNAs), get incorporated into the RNA-induced silencing complex (RISC). After additional processing, the siRNAs are transformed into single-stranded RNAs that act as guide sequences to eventually cleave target messenger RNAs. By using RNAi to specifically silence relevant target genes, one can alter basic traits of an organism. Specifically for plants, it holds incredible potential for modifications that may lead to increased stress resistance and better crop yield.

In plants, RNAi is typically performed by producing transgenic plants that over-express a DNA fragment that is transcribed to produce a dsRNA. This dsRNA is then processed into siRNAs that mediate the cleavage and silencing of target genes.

The major technical limitation for this technology is that many important plant crop species are difficult or impossible to transform, precluding the constitutive expression of constructs directing production of dsRNA. Moreover, questions concerning the potential ecological impact of virus-resistant transgenic plants have so far significantly limited their use (Tepfer, 2002, Annu. Rev. Phytopathol. 40, 467-491). An additional hurdle for obtaining transgenic plants is attributed to the difficulty of having the transformation and regeneration events occur in the same cell types.

Therefore the development of a method for obtaining transformed seeds which is independent of the methods inherent to tissue culture procedures is at the cutting edge of plant molecular biology research.

WO 2007/039454 is in the field of genetics, especially plant genetics, and provides agents capable of controlling gene expression. More specifically it relates to methods for engineering ta-siRNA primary transcripts in order to target gene-of-interest (GOI) and control their expression. It further provides for a method for modulating transgenic expression by said engineered ta-siRNAs.

WO 2012/092580 concerns nucleic acid molecules and methods of use thereof for control of coleopteran pests through RNA interference mediated inhibition of target coding and transcribed non coding sequences in coleopteran pests. The disclosure also concerns methods for making transgenic plants that express nucleic acid molecules useful for the control of coleopteran pests, and the plant cells and plants obtained thereby.

WO 2005/110068 is directed to controlling pest infestation by inhibiting one or more biological functions in an invertebrate pest. It discloses methods and compositions for use in controlling pest infestation by feeding one or more different recombinant double stranded RNA molecules to the pest in order to achieve a reduction in pest infestation through suppression of gene expression. It is also directed to methods for making transgenic plants that express the double stranded RNA molecules, and to particular combinations of transgenic pesticidal agents for use in protecting plants from pest infestation.

WO 99/32619 relates to a process of introducing an RNA into a living cell to inhibit gene expression of a target gene in that cell. The process may be practiced ex vivo or in vivo. The RNA has a region with double-stranded structure. Inhibition is sequence-specific in that the nucleotide sequences of the duplex region of the RNA and of a portion of the target gene are identical. It is distinguished from prior art interference in gene expression by antisense or triple-strand methods.

WO 2011/112570 provides polynucleotide molecules and methods for regulating genes in plants, *e.g.,* by providing RNA for systemic regulation of genes. Various aspects provide polynucleotide molecules and methods for regulating endogenous genes and transgenes in a plant cell and polynucleotide molecules.

WO 2008/007100 provides seed treatment compositions as well as their use, methods for treating seeds, methods of protecting plants against pests and also treated seeds and plants. In one embodiment there is provided a method of treating a seed with a seed treatment composition to induce a plant resistance mechanism against one or more pests in a plant grown from said seed.

WO 2008/148223 provides a novel method for the transduction and/or transfection of plant cells. Cell-penetrating peptides (CPPs) have been successfully employed as nanocarriers to deliver proteins and oligonucleotides to single plant cell microspores as well as multi-cellular zygotic embryos. The efficiency of CPP internalization and further delivery of a macromolecular cargo comprising a protein and/or an oligonucleotide can be enhanced by permeabilization of the zygotic embryos.

WO 2011001434 provides means and methods for simple and efficient introduction of foreign genetic material into the plant cell. Particularly, the it combines seed priming and virus-based DNA constructs for efficient introduction of heterologous DNA into plants.

EP 1416049 relates to degrading a specific form of any single-stranded RNA (ssRNA) sequence, particularly viral RNA, in a plant cell.

WO 2013/025670 provides a method to silence an endogenous target gene expression in plants by applying a specific dsRNA onto the exterior surface of a plant. Application, such as by spraying or brushing a plant with dsRNA is done without wounding the plant tissue and cells such as by mechanical-type wounding, particle bombardment or mechanical infection with viral vectors. It enables the regulation of gene expression in plants. In some embodiments the dsRNA is directed to an essential gene of a plant pathogen or pest, whereby the pathogen and/or pest damage is controlled, resulting in desired agronomic performance.

WO 2013/175480 relates to a method of introducing naked dsRNA into a seed. The method comprising contacting the seed with the naked dsRNA under conditions which allow penetration of the dsRNA into the seed, thereby introducing the dsRNA into the seed.

Unnamalai et al., "Cationic oligopeptide-mediated delivery of dsRNA for post-transcriptional gene silencing in plant cells," FEBS Letters, 566:307 - 310 (2004) relates to the delivery of dsRNA molecules to plant cells using cationic oligopeptides.

### SUMMARY OF THE INVENTION

The present disclosure relates to a method of treating a seed to improve insect resistance of a plant grown from the seed, the method comprising:
(a) priming the seed prior to introducing a naked exogenous double-stranded RNA (dsRNA) into the seed, wherein said priming is effected by:
   (i) washing the seed prior to said contacting; and
   (ii) drying the seed following step (i);
(b) wherein said introducing is effected by directly contacting said seed with said naked exogenous dsRNA,
   wherein said naked exogenous dsRNA molecule comprises at least one polynucleotide strand comprising at least one segment of 18 or more contiguous nucleotides of an insect pest gene in either anti-sense or sense orientation, wherein said contacting is effected by soaking the seed in a solution comprising a final concentration of 0.005 µg/µL to 1.5 µg/µL of said naked exogenous dsRNA and shaking the seed in the solution for 4 to 24 hours, wherein the dsRNA molecule is introduced into the seed but is not expressed from the plant genome, thereby not being an integral part of the genome; and wherein the plant grown from the seed exhibits improved insect resistance relative to a control plant, wherein the control plant is grown from a seed not contacted with the exogenous dsRNA molecule.

Preferably, the insect pest gene is selected from the group consisting of ATPase, NADPH Cytochrome P450 Oxidoreductase, IAP, Chitin Synthase, EF1α, and β-actin.

Preferably, the plant is resistant to *Spodoptera littoralis, Diabrotica virgifera virgifera* or *Leptinotarsa decemlineata* infestation.

Preferably, said washing is effected for 2 to 6 hours.

Preferably, said washing is effected at 4°C to 28°C.

Preferably, said drying is effected at 25°C to 30°C for 10 to 16 hours.

Preferably, washing is effected in the presence of double deionized water.

### BRIEF DESCRIPTION OF THE DRAWINGS

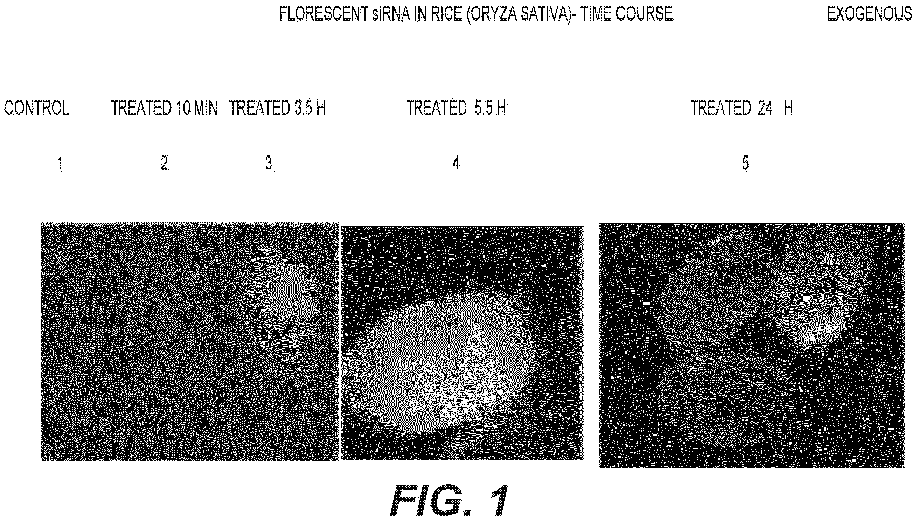

FIG. 1 shows a time-course siGLO-treatment results on rice seeds. The effect of incubation time with siGLO dsRNA on fluorescence intensity, indicating quantity and quality of dsRNA penetration, was tested. Control seeds that were left untreated (1), were imaged along with seeds treated with siGLO dsRNA for four different incubation times; 10 min (2), 3.5 hours (3), 5.5 hours (4), and 24 hours (5).

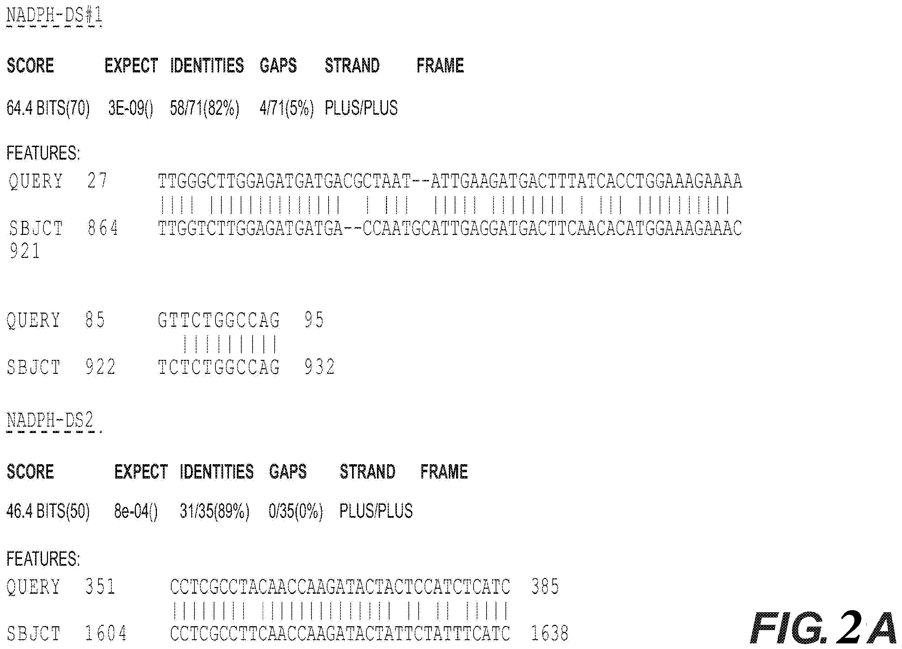

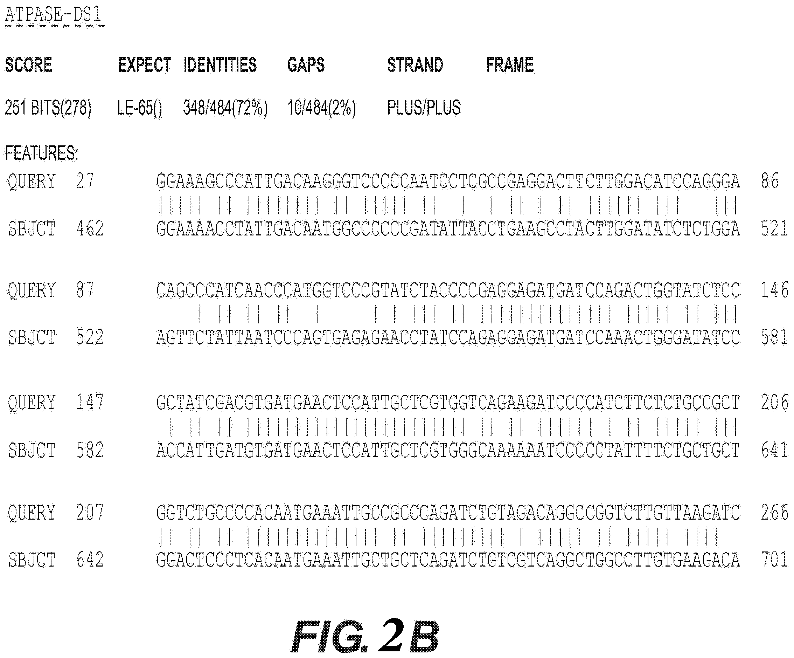

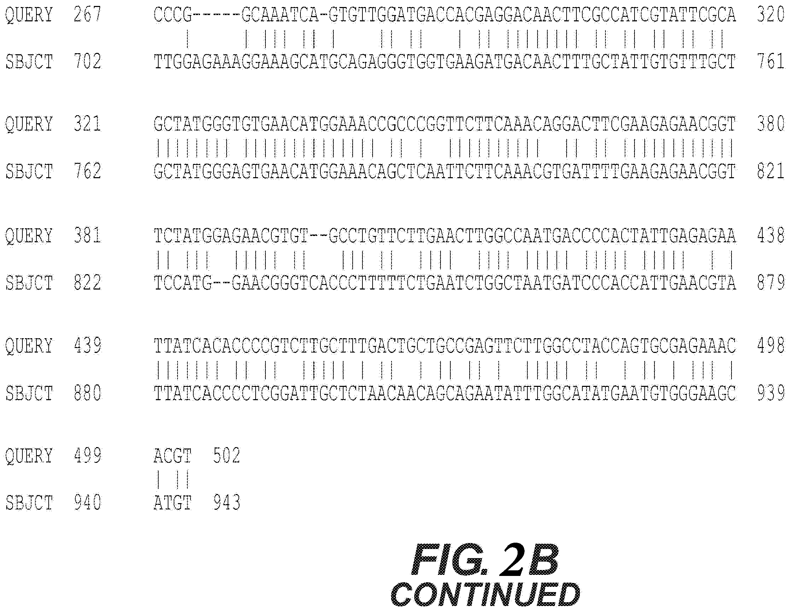

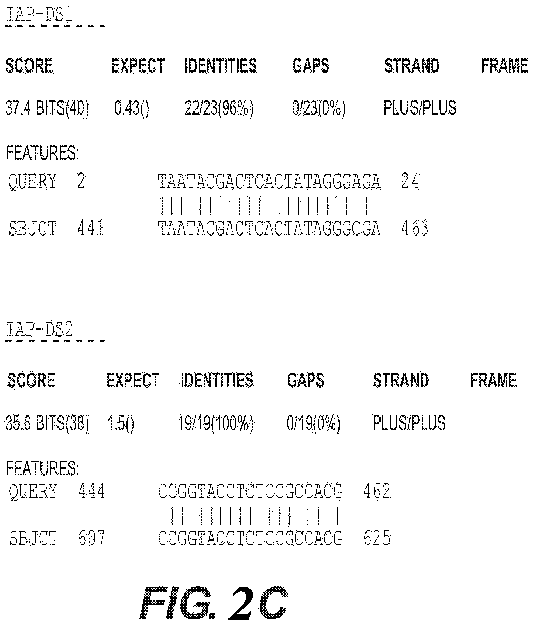

FIGs. 2A-C show the homology between the *Spodoptera littoralis* genes used for seed treatment and the corn genome. FIG. 2A - NADPH gene, sequence 1 (top panel, SEQ ID NO: 14 and 22) and sequence 2 (bottom panel, SEQ ID NO: 23 and 24) showing 82% identity over 71 nucleotides and 89% identity over 35 nucleotides respectively, FIG. 2B - ATPase (SEQ ID NOs: 25 and 26) showing 72% identity over 484 nucleotides, and FIG. 2C - IAP, sequence 1 (top panel, SEQ ID NO: 27 and 28) and sequence 2 (bottom panel, SEQ ID NO: 29 and 30) showing 81% identity over 36 nucleotides and 87% identity over 31 nucleotides respectively. "Query" stands for S. *littoralis* sequences and "Subject" stands for corn sequences.

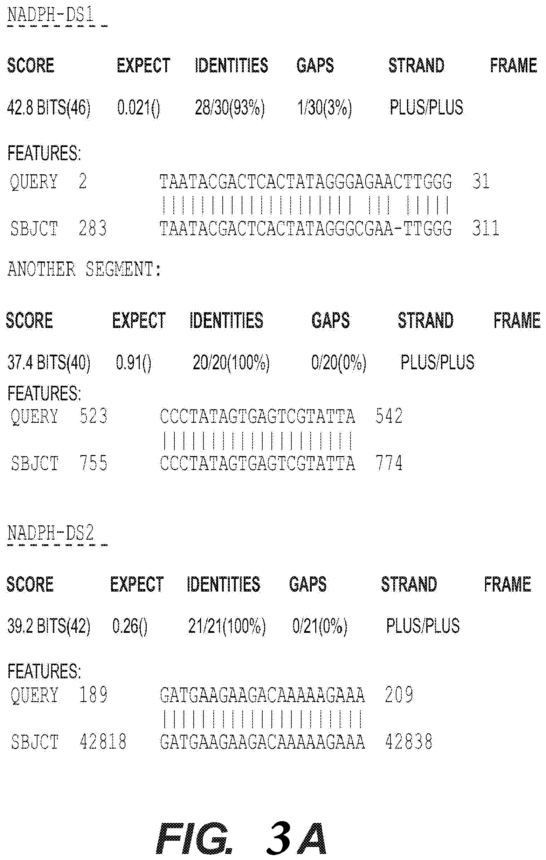

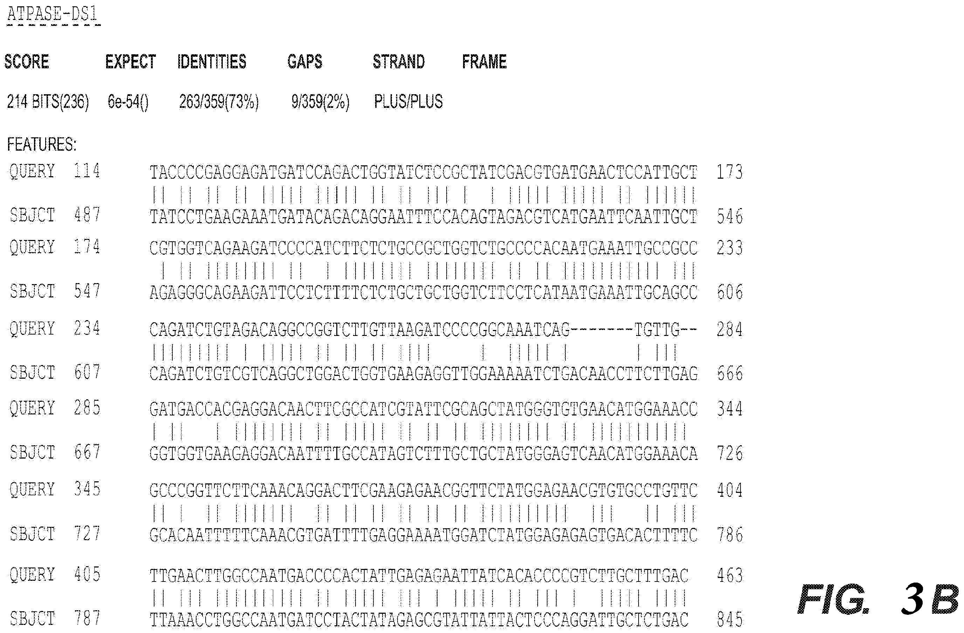

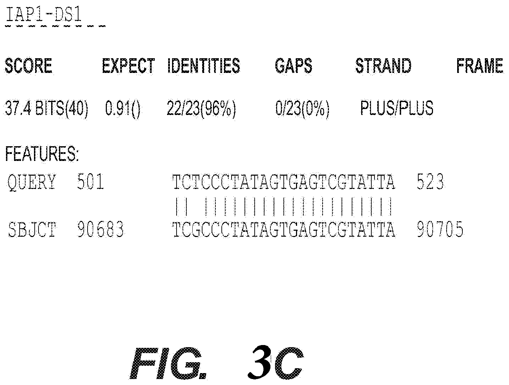

FIGs. 3A-C show the homology between the *Spodoptera littoralis* genes used for seed treatment and the tomato genome. FIG. 3A - NADPH gene, sequence 1 (top panel, SEQ ID NO: 31 and 32) showing 93% identity over 30 nucleotides and 88% identity over 25 nucleotides respectively and sequence 2 (bottom panel, SEQ ID NO: 33 and 34) FIG. 3B - ATPase (SEQ ID NOs. 35 and 36) showing 73% identity over 359 nucleotides, and FIG. 3C - IAP (SEQ ID NOs. 37 and 38) showing 93 % identity over 28 nucleotides. "Query" stands for S. *littoralis* sequences and "Subject" stands for tomato sequences.

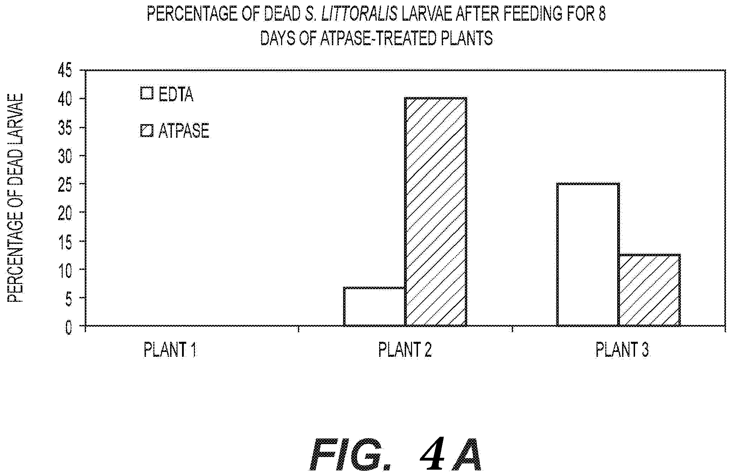

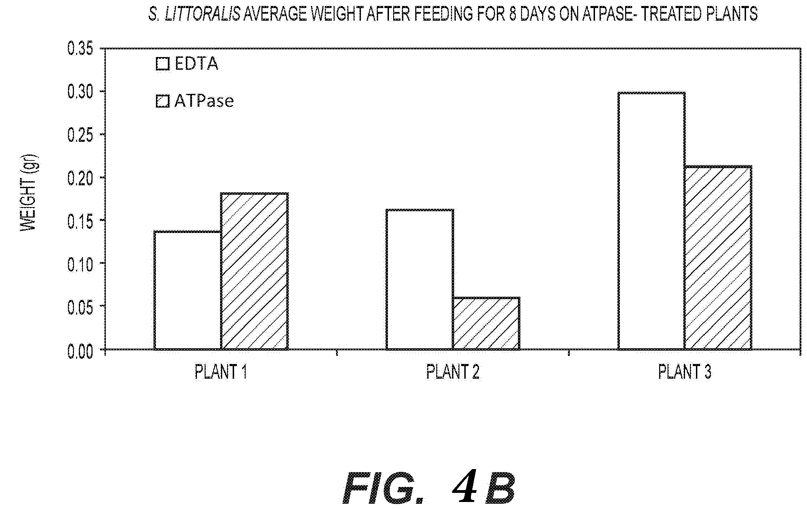

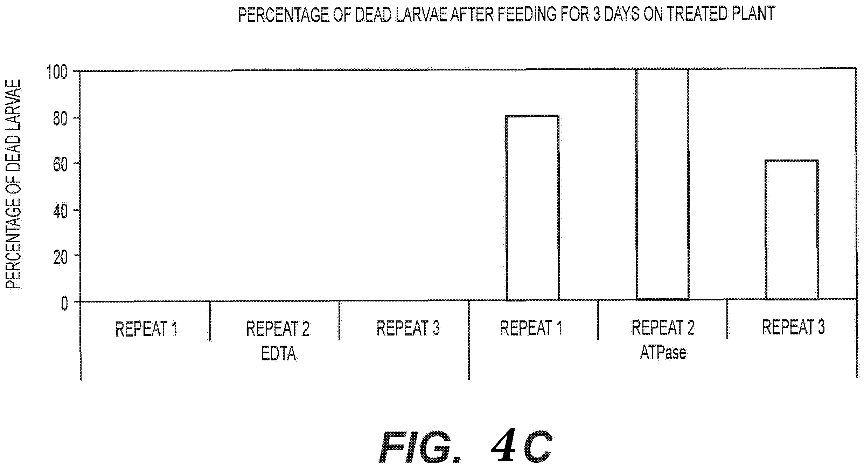

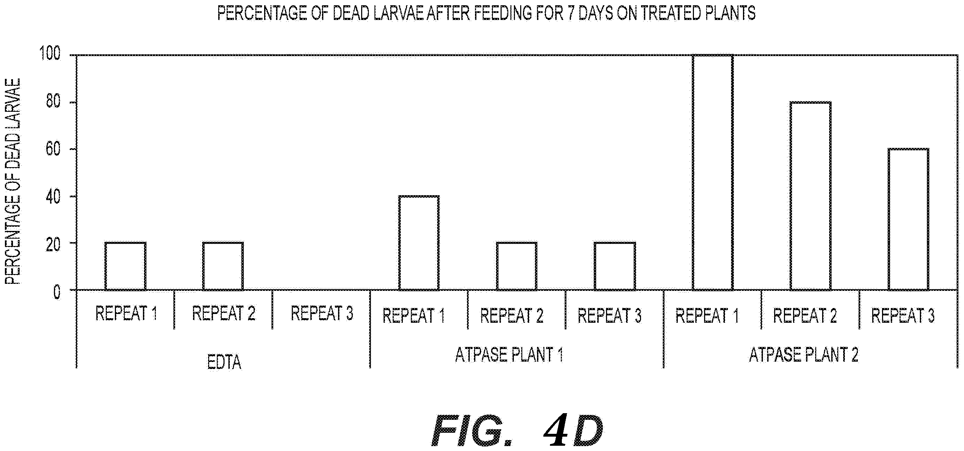

FIGs. 4A-D are bar graphs showing mortality and average weight of live S. *littoralis* larvae. FIG. 4A shows percentage of dead worms eight days after feeding on three 43-day-old ATPase dsRNA trigger-treated and control corn plants. FIG. 4B shows average weight of live *S. littoralis* larvae at the same time point. FIG. 4C is a bar graph showing percentage of dead S. *littoralis* larvae three days after feeding on 85-days old ATPase-treated and control corn plants. FIG. 4D is a bar graph showing percentage of dead *S. littoralis* larvae seven days after feeding on 91-day-old ATPase dsRNA trigger-treated and control corn plants.

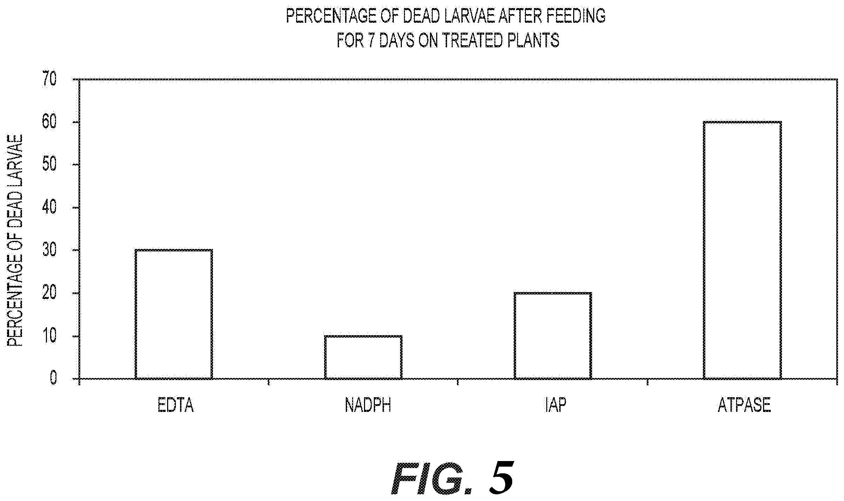

FIG. 5 is a bar graph showing percentage of dead *S. littoralis* larvae seven days after feeding on 67-day-old dsRNA trigger treated (NADPH, IAP, and ATPase) and control (EDTA) corn plants.

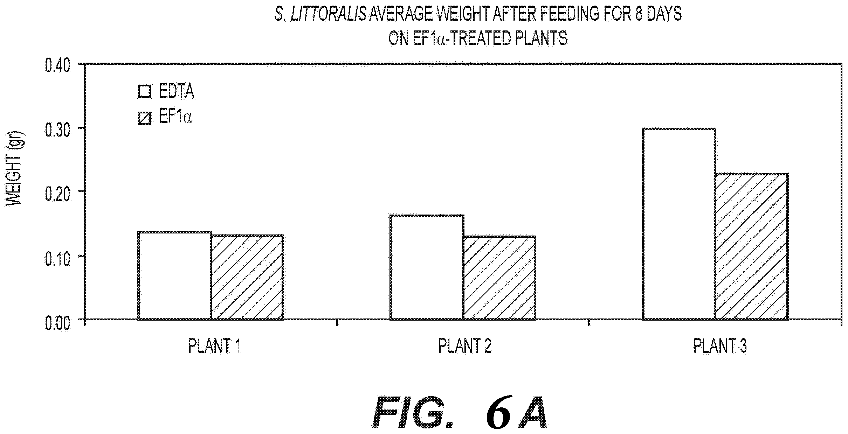

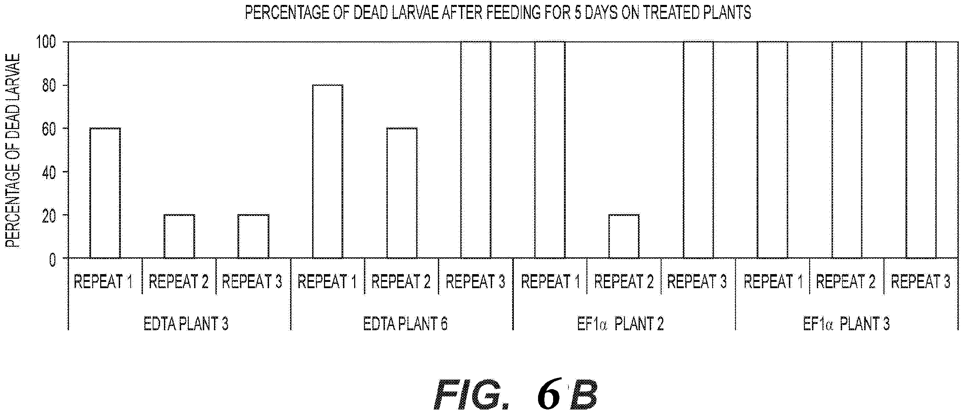

FIGs. 6A-B: FIG. 6A is a bar graph showing average weight of live *S. littoralis* larvae eight days after feeding on 43-day-old EF1α dsRNA trigger-treated and control (EDTA) corn plants. FIG. 6B is a bar graph showing percentage of dead *S. littoralis* larvae five days after feeding on 87-day-old EF1α dsRNA trigger-treated and control (EDTA) corn plants.

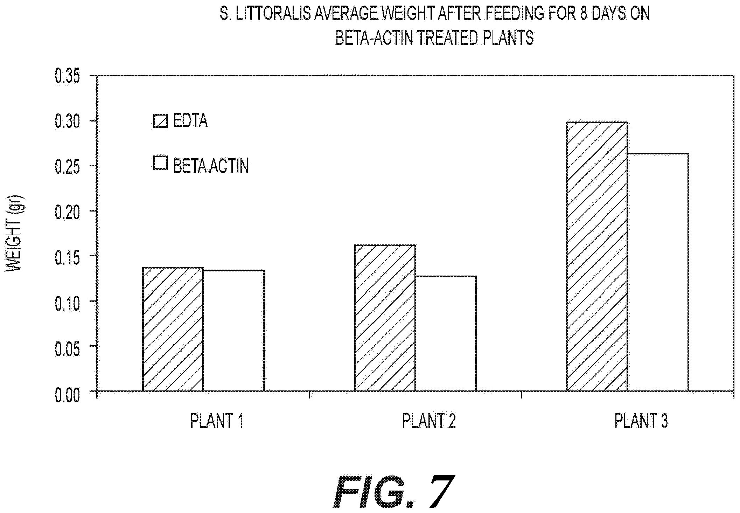

FIG. 7 is a bar graph showing average weight of live *S. littoralis* larvae eight days after feeding on 43-day-old Beta-actin dsRNA trigger-treated and control (EDTA) corn plants.

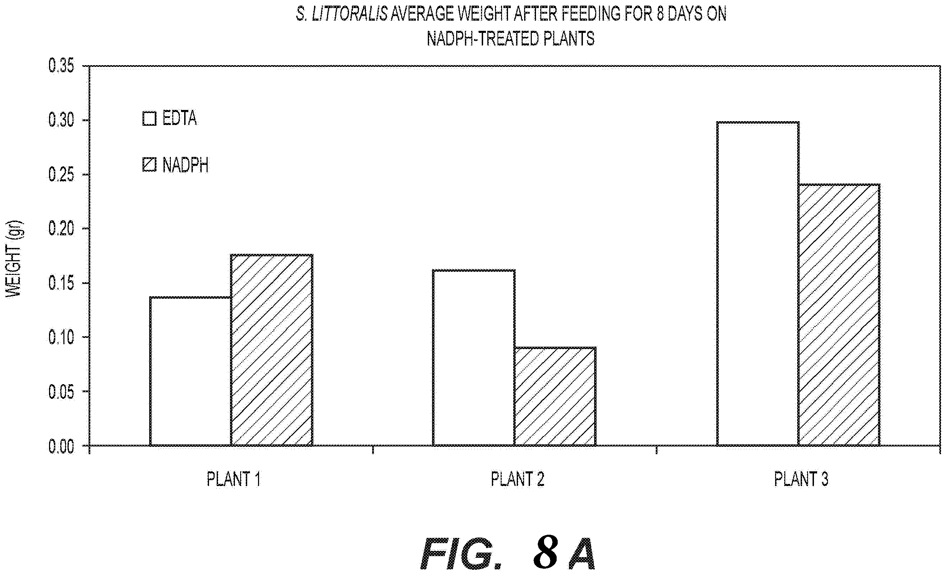

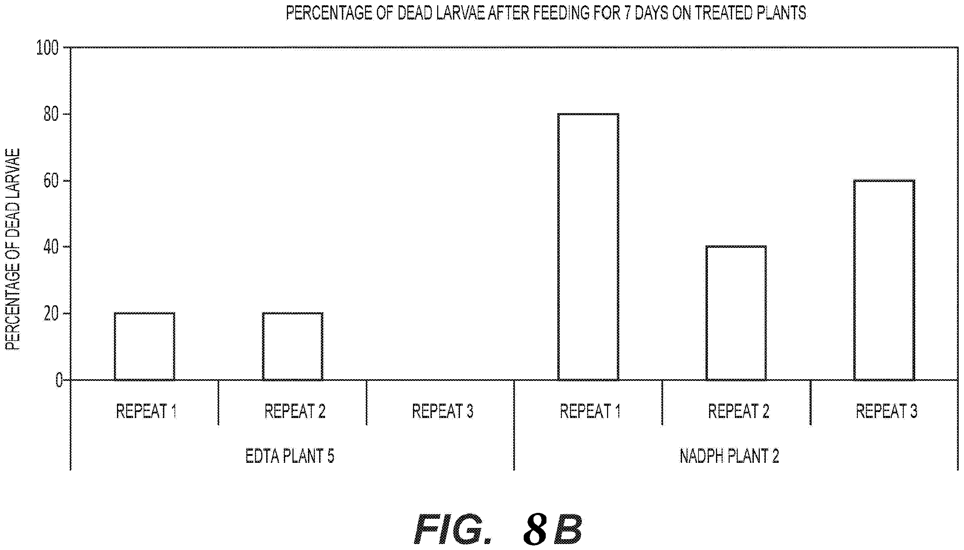

FIGs. 8A-B: FIG. 8A is a bar graph showing average weight of live *S. littoralis* larvae eight days after feeding on 43-day-old NADPH dsRNA trigger-treated and control (EDTA) corn plants. FIG. 8B is a bar graph showing percentage of dead *S. littoralis* larvae seven days after feeding on 91-day-old NADPH dsRNA trigger-treated and control (EDTA) corn plants.

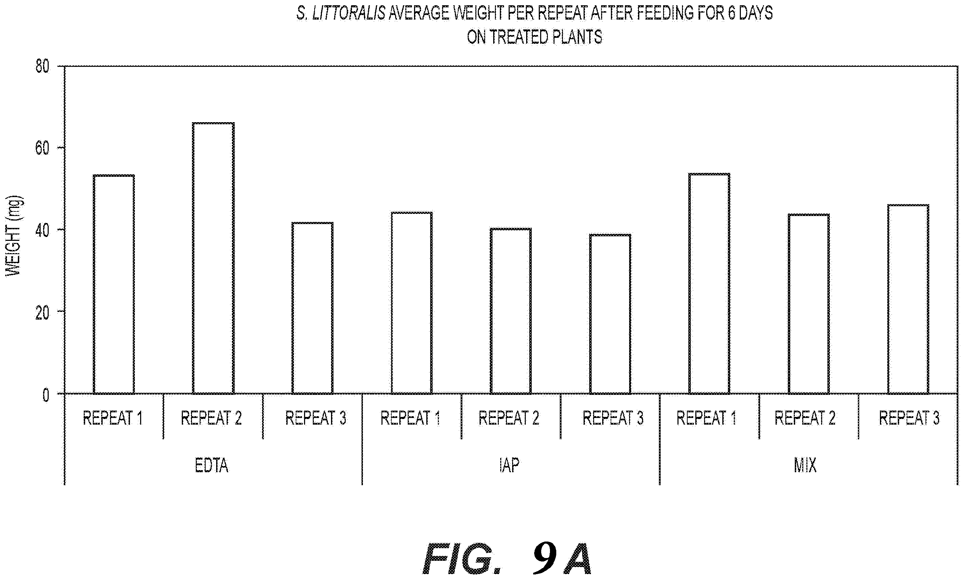

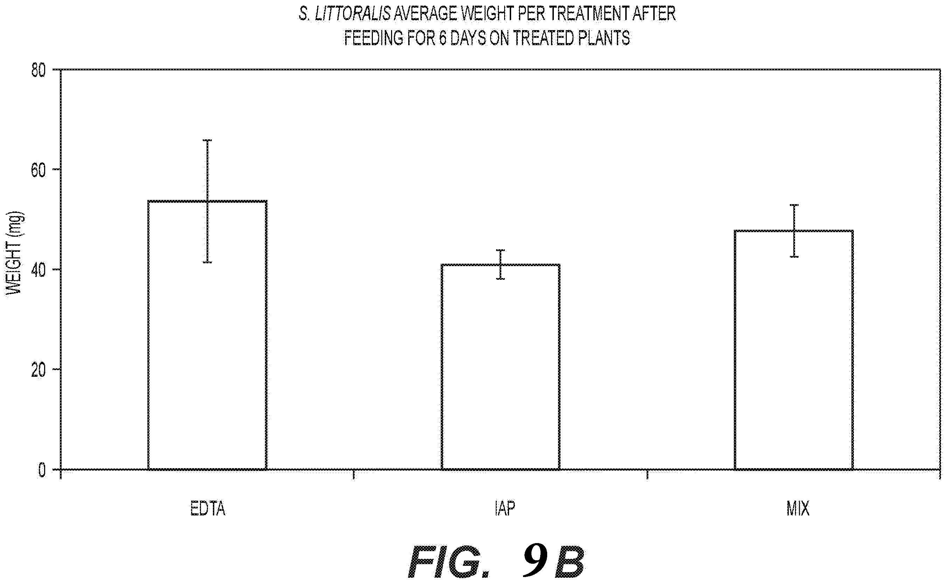

FIGs. 9A-B are bar graphs showing average weight of live *S. littoralis* larvae six days after feeding on 27-day-old dsRNA trigger-treated (IAP or MIX (IAP, NADPH and ATPase)) compared to control (EDTA) plants. FIG. 9A shows average weight per repeat and FIG. 9B shows average weight per treatment.

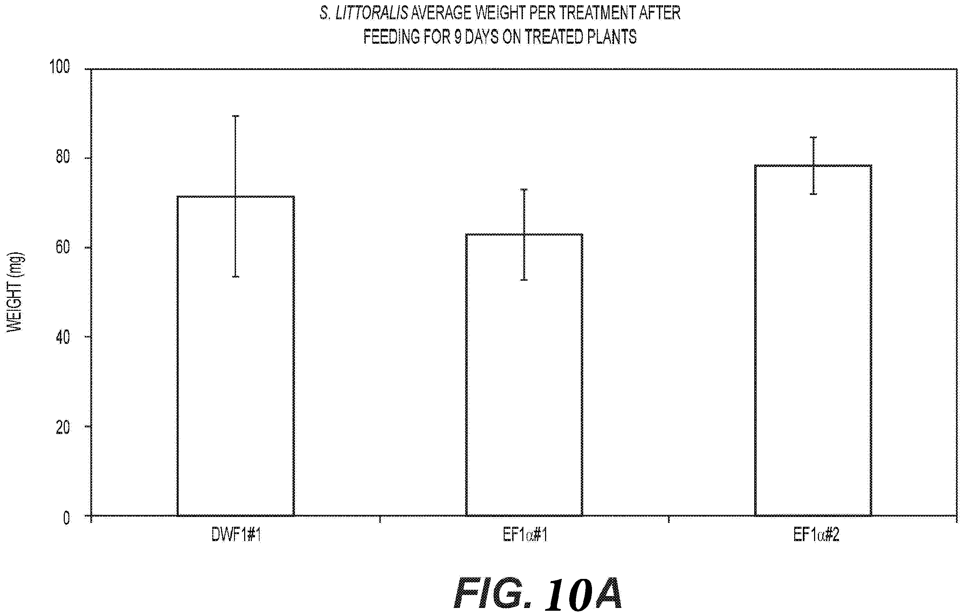

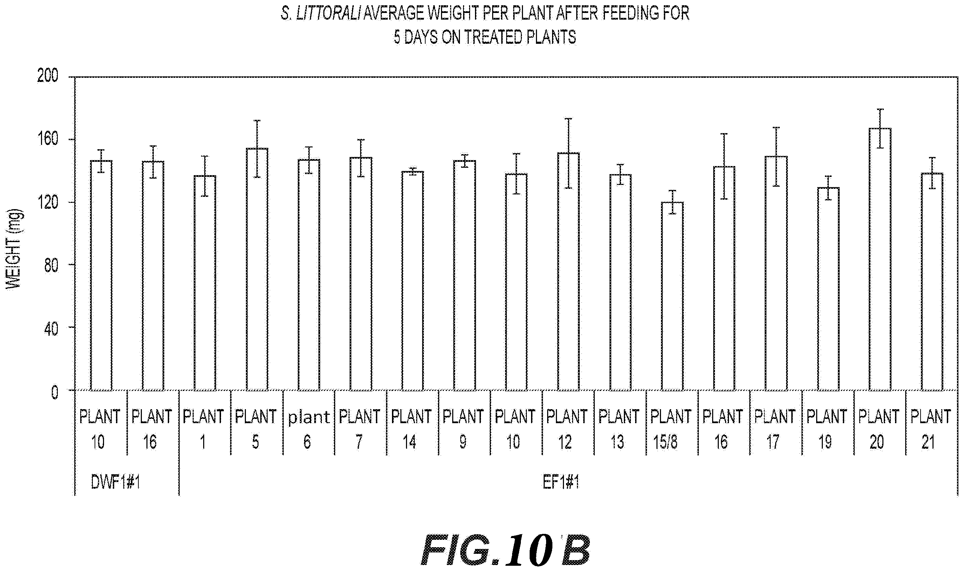

FIGs. 10A-B are bar graphs showing average weight of live *S. littoralis* larvae after feeding on EF1α dsRNA trigger-treated corn plants. FIG. 10A shows average weight nine days after feeding on 35-day-old plants. Error bars represent standard deviation for each treatment. FIG. 10B shows average weight five days after feeding on 36-day-old plants. Error bars represent standard deviation for each plant.

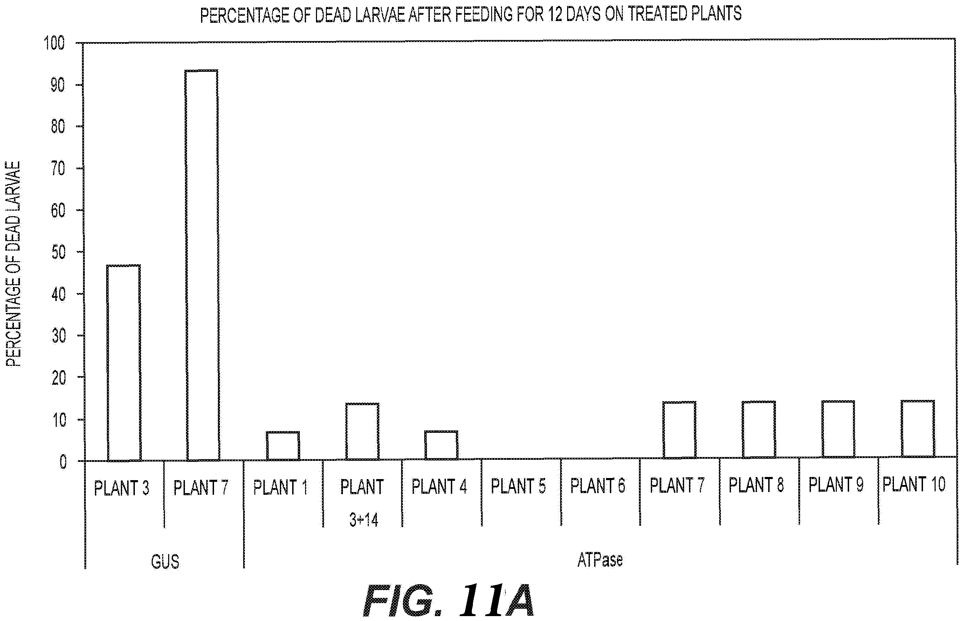

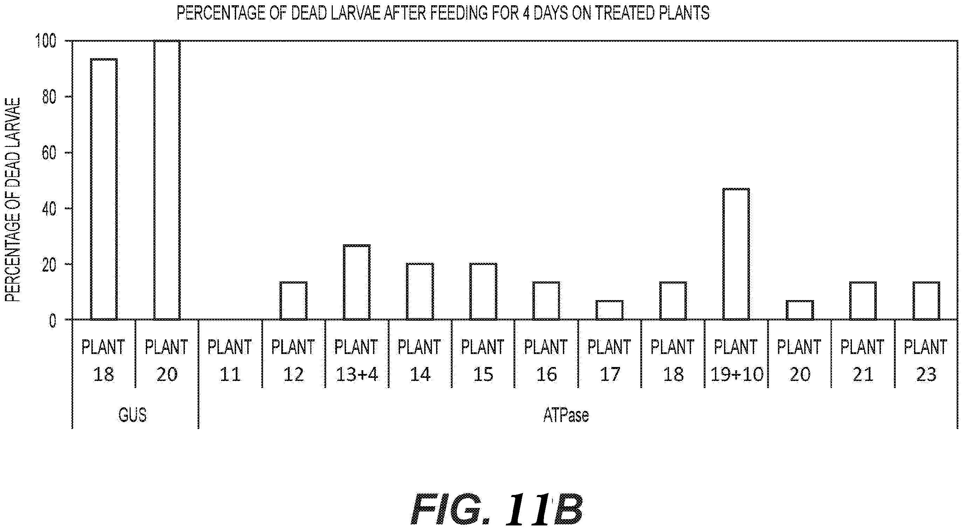

FIGs. 11A-B: FIG. 11A is a bar graph showing percentage of dead *S. littoralis* larvae 12 days after feeding on 56-day-old ATPase dsRNA trigger-treated and control (GUS) corn plants. FIG. 11B is a bar graph showing percentage of dead *S. littoralis* larvae four days after feeding on 57-day-old ATPase dsRNA trigger-treated and control (GUS) corn plants.

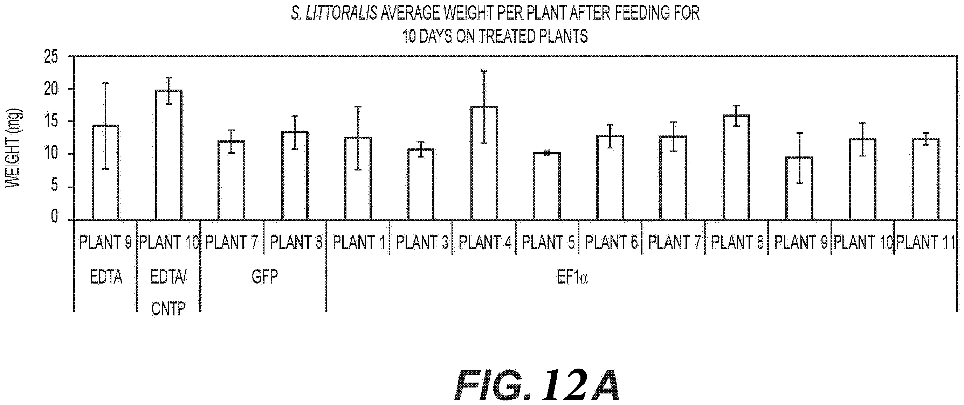

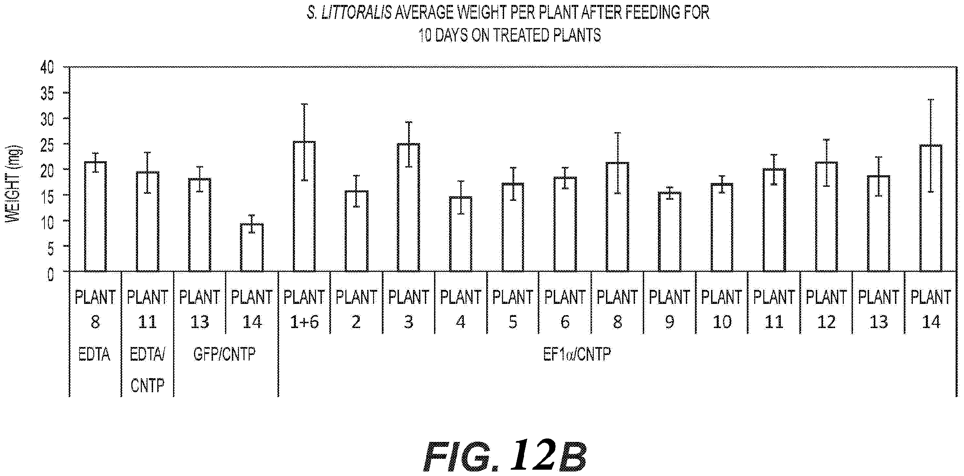

FIGs. 12A-B: FIG. 12A is a bar graph showing average weight of live *S. littoralis* larvae ten days after feeding on 24-day-old dsRNA trigger-treated and control (EDTA, EDTA/CNTP and GFP) corn plants. Error bars represent standard deviation for each plant. FIG. 12B is a bar graph showing average weight of live *S. littoralis* larvae ten days after feeding on 25-day-old dsRNA trigger-treated and control (EDTA, EDTA/CNTP and GFP/CNTP) corn plants. Error bars represent standard deviation for each plant.

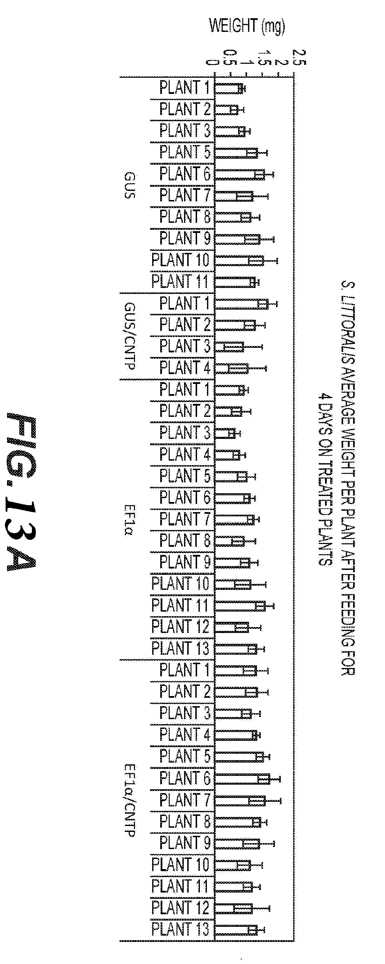

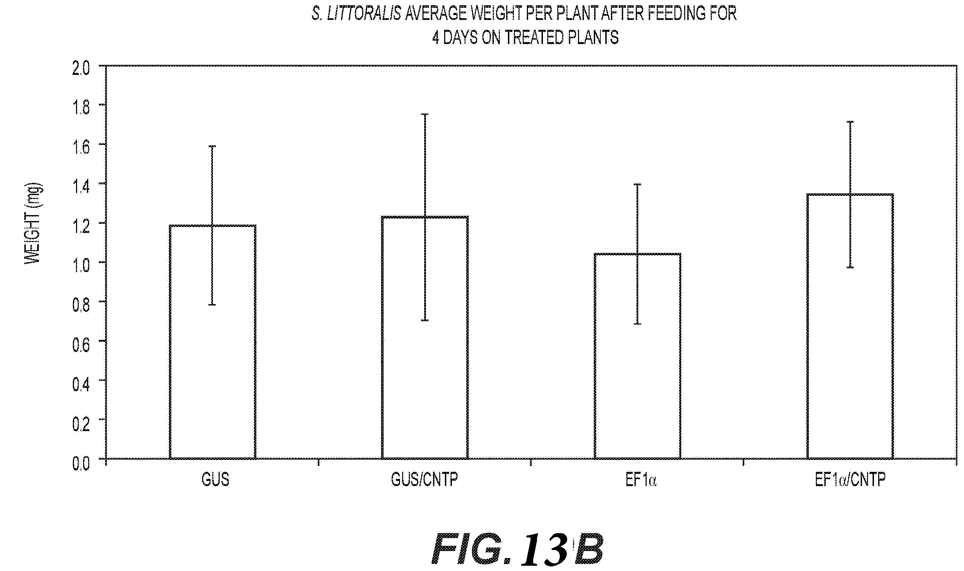

FIGs. 13A-B are bar graphs showing average *S. littoralis* larvae weight 4 days after feeding on eight-day-old dsRNA trigger-treated (EF1α and EF1α/CNTP) and control (GUS and GUS/CNTP) corn plants. FIG. 13A shows average weight of S. *littoralis* larvae per plant and FIG. 13B shows average weight of *S*. *littoralis* larvae per treatment. Error bars represent standard deviation of the data.

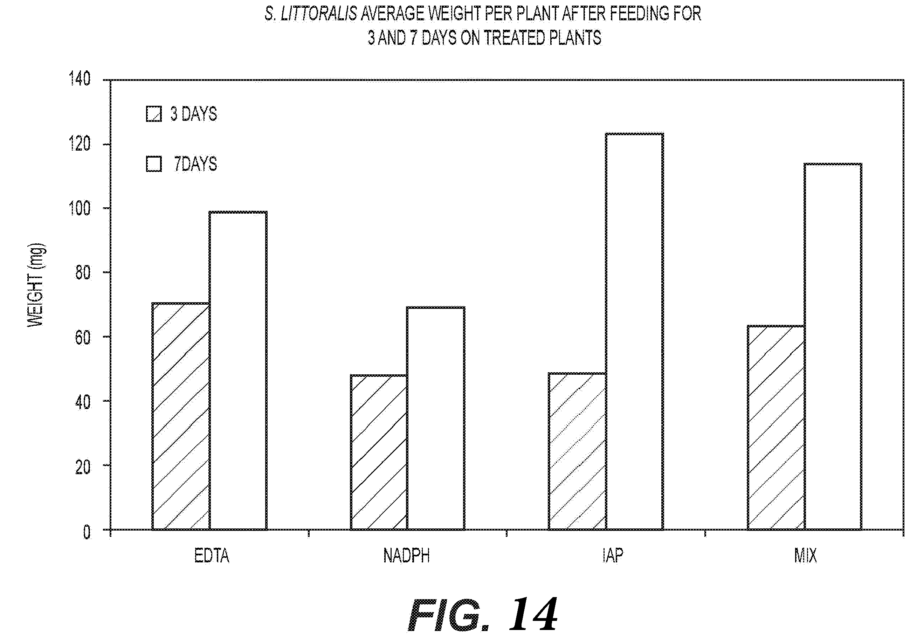

FIG. 14 is a bar graph showing average weight of live *S. littoralis* larvae three and seven days after feeding on 48-day-old dsRNA trigger-treated (NADPH, IAP, and MIX (IAP, ATPase and NADPH))and control (EDTA) tomato plants.

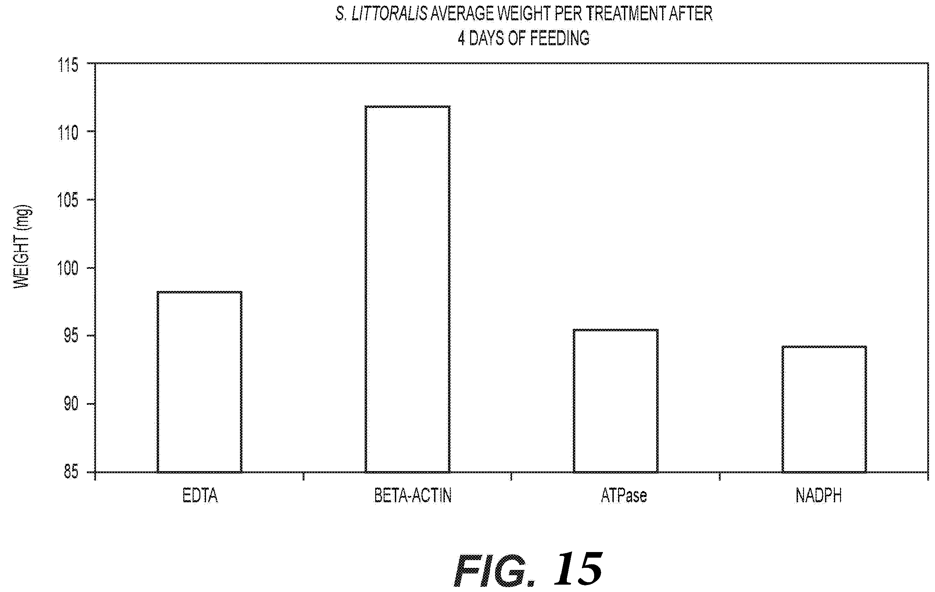

FIG. 15 is a bar graph showing average weight of live *S. littoralis* larvae after feeding for four days on 42-day-old dsRNA trigger-treated (Beta-actin, ATPase and NADPH) and control (EDTA) tomato plants.

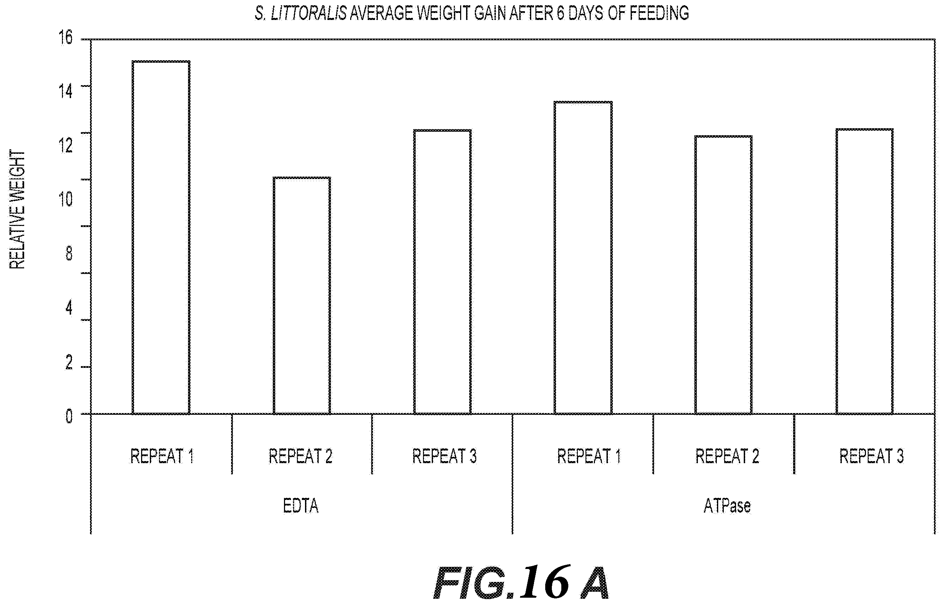

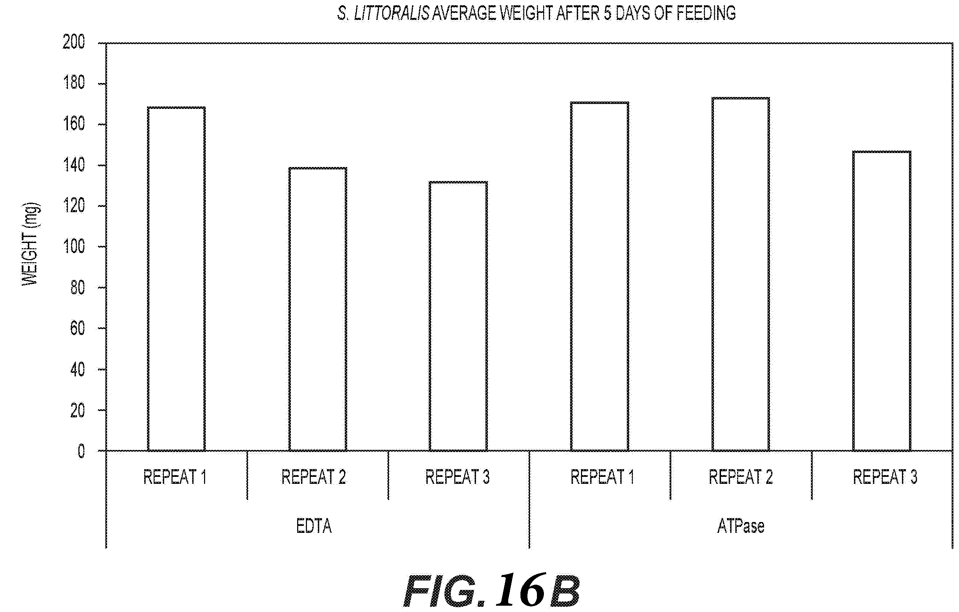

FIGs. 16A-B: FIG. 16A is a bar graph showing weight of *S. littoralis* larvae after feeding for six days on 85-day-old ATPase dsRNA trigger-treated and control (EDTA) tomato plants relative to their initial weight before feeding. FIG. 16B is a bar graph showing average weight of live *S. littoralis* larvae after feeding for five days on 88-day-old ATPase dsRNA trigger-treated and control (EDTA) tomato plants.

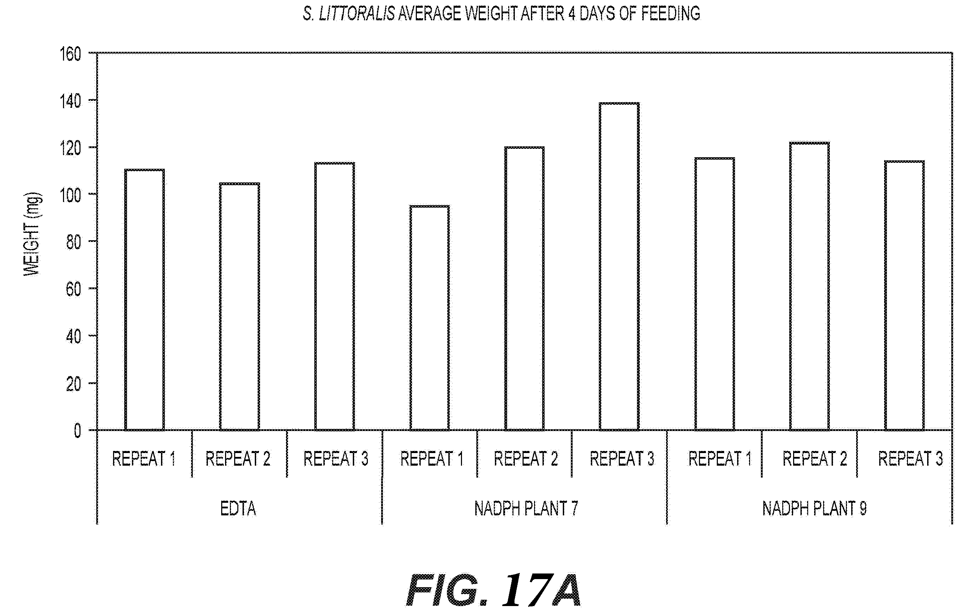

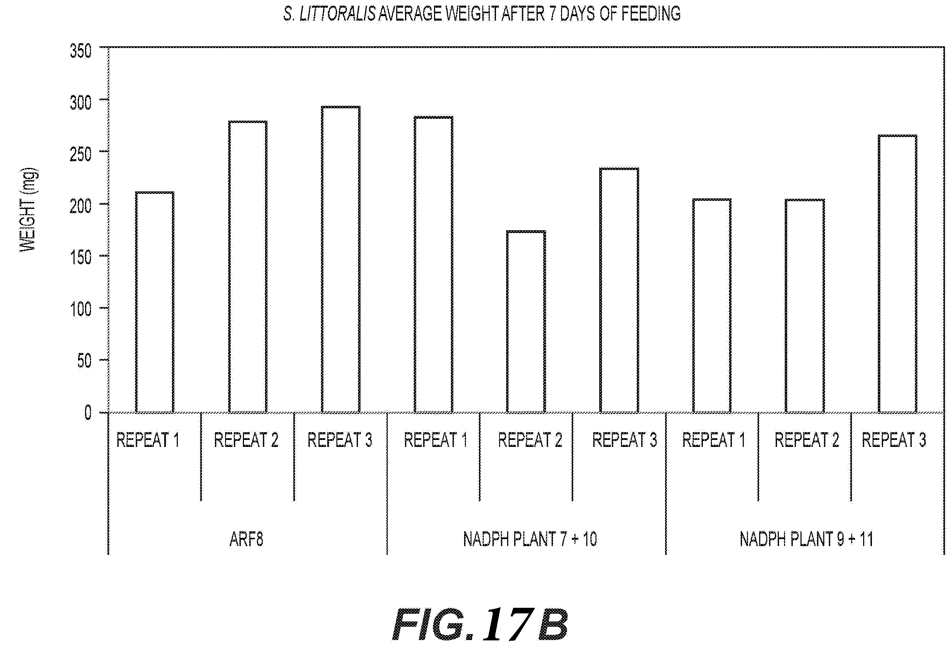

FIGs. 17A-B: FIG. 17A is a bar graph showing average weight *of S. littoralis* larvae after feeding for four days on 95-day-old NADPH dsRNA trigger-treated and control (EDTA) tomato plants. FIG. 17B is a bar graph showing average weight *of S. littoralis* larvae after feeding for seven days on 95-day-old NADPH dsRNA trigger-treated and control (ARF8) tomato plants.

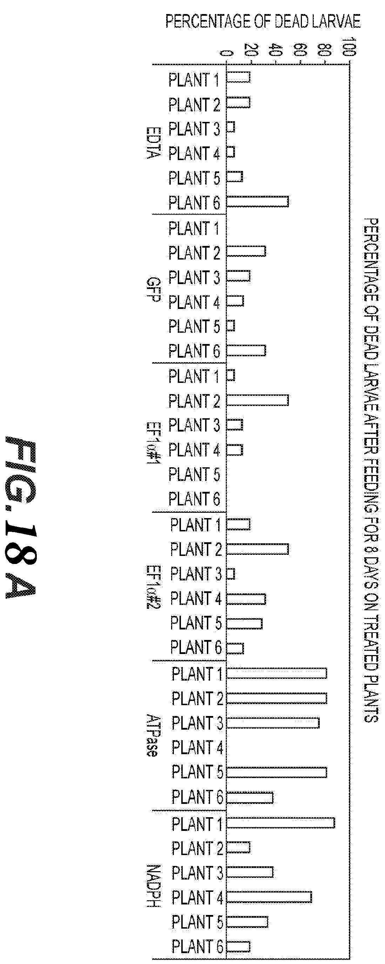

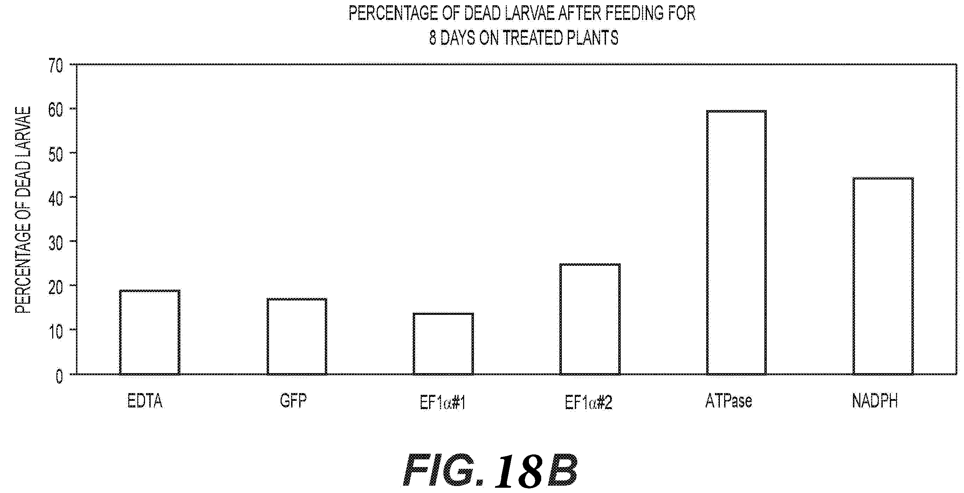

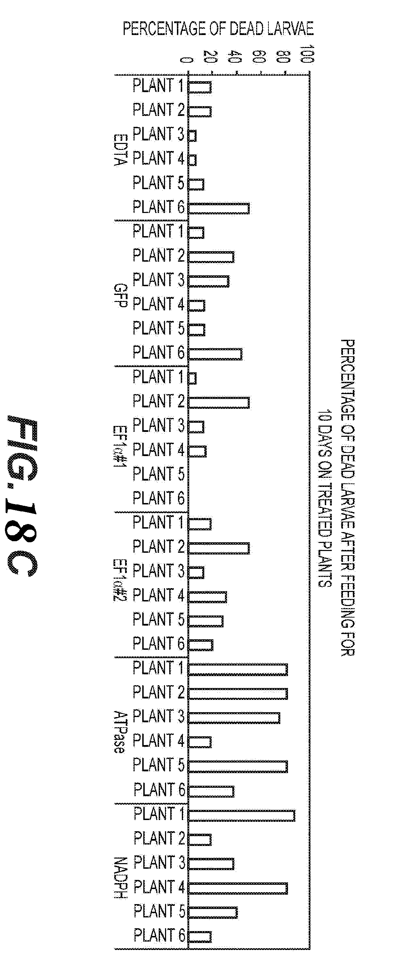

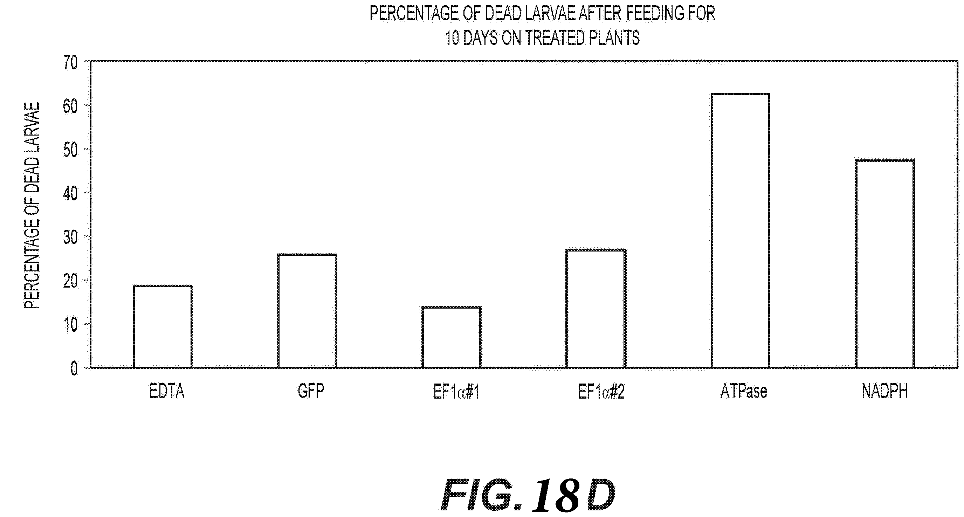

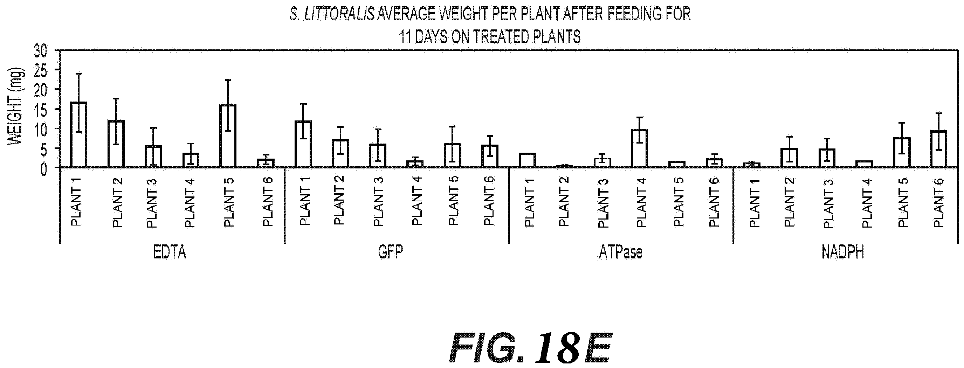

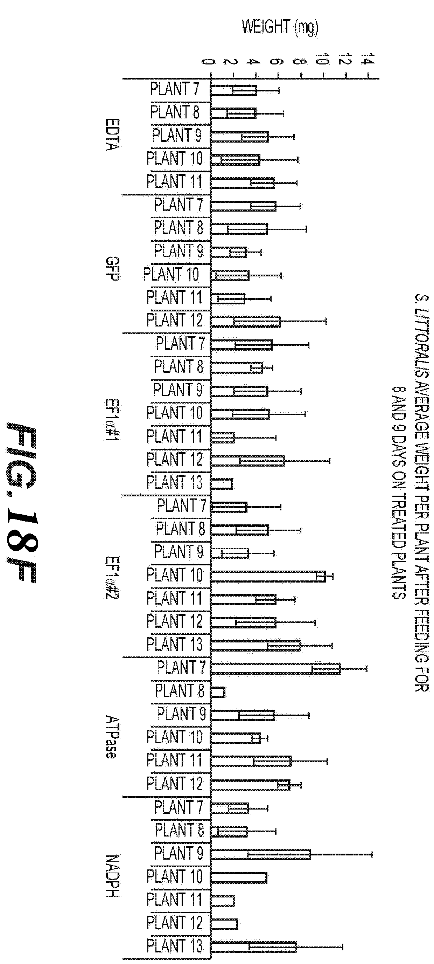

FIGs. 18A-F: FIGs. 18A and C are bar graphs showing percentage of dead *S. littoralis* larvae per plant eight and ten days, respectively, after feeding on 31-day-old dsRNA trigger-treated (EF1α#1, EF1α#2, ATPase and NADPH) and control (EDTA and GFP) corn plants. FIGs. 18 B and D are bar graphs combining the data shown in FIGs. 18 A and C into treatments. FIG. 18E is a bar graph showing average weight of live *S. littoralis* larvae 11 days after feeding on treated and control corn plants. Error bars represent standard deviation of the data. FIG. 18F is a bar graph showing average weight of live *S. littoralis* larvae after feeding for eight and nine days on 32-days old treated and control corn plants. Weight scored after eight days is shown in dark colors and weight scored after nine days is shown in bright colors. Error bars represent standard deviation of the data.

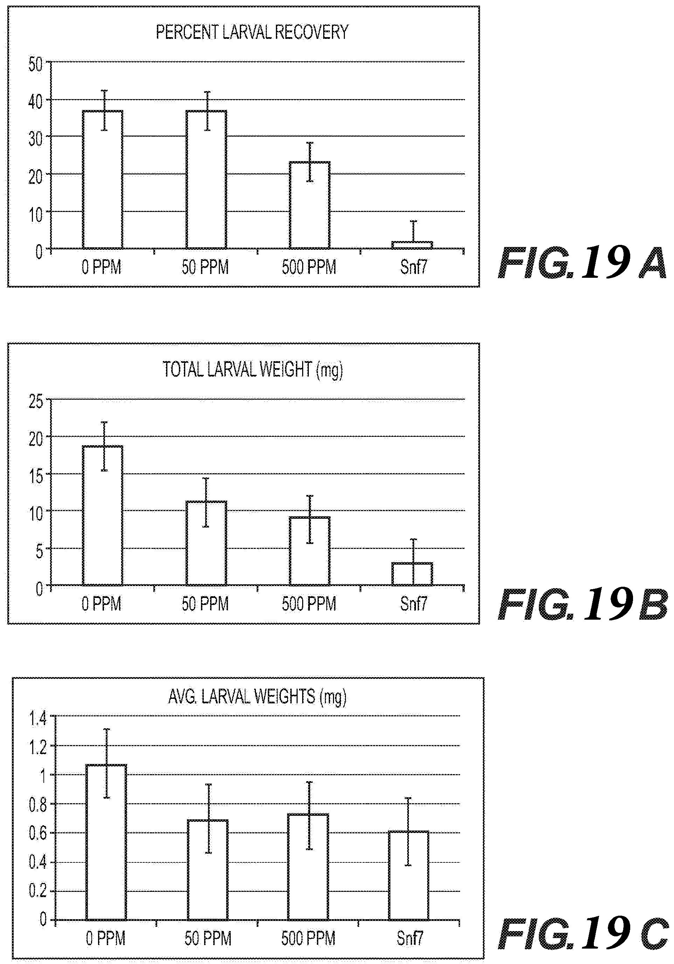

FIGs. 19A-C are bar graphs showing larval recovery and weight of Western corn rootworm (WCR) fed on corn plants grown from seeds treated with 0ppm (Null control), 50ppm or 500ppm MON104454 or transgenic maize plants expressing an RNA suppression construct targeting WCR Snf7 (positive control). FIG. 19A is a bar graph showing the percentage of larval recovery after 4 weeks. FIG. 19B is a bar graph showing the total weight of WCR larvae recovered after 4 weeks. FIG. 19C is a bar graph showing the average weight of the WCR larvae recovered after 4 weeks.

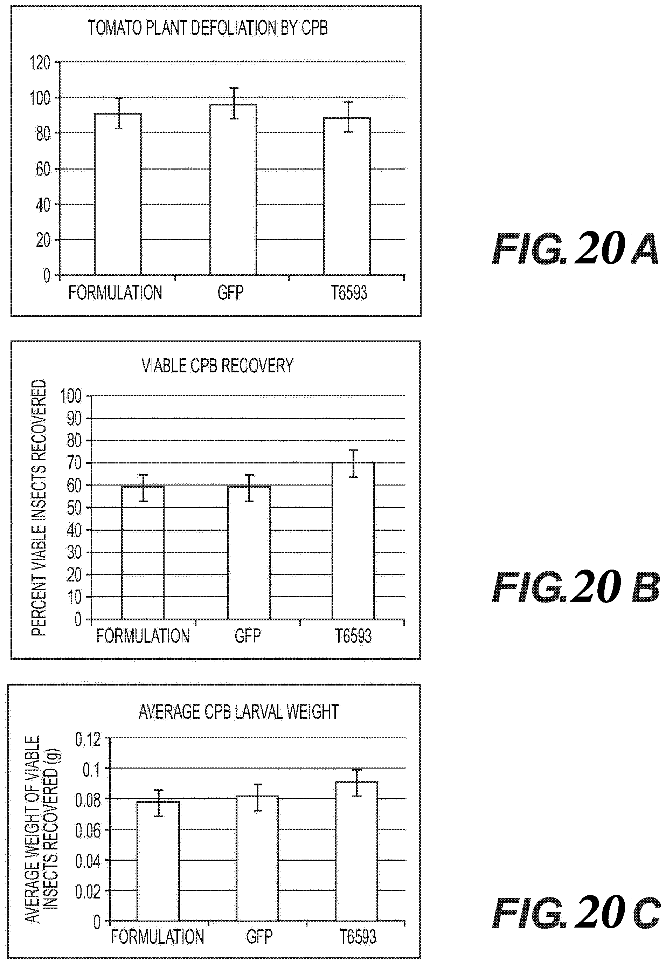

FIGs. 20A-C are bar graphs showing the results of a Colorado potato beetle (CPB) infestation assay on tomato plants grown from seeds treated with T6593, buffer ("formulation") or a GFP dsRNA trigger. FIG. 20A shows the average defoliation of the T6593 treated and control (formulation and GFP) tomato plant by CPB. FIG. 20B shows the percentage of CPB larvae recovered. FIG. 20C shows the average weight of WCR larvae recovered from the treated plants.

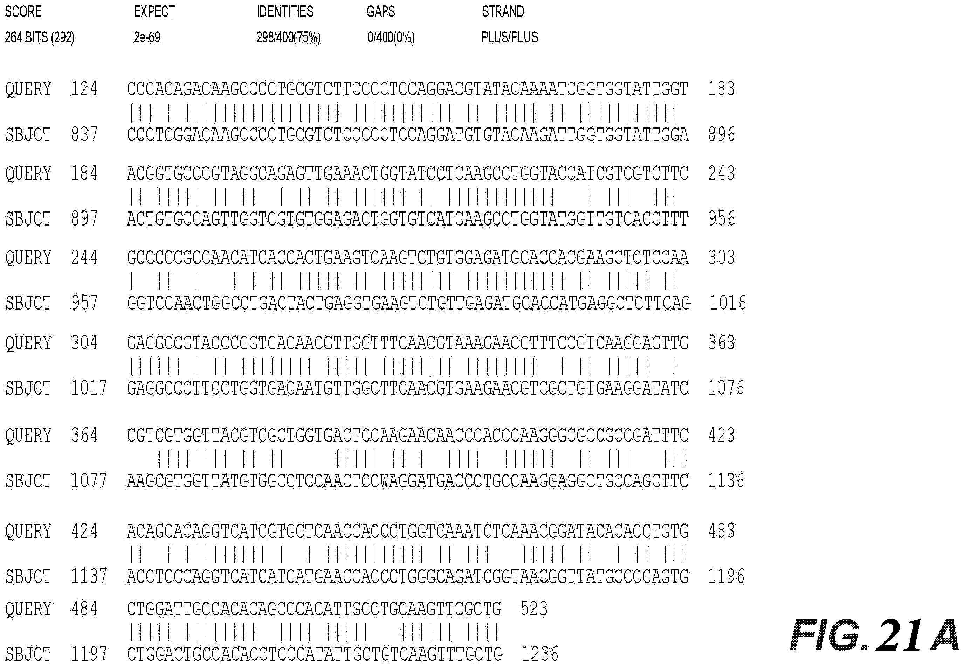

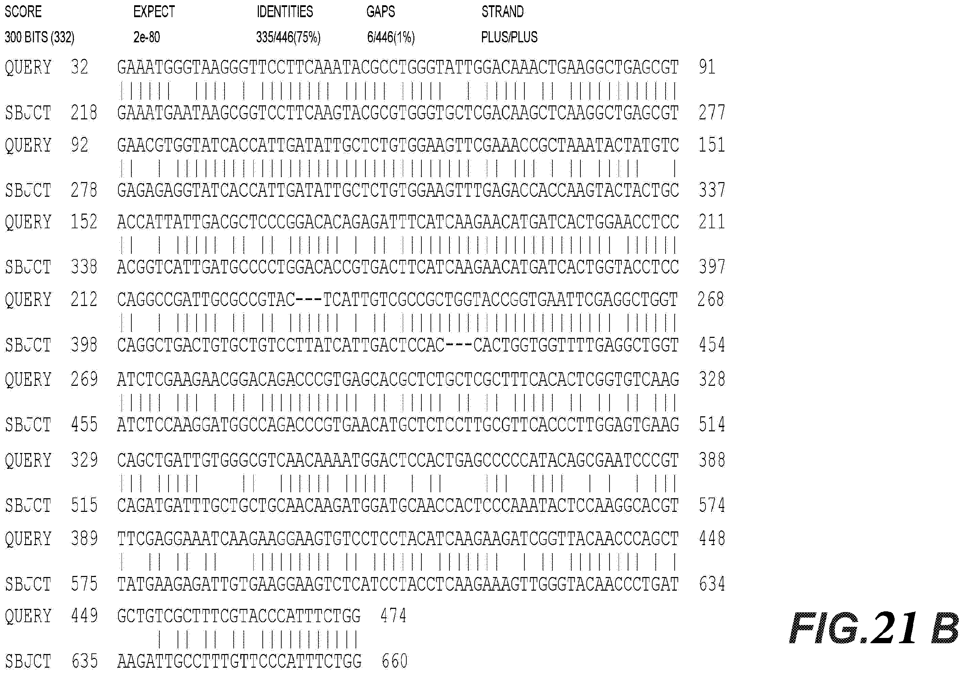

FIGs. 21A-B show the homology between the *Spodoptera littoralis* EF1α gene used for seed treatment and the corn genome. FIG. 21A - EF1α gene, sequence 1 showing 75% identity over 400 nucleotides. FIG. 21B - EF1α gene, sequence 2 showing 75% identity over 446 nucleotides. "Query" stands for *S. littoralis* sequences and "Subject" stands for corn sequences.

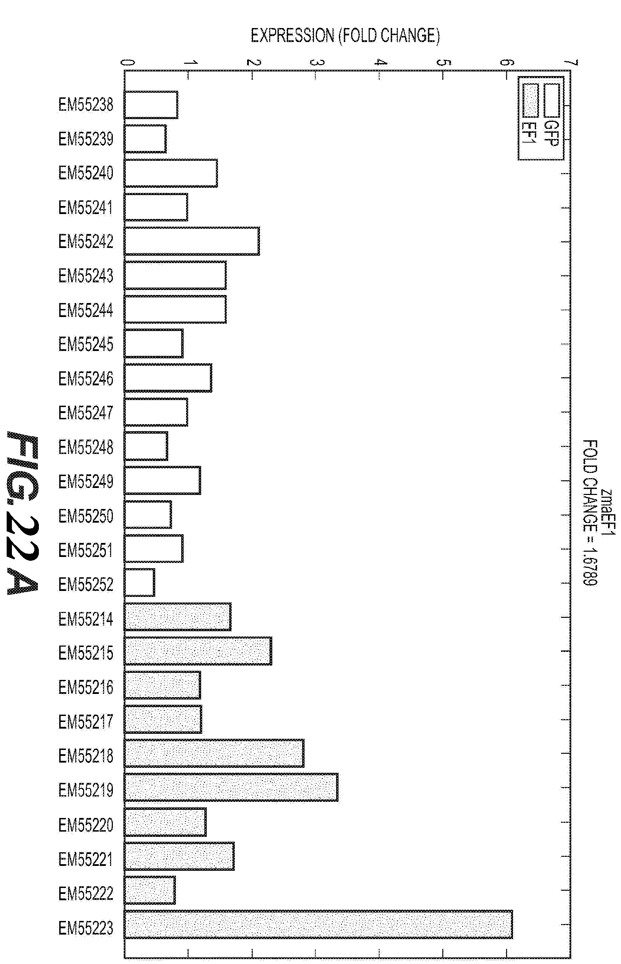

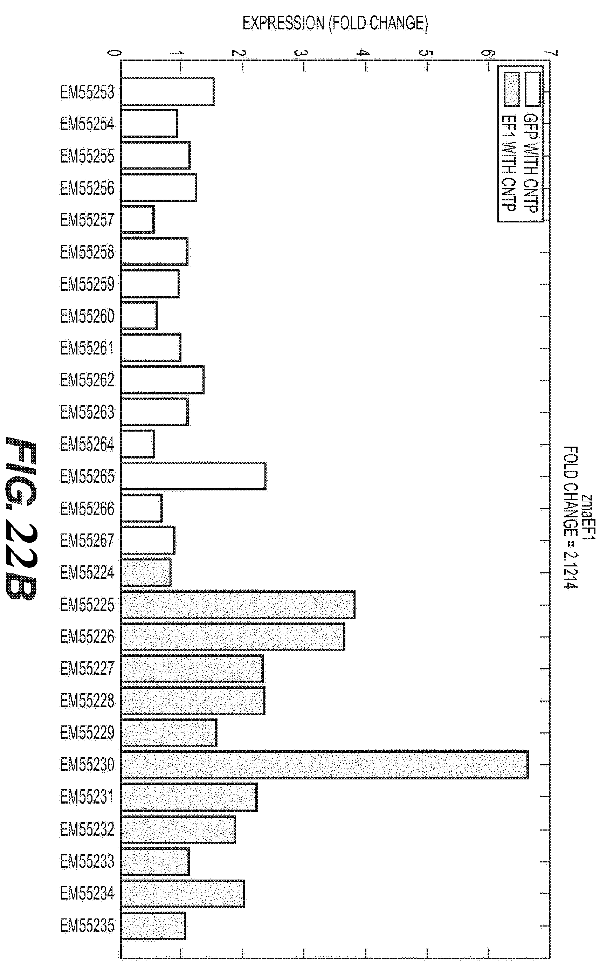

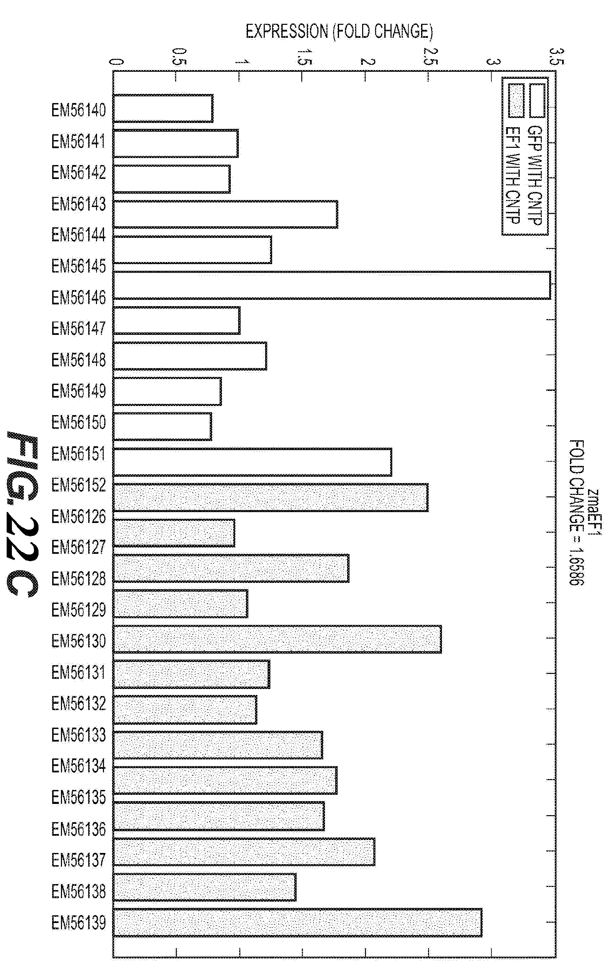

FIGs. 22A-C are bar graphs showing real-time PCR analyses of corn EF1α mRNA expression in 20-day-old and 48-day-old corn plants germinated from seeds treated with 50 µg/ml dsRNA for 4 hours. FIG. 22A shows fold change in corn EF1α mRNA expression following treatment with *S. littoralis* EF1α dsRNA for which GFP dsRNA treatment was used as control baseline. Expression values per individual plants were normalized to the median expression of all plants treated with GFP dsRNA. The difference in expression relative to control group had a p-value of 0.016. FIG. 22B shows fold change in corn EF1α mRNA expression following treatment with a mixture of the same dsRNAs as in Figure 22A and PEG-modified carbon nanotubes (CNTP). Expression values per individual plants were normalized to the median expression of all plants treated with GFP dsRNA\CNTP. The difference in expression relative to control group had a p-value of 0.003. FIG. 22C shows fold change in the same corn plants 48 days post seed treatment. Expression values per individual plants were normalized to the median expression of all plants treated with GFP dsRNA\CNTP. The difference in expression relative to control group had a p-value of 0.07.

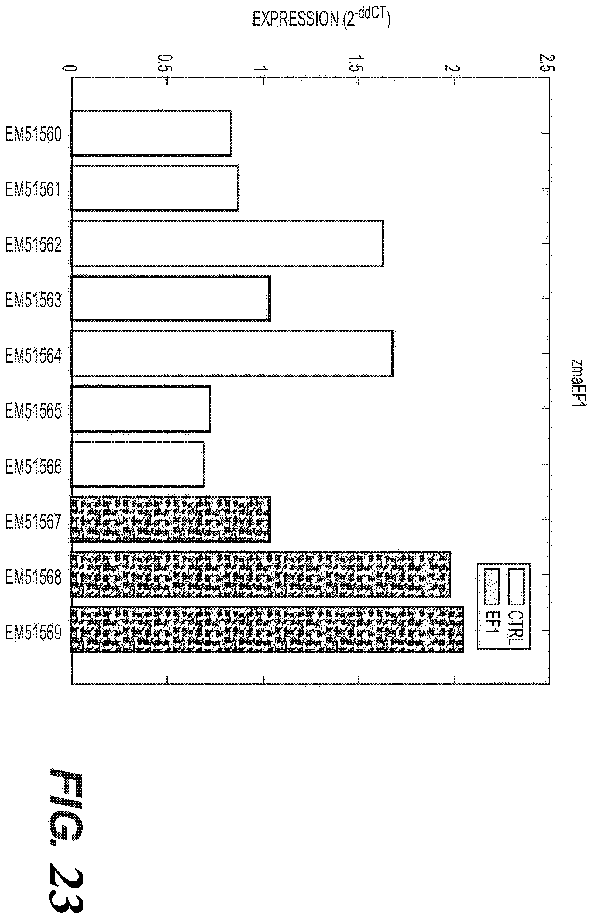

FIG. 23 is a bar graph showing real-time PCR analysis of corn EF1α mRNA expression in nine-week-old corn plants germinated from seeds treated with 132 µg/ml dsRNA derived from *S. littoralis* sequence. Expression values per individual plants were normalized to the median expression of all control plants. The difference in expression relative to control group had a p-value of 0.12.

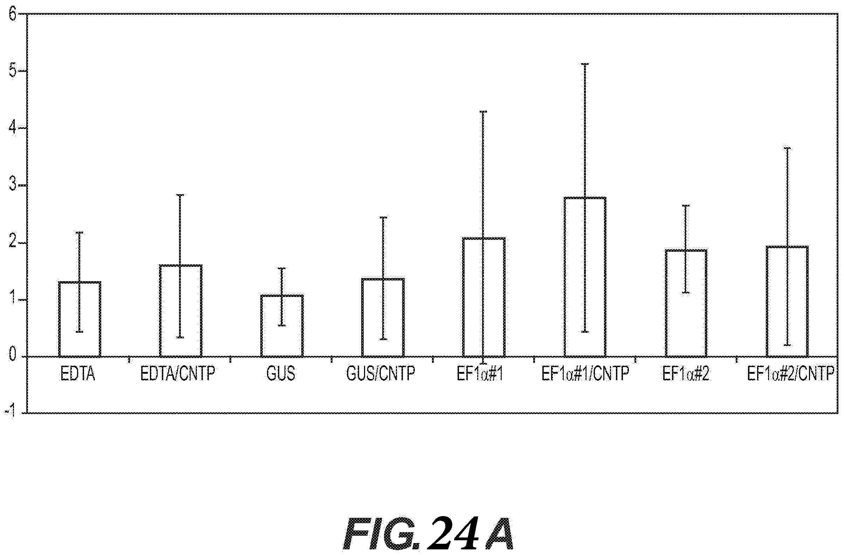

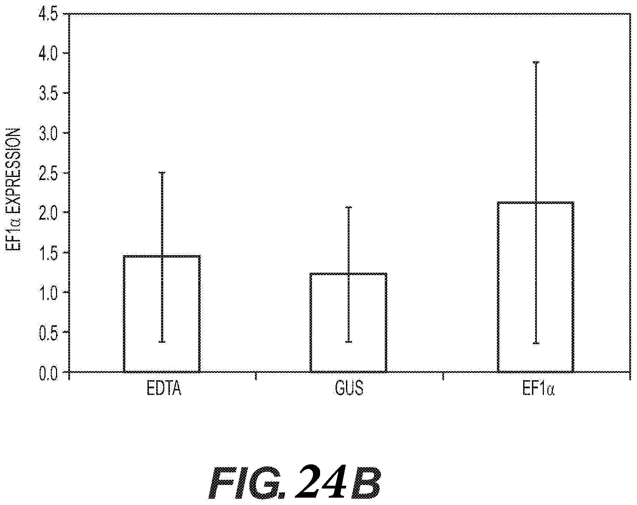

FIGs. 24A-B are bar graphs showing real-time PCR analyses of corn EF1α mRNA expression in six-day-old corn plants germinated from seeds treated with 160 µg/ml dsRNA for 7 hours. FIG. 24A shows fold change in corn EF1α mRNA expression with respect to the GUS dsRNA treatment. FIG. 24B shows the average fold change in corn EF1α mRNA expression for all plants treated with EF1α dsRNA (both dsRNA #1 and #2, with and without CNTP), GUS dsRNA (with and without CNTP) and EDTA (with and without CNTP). Error bars represent standard deviation of the data.

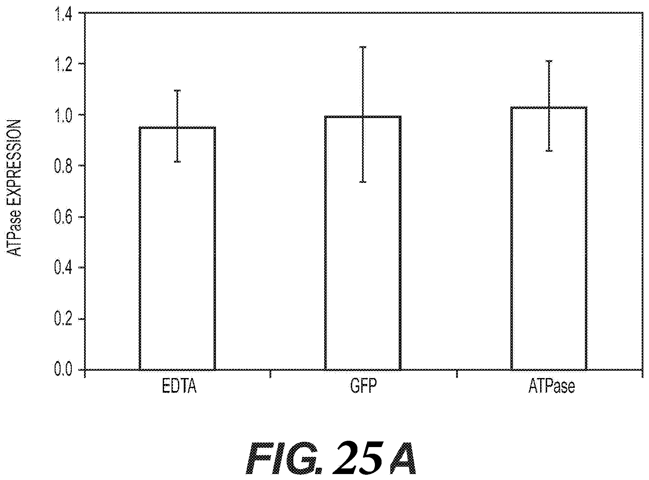

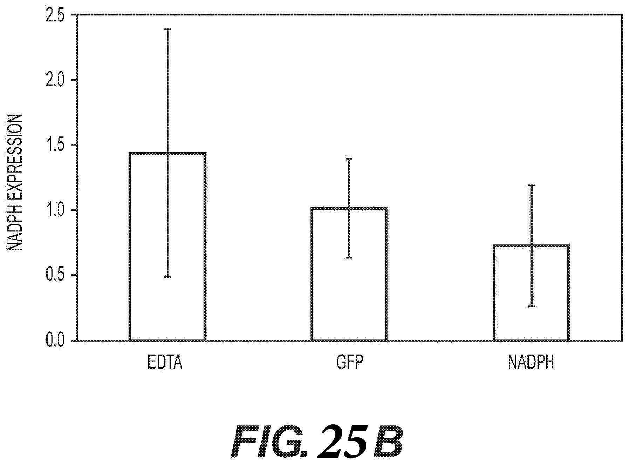

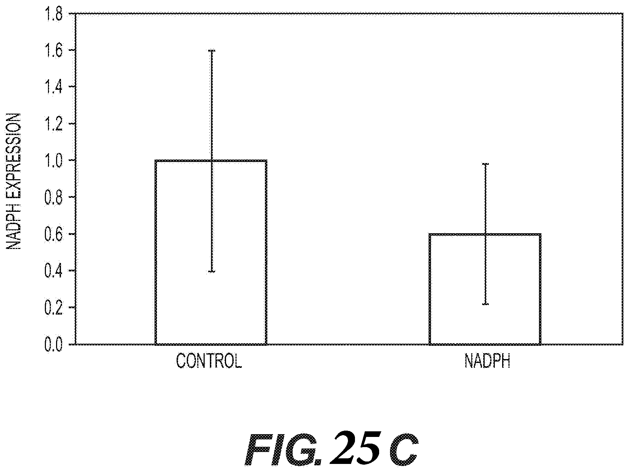

FIGs. 25A-C are bar graphs showing real-time PCR analyses of corn ATPase and NADPH mRNA expression in 27-days old corn plants germinated from seeds treated with 160 µg/ml dsRNA for 2 hours. FIG. 25A shows the average fold change in corn ATPase mRNA expression. FIGs. 25B and 25C shows the average fold change in corn NADPH mRNA expression. Expression values were normalized to the average expression of plants treated with GFP dsRNA (FIGs. 25A and 25B) or to the average expression of EDTA-treated control plants (FIG. 25C). Error bars represent standard deviation of the data.

### DETAILED DESCRIPTION

Unless otherwise stated, nucleic acid sequences in the text of this specification are given, when read from left to right, in the 5' to 3' direction. Nucleic acid sequences may be provided as DNA or as RNA, as specified; disclosure of one necessarily defines the other, as is known to one of ordinary skill in the art. Further, disclosure of a nucleic acid sequence discloses the sequence of its reverse complement, as one necessarily defines the other, as is known by one of ordinary skill in the art. Where a term is provided in the singular, the inventors also contemplate aspects of the invention described by the plural of that term.

With the extensive growth of the world-population and the limited habitats for plant growth and cultivation, there is an urgent need to improve plant yields under these changing conditions. RNAi has emerged as a powerful tool for modulating gene expression which can be used for generating plants with improved stress tolerance. In plants, RNAi is typically performed by producing transgenic plants that comprise a DNA fragment that is transcribed to produce a dsRNA. This dsRNA is then processed into siRNAs that mediate the silencing of target genes, typically by targeting cleavage of the target gene by an RNA Induced Silencing Complex (RISC) or by translational repression. The major technical limitation for this technology is that many important plant crop species are difficult or impossible to transform, precluding the constitutive expression of constructs directing production of dsRNA. Moreover, questions concerning the potential ecological impact of virus-resistant transgenic plants have so far significantly limited their use (Tepfer, 2002, Annu. Rev. Phytopathol. 40, 467-491).

Methods are described of introducing exogenous non-transcribable polynucleotide trigger, for example dsRNA, molecules into plant seeds for modulating gene expression in a plant grown from the seed and/or in a phytopathic organism that feeds on or infects a plant grown from the treated seed. Further descriptions relate to methods of introducing exogenous non-transcribable polynucleotide triggers into plant seeds for controlling insect pest infestation and/or viral infection of plants grown from the seeds. Ingestion of plant material produced from seeds treated with exogenous non-transcribable polynucleotide trigger, for example dsRNA, molecules according to the present embodiments results in the cessation of feeding, growth, development, reproduction, infectivity, and eventually may result in the death of the phytopathogen. The exogenous non-transcribable polynucleotide triggers are designed to silence a target gene of an insect pest.

A novel technology is described for introducing exogenous non-transcribable polynucleotide triggers, for example dsRNA molecules, directly to the plant seed. These non-transcribable polynucleotide trigger, for example dsRNA, molecules enter seeds and start a silencing process, which is continued during the life cycle of the plant, resulting in a plant with an improved trait of interest. The introduced polynucleotide triggers are naked and as such no exogenous transcription regulatory elements are introduced into the plant thus lowering the environmental concerns associated with transgenic plants. In some embodiments, the introduced polynucleotide trigger is naked dsRNA and as such no exogenous transcription regulatory elements are introduced into the plant. In addition, the modified seed can be germinated to generate a plant without the need of going through the laborious and cumbersome steps of tissue culture regeneration.

A delivery system is provided for the delivery of pest control agents to pests through their exposure to a diet containing plant material produced from seeds treated with exogenous non-transcribable polynucleotide trigger, for example dsRNA, molecules according to the present embodiments.

As is illustrated herein below and in the Examples section, which follows, the present embodiments include configuring the conditions necessary to introduce exogenous non-transcribable polynucleotide triggers, for example naked dsRNA into the seeds (see *e.g.,* Example 1). The exogenous non-transcribable polynucleotide trigger, for example naked dsRNA, doesn't integrate into the genome and is highly stable in the plant and in solution (see Examples 2-4). The exogenous non-transcribable polynucleotide trigger, for example naked dsRNA, penetrates through the seed coat (testa) of both monocot and dicot plants and distributes in the endosperm and seed embryo (Examples 5-6). The present embodiments include introducing into seeds exogenous non-transcribable polynucleotide triggers, for example dsRNA, directed to exogenous genes (insect pest genes). These results are reproduced over a number of plants of both monocot and dicot groups. In a further aspect, the present embodiments include introducing into seeds exogenous non-transcribable polynucleotide triggers, for example dsRNA, directed to essential genes of insect pests in a wide range of doses and kinetics which resulted in a significant alteration of gene expression. Interestingly, the dsRNA introduced according to the present embodiments is able to down-regulate essential genes in an insect which feeds on or infects a plant grown from a treated seed (e.g., *Spodoptera littoralis,* Example 7). Thus, the present results are sufficient to show that the present teachings provide a cost-effective treatment of plant seeds to achieve a desired resistance to insect pests.

Provided herein are compositions and methods for inducing systemic regulation (e.g., systemic suppression or silencing) of a target gene in an insect by application to the plant seed of a polynucleotide trigger molecule with a segment in a nucleotide sequence essentially identical to, or essentially complementary to, a sequence of 18 or more contiguous nucleotides in either the target gene or RNA transcribed from the target gene, whereby the composition permeates the interior of the plant seed and induces systemic regulation of the target gene in a phytopathogen of the plant grown from the seed. The polynucleotide trigger molecule can be one or more polynucleotide molecules with a single such segment, multiples of such a segment, multiple different such segments, or combination thereof.

Without being bound by a particular theory, it is suggested that the newly suggested transformation modality and modulation of gene expression is associated with:
(i) Introduction of an exogenous non-transcribable polynucleotide trigger molecule, for example naked dsRNA, into the interior of seeds (as opposed to mere seed coating). The introduction is effected by soaking the seeds in a solution which comprises the exogenous non-transcribable polynucleotide trigger, for example dsRNA, such that the exogenous non-transcribable polynucleotide trigger penetrates through the seed coat or by dipping such that the exogenous non-transcribable polynucleotide trigger coats the seed and penetrates through the coat after sowing;
(ii) Amplification of the signal generated by the exogenous non-transcribable polynucleotide trigger, for example dsRNA; and
(iii) Spreading of the signal throughout the plant.

The first step occurs only once, during and shortly after the initial seed treatment, while the second and third steps occur in a repetitive loop for as long as the silencing signal remains active in the plant.

Without being bound by theory, a suggested unbinding mode of action for the described invention is based on each step:
Introduction of an exogenous non-transcribable polynucleotide trigger, for example dsRNA, into seeds.

A typical mature seed consists of an embryo encapsulated within a maternal seed coat (testa) and an abundant layer of endosperm tissue between the embryo and seed coat. The endosperm serves as a nutrient source for the embryo during seed development, germination and seedling establishment.

Seed germination typically begins with exposure of the seeds to water, which is absorbed by the embryo and endosperm. The endosperm then expands in volume, with the endosperm of some plant species being able to grow several-fold from their original volume. The embryo, which was dormant until this stage, is now released from dormancy and cell division, expansion and differentiation begin. The endosperm feeds the developing embryo until it is developed enough to begin photosynthesis and autotrophic growth.

Based on these known mechanisms of seed germination, two possible modes of action for the initial step of "Introduction of the exogenous non-transcribable polynucleotide trigger, for example dsRNA, into seeds" are suggested:
The exogenous non-transcribable polynucleotide trigger, for example dsRNA, molecules enter the embryo directly, carried by the water-based solution which is used for the seed treatment.

The exogenous non-transcribable polynucleotide trigger, for example dsRNA, molecules enter the endosperm as part of the endosperm's water-absorption process. These molecules then feed the embryo as it develops as part of the nutrient flow from the endosperm during germination and seed development.

Based on the results described in Figures 2-6, it is estimated that a combination of the two options takes place. That is, some of the dsRNA enters the embryo directly and some is retained in the endosperm and feeds the developing embryo during seed germination.

### Amplification of the signal

Once dsRNA molecules enter the embryo, they are recognized and processed by RNAse III-like enzymes such as Dicer or Dicer-like (DCL) enzymes. DCL enzymes process the long dsRNA molecules into short, double strand RNAs (known as siRNAs or shRNAs), which are typically 21-24 nucleotides (nt) long. One of the siRNA strands is typically rapidly degraded and the second one can be incorporated in RISC (RNA Induced Silencing Complex) protein complexes, which contain an Argonaute (AGO) protein. AGO proteins contain a PIWI domain to bind siRNAs and a PAZ domain with RNAse activity. Subsequently, the siRNA/AGO complex identifies an mRNA molecule, which is complementary to the siRNA and results in its silencing by cleavage or translational repression.

The siRNA is then released from the RISC complex and can now act as a primer for an RNA-Dependant RNA Polymerase (RDRP), this is an enzyme which is unique to the plant kingdom and can generate amplification of the silencing signal by generating new dsRNA molecules (secondary siRNA). These newly-synthesized dsRNAs can be processed again as described above, therefore maintaining and amplifying the silencing signal.

### Spreading of the silencing signal

Silencing spreading is a known and well-understood phenomenon in plants. Not wishing to be bound by a particular theory, it is believed that short distance, cell-to-cell spreading occurs through plasmodesmata. This process is thought to be mediated by a 21nt-long siRNA, which is the product of a DCL enzyme. Additionally, systemic spreading is achieved through the phloem across the entire plant from source to sink.

Without being bound by particular theory, it is suggested that in the described methodology, spreading of the silencing signal occurs once the silencing signal begins and is amplified as described above. This may include both short-distance and systematic spreading by various siRNA signal molecules.

There is provided a method of introducing an exogenous non-transcribable polynucleotide trigger, for example naked double-stranded RNA (dsRNA), into a seed, the method comprising contacting the seed with the exogenous non-transcribable polynucleotide trigger, for example naked dsRNA, under conditions which allow penetration of the exogenous non-transcribable polynucleotide trigger, for example naked dsRNA into the seed, thereby introducing the dsRNA into the seed as described in claim 1.

As used herein, the term "target sequence" refers to a nucleotide sequence that occurs in a gene or gene product against which a trigger polynucleotide is directed. In this context, the term "gene" means a locatable region of genomic sequence, corresponding to a unit of inheritance, which includes regulatory regions, such as promoters, enhancers, 5' untranslated regions, intron regions, 3' untranslated regions, transcribed regions, and other functional sequence regions that may exist as native genes or transgenes in a plant genome. Depending upon the circumstances, the term target sequence can refer to the full-length nucleotide sequence of the gene or gene product targeted for suppression or the nucleotide sequence of a portion of the gene or gene product targeted for suppression.

As used herein, the term "derived from" refers to a specified nucleotide sequence that may be obtained from a particular specified source or species, albeit not necessarily directly from that specified source or species.

As used herein, the terms "sequence," "nucleotide sequence" or "polynucleotide sequence" refer to the nucleotide sequence of an RNA molecule or a portion thereof.

The term "polynucleotide" refers to any polymer of mononucleotides that are linked by internucleotide bonds. Polynucleotides may be composed of naturally-occurring ribonucleotides, naturally-occurring deoxyribonucleotides, analogs of naturally-occurring nucleotides (*e.g.,* enantiomeric forms of naturally-occurring nucleotides), or any combination thereof. Where a polynucleotide is single-stranded, its length can be described in terms of the number of nucleotides. Where a polynucleotide is double-stranded, its length can be described in terms of the number of base pairs.

As used herein, the term "non-transcribable polynucleotide" refers to a polynucleotide that does not comprise a complete polymerase II transcription unit.

The term "gene expression" refers to the process of converting genetic information encoded in genomic DNA into RNA (e*.g.,* mRNA, rRNA, tRNA, or snRNA) through transcription of the gene via the enzymatic action of an RNA polymerase, and into protein, through translation of mRNA. Gene expression can be regulated at many stages in the process.

As used herein, the phrases "inhibition of gene expression" or "gene suppression" or "silencing a target gene" and similar terms and phrases refer to the absence or observable reduction in the level of protein and/or mRNA product from the target gene. The consequences of inhibition, suppression, or silencing can be confirmed by examination of the outward properties of a cell or organism or by biochemical techniques.

As used herein, the term "sequence identity," "sequence similarity" or "homology" is used to describe the degree of similarity between two or more nucleotide sequences. The percentage of "sequence identity" between two sequences is determined by comparing two optimally aligned sequences over a comparison window, such that the portion of the sequence in the comparison window may comprise additions or deletions (gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity. A sequence that is identical at every position in comparison to a reference sequence is said to be identical to the reference sequence and vice-versa. An alignment of two or more sequences may be performed using any suitable computer program. For example, a widely used and accepted computer program for performing sequence alignments is CLUSTALW v1.6 (Thompson, et al. Nucl. Acids Res., 22: 4673-4680, 1994).

By "essentially identical" or "essentially complementary" is meant that the bioactive polynucleotide trigger (or at least one strand of a double-stranded polynucleotide or portion thereof, or a portion of a single strand polynucleotide) hybridizes under physiological conditions to the endogenous gene, an RNA transcribed there from, or a fragment thereof, to effect regulation or suppression of the endogenous gene. For example, in some embodiments, a bioactive polynucleotide trigger has 100 percent sequence identity or at least about 83, 84, 85, 86, 87, 88, 89, 90, 91, 92, 93, 94, 95, 96, 97, 98, or 99 percent sequence identity when compared to a sequence of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more contiguous nucleotides in the target gene or RNA transcribed from the target gene. In some embodiments, a bioactive polynucleotide trigger has 100 percent sequence complementarity or at least about 83, 84, 85, 86, 87, 88, 89, 90, 91, 92, 93, 94, 95, 96, 97, 98, or 99 percent sequence complementarity when compared to a sequence of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more contiguous nucleotides in the target gene or RNA transcribed from the target gene. In some embodiments, a bioactive polynucleotide trigger has 100 percent sequence identity with or complementarity to one allele or one family member of a given target gene (coding or non-coding sequence of a gene). In some embodiments, a bioactive polynucleotide trigger has at least about 83, 84, 85, 86, 87, 88, 89, 90, 91, 92, 93, 94, 95, 96, 97, 98, or 99 percent sequence identity with or complementarity to multiple alleles or family members of a given target gene. In some embodiments, a bioactive polynucleotide trigger has 100 percent sequence identity with or complementarity to multiple alleles or family members of a given target gene.

As used herein, nucleic acid sequence molecules are said to exhibit "complete complementarity" when every nucleotide of one of the sequences read 5' to 3' is complementary to every nucleotide of the other sequence when read 3' to 5'. A nucleotide sequence that is completely complementary to a reference nucleotide sequence will exhibit a sequence identical to the reverse complement sequence of the reference nucleotide sequence.

Homologous sequences include both orthologous and paralogous sequences. The term "paralogous" relates to gene-duplications within the genome of a species leading to paralogous genes. The term "orthologous" relates to homologous genes in different organisms due to ancestral relationship.

As used herein, the terms "exogenous polynucleotide" and "exogenous nucleic acid molecule" relative to an organisms refer to a heterologous nucleic acid sequence which is not naturally expressed within that organism, for example a plant. An exogenous nucleic acid molecule may comprise a nucleic acid sequence which is identical or partially homologous to an endogenous nucleic acid sequence of the organism.

As used herein, the terms "endogenous polynucleotide" and "endogenous nucleic acid" refers to nucleic acid sequences that are found in an organism's cell. In certain aspects, an endogenous nucleic acid may be part of the nuclear genome or the plastid genome. As used herein, endogenous nucleic acids do not include viral, parasite or pathogen nucleic acids, for example an endovirus sequence.

As used herein the phrase "naked dsRNA" refers to a dsRNA nucleic acid molecule which is non-transcribable in a plant cell. Thus, the naked dsRNA molecule is not comprised in a nucleic acid expression construct such as a viral vector. According to some embodiments of the invention, the naked dsRNA molecule is not derived from a viral vector. According to some embodiments, the dsRNA is not a product of a natural pathogenic or viral infection. According to some embodiments, the naked dsRNA may comprise regulatory elements for in-vitro transcription, such as the T7 promoter. According to some embodiments of the invention, the naked dsRNA may be modified e.g., chemically modified, to confer higher bioavailability, penetration into the seeds and/or improved shelf-life.

As used herein the term "dsRNA" relates to two strands of anti-parallel polyribonucleic acids held together by base pairing. The dsRNA molecule may be formed by intramolecular hybridization or intermolecular hybridization. In some embodiments, the dsRNA may comprise a single strand of RNA that self-hybridizes to form a hairpin structure having an at least partially double-stranded structure including at least one segment that will hybridize to an RNA transcribed from the gene targeted for suppression. In some embodiments, the dsRNA may comprise two separate strands of RNA that hybridize through complementary base pairing. The RNA strands may or may not be polyadenylated; the RNA strands may or may not be capable of being translated into a polypeptide by a cell's translational apparatus. The two strands can be of identical length or of different lengths provided there is enough sequence homology between the two strands that a double stranded structure is formed with at least 80%, 90%, 95% or 100% complementarity over the entire length. According to an embodiment of the invention, there are no overhangs for the dsRNA molecule. According to another embodiment of the invention, the dsRNA molecule comprises overhangs. According to other embodiments, the strands are aligned such that there are at least 1, 2, or 3 bases at the end of the strands which do not align (*i.e.,* for which no complementary bases occur in the opposing strand) such that an overhang of 1, 2 or 3 residues occurs at one or both ends of the duplex when strands are annealed.

As will be appreciated by one of ordinary skill in the art, a dsRNA molecule of the present disclosure may refer to either strand of the anti-parallel nucleic acids. As will also be appreciated by one of ordinary skill in the art, a dsRNA molecule of the present disclosure includes both a 'sense' and 'antisense' strand and that the sense and antisense strands are reverse complements of each other in a region of base pairing. As used herein the sequence of a dsRNA molecule for regulating a target gene of interest is provided as the 'sense' orientation with respect to the target gene of interest. As used herein, "the reverse complement of a dsRNA molecule for regulating a target gene of interest" refers to a nucleic acid sequence in the 'antisense' orientation.

As mentioned, any dsRNA molecule can be used in accordance with the present teachings. In some embodiments, dsRNA used in the present application is subject to amplification by RNA-Dependant RNA Polymerase (RDRP). Without being limited, dsRNA can be siRNA, shRNA, pre-miRNA, or pri-miRNA.

In one embodiment, the dsRNA in the present application is between 20 and 100 bp, between 25 and 90 bp, between 30 and 80 bp, between 30 and 70 bp, between 30 and 60 bp, or between 30 and 50 bp. In another embodiment, the dsRNA in the present application is about 50 bp. In a further embodiment, the dsRNA comprises 1-base, 2-base or 3-base 5'-overhangs on one or both termini. In another embodiment, the dsRNA does not comprise 1-base, 2-base or 3-base 5'-overhangs on one or both termini. In a further embodiment, the dsRNA comprises 1-base, 2-base or 3-base 3'-overhangs on one or both termini. In another embodiment, the dsRNA does not comprise 1-base, 2-base or 3-base 3'-overhangs on one or both termini.

In another embodiment, the dsRNA in the present application is between 100 and 1,000 bp, between 200 and 900 bp, between 300 and 800 bp, between 400 and 700 bp, between 400 and 600 bp, or between 400 and 500 bp. In another embodiment, the dsRNA in the present application is about 450 bp. In another embodiment, the dsRNA in the present application is about 550 bp. In another embodiment, the dsRNA in the present application is about 650 bp. In another embodiment, the dsRNA in the present application is about 750 bp. In another embodiment, the dsRNA in the present application is about 850 bp. In a further embodiment, the dsRNA comprises 1-base, 2-base or 3-base 5'-overhangs on one or both termini. In another embodiment, the dsRNA does not comprise 1-base, 2-base or 3-base 5'-overhangs on one or both termini. In a further embodiment, the dsRNA comprises 1-base, 2-base or 3-base 3'-overhangs on one or both termini. In another embodiment, the dsRNA does not comprise 1-base, 2-base or 3-base 3'-overhangs on one or both termini.

In one embodiment, the dsRNA in the present application is between 15 and 500 bp, between 15 and 450 bp, between 15 and 400 bp, between 15 and 350 bp, between 15 and 300 bp, between 15 and 250 bp, between 15 and 200 bp, between 15 and 150 bp, between 15 and 100 bp, between 15 and 90 bp, between 15 and 80 bp, between 15 and 70 bp, between 15 and 60 bp, between 15 and 50 bp, between 15 and 40 bp, between 15 and 35 bp, between 15 and 30 bp, or between 15 and 25 bp. In another embodiment, the dsRNA in the present application is at least about 20, 25, 30, 35, 40, 45, 50, 75, 100, 150, 200, 250, 300, 350, 400, 500, 600, 800, 900, 1000 bp long. In a further embodiment, the dsRNA in the present application is between 100 and 1000 bp, between 200 and 1000 bp, between 300 and 1000 bp, between 400 and 1000 bp, between 500 and 1000 bp, between 600 and 1000 bp, between 700 and 1000 bp, between 800 and 1000 bp, or between 900 and 1000 bp.

According to the present teachings, the dsRNA molecules may be naturally occurring or synthetic.

The dsRNA can be a mixture of long and short dsRNA molecules such as, dsRNA, siRNA, siRNA+dsRNA, siRNA+miRNA, or any combination of same. According to a specific embodiment, the dsRNA is a siRNA (100 %). According to a specific embodiment the dsRNA is a siRNA+dsRNA combination in various ratios. Any dsRNA to siRNA ratio can be used for the siRNA+dsRNA combination. For example, a ratio of 1 to 1: one dsRNA mixed with the same sequence after RNAse III treatment. According to another embodiment, the dsRNA to siRNA ratio is 2:1, 1.5:1, 1.3:1, 1:0.01, 1:0.05 or 1:0.1. According to a further embodiment, the dsRNA to siRNA ratio is 2:1 to 1:0.1. According to a specific embodiment, the dsRNA is purified dsRNA (100 %). According to another embodiment, the dsRNA to siRNA ratio is 1:2, 1:5, 1:10, 1:20, or 1:50. According to a further embodiment, the dsRNA is purified siRNA (100%).

The dsRNA molecule can be designed for specifically targeting a target gene of interest, whereby the target gene is an essential gene of an insect pest. It will be appreciated that the dsRNA can be used to down-regulate one or more target genes. If a number of target genes are targeted, a heterogenic composition which comprises a plurality of dsRNA molecules for targeting a number of target genes is used. Alternatively said plurality of dsRNA molecules are separately applied to the seeds (but not as a single composition). According to a specific embodiment, a number of distinct dsRNA molecules for a single target are used, which may be separately or simultaneously (*e.g.,* co-formulation) applied.

The target gene is exogenous to the plant, and the target gene is an insect pest gene. It will further be appreciated that the treatment with the dsRNA may result in an up-regulation of a plant ortholog of the target gene.

Described herein are guidelines for the design and selection of non-transcribable polynucleotide trigger, for example dsRNA, molecules for efficient RNA silencing in phytopathogens, which nourish or depend on a plant for growth/replication and/or survival. Not wishing to be bound by a particular theory, non-transcribable polynucleotide trigger, for example dsRNA, molecules having a sufficient level of homology to an endogenous plant gene allows for degradation and amplification of the primary siRNAs (those which are triggered by Dicer processing) to generate secondary siRNAs formed by DICER-LIKE 4 (DCL4). Such non-transcribable polynucleotide trigger, for example dsRNA, molecules can be selected for having minimal effect on the plant growth and viability. In some embodiments, the secondary siRNAs are of sufficient homology to a gene of a phytopathogen so as allow the degradation of the targeted phytopathogen gene via an RNA interference mode. In some embodiments, a phytopathogen provided with a plant material grown from a seed treated with a non-transcribable polynucleotide trigger, for example dsRNA, molecule as described herein will lose viability either by the induction of growth arrest or death. Such non-transcribable polynucleotide trigger, for example dsRNA molecules are considered as valuable pesticides and can have wide applications in agriculture and horticulture.

Without being bound by particular theory, it is suggested that one mode of modulation of gene expression is associated with: (i) introduction of non-transcribable polynucleotide trigger, for example dsRNA, molecules into the interior of seeds (as opposed to mere seed coating); (ii) amplification of the signal produced from introduction of the non-transcribable polynucleotide trigger, for example dsRNA, molecule; and spreading of the signal throughout the plant. The first step occurs only once, during and shortly after the initial seed treatment, while the second and third steps occur in a repetitive loop for as long as the silencing signal remains active in the plant. As mentioned, introduction of the compositions of the present invention can also be performed to other organs/cells of the plant (as opposed to seeds) using conventional delivery methods such as particle bombardment, grafting, soaking, topical application with a transfer agent and the like. Thus steps (i) and (ii), defined above, are shared also by this mode of administration.

A phytopathogen feeding-on or infecting a plant which comprises any of the dsRNA, primary siRNA or secondary siRNAs which target an essential gene of the phytopathogen will exhibit a growth arrest or death, thereby reducing its injurious effect on the plant or plant product.

Described is a method of introducing naked double-stranded RNA (dsRNA) into a seed, the method comprising contacting the seed with the naked dsRNA under conditions which allow penetration of a nucleic acid sequence having: a homology level to a gene of an insect sufficient to induce degradation of said gene of said insect, wherein said insect depends on said plant for growth and wherein said degradation induces a growth arrest or death of said insect. In some embodiments, the dsRNA targets a gene that contains regions that are poorly conserved between individual insects, or between the insect and the host plant. In certain embodiments it may be desirable to target a gene in an insect that has no known homologs in other organisms, such as the host plant.

Described is a method of introducing naked double-stranded RNA (dsRNA) into a seed, the method comprising contacting the seed with the naked dsRNA under conditions which allow penetration of a nucleic acid sequence having
a homology level to a gene of an insect sufficient to induce degradation of said gene of said insect, wherein said insect depends on said plant for growth and wherein said degradation induces a growth arrest or death of said insect.

In some embodiments, the dsRNA has a homology level to a plant gene sufficient to induce amplification of secondary siRNA products of said dsRNA in a plant cell comprising the dsRNA and wherein altering expression of the plant gene by said dsRNA does not substantially affect any of biomass, vigor or yield of said plant.

According to some embodiments, the nucleic acid sequence of the non-transcribable polynucleotide trigger, for example dsRNA, molecule is selected so as to exhibit sufficient homology to recruit the RDR6 system and generate secondary siRNA transcripts. Such a homology level is typically at least 80 % identity to a gene over at least 25 consecutive bp. According to an alternative embodiment, the homology level of the non-transcribable polynucleotide trigger, for example dsRNA, molecule is at least 85% identity to a plant gene over at least 25 consecutive bp. According to an alternative embodiment, the homology level of the non-transcribable polynucleotide trigger, for example dsRNA, molecule is at least 88% identity to the plant gene over at least 25 consecutive bp. According to an alternative embodiment, the homology level of the non-transcribable polynucleotide trigger, for example dsRNA, molecule is at least 90 % identity to the plant gene over at least 25 consecutive bp of the target gene. According to an alternative embodiment, the homology level of the non-transcribable polynucleotide trigger, for example dsRNA, molecule is at least 92% identity to the plant gene over at least 25 consecutive bp. According to an alternative embodiment, the homology level of the non-transcribable polynucleotide trigger, for example dsRNA, molecule is at least 95% identity to the plant gene over at least 25 consecutive bp. According to an alternative embodiment, the homology level of the non-transcribable polynucleotide trigger, for example dsRNA, molecule is at least 25 consecutive bp.

According to some embodiments, the non-transcribable polynucleotide trigger, for example dsRNA, molecule is at least is 70 bp or longer say 70-700, 70-600, 70-500, 70-400, 70-300, 70-200, 70-100 bp.

According to some embodiments, the non-transcribable polynucleotide trigger, for example dsRNA, molecule comprises a nucleic acid segment at least 70 bp in length which is at least 65 % identical to the gene. According to a specific embodiment, the nucleic acid sequence comprises a nucleic acid segment at least 70 bp in length which is at least 70 % identical (over the entire sequence) to the gene. According to a specific embodiment, the nucleic acid sequence comprises a nucleic acid segment at least 70 bp in length which is at least 75 % identical (over the entire sequence) to the gene. According to a specific embodiment, the nucleic acid sequence comprises a nucleic acid segment at least 70 bp in length which is at least 80% identical (over the entire sequence) to the gene. According to a specific embodiment, the nucleic acid sequence comprises a nucleic acid segment at least 70 bp in length which is at least 85 % identical (over the entire sequence) to the gene. According to a specific embodiment, the nucleic acid sequence comprises a nucleic acid segment at least 70 bp in length which is at least 90 % identical (over the entire sequence) to the gene. According to a specific embodiment, the nucleic acid sequence comprises a nucleic acid segment at least 70 bp in length which is at least 95 % identical (over the entire sequence) to the gene. According to a specific embodiment, the nucleic acid sequence comprises a nucleic acid segment at least 70 bp in length which is 100 % identical (over the entire sequence) to the gene.

In some embodiments, the nucleic acid sequence of the non-transcribable polynucleotide trigger, for example dsRNA, molecule comprises a second nucleic acid segment at least 17 bp in length (over at least 17 consecutive bp) which is at least 85% identical to a gene. According to a specific embodiment, the nucleic acid sequence of the non-transcribable polynucleotide trigger, for example dsRNA, molecule comprises a second nucleic acid segment at least 17 bp in length (over at least 17 consecutive bp) which is at least 90 % identical to a gene. According to a specific embodiment, the nucleic acid sequence of the non-transcribable polynucleotide trigger, for example dsRNA, molecule comprises a second nucleic acid segment at least 17 bp in length (over at least 17 consecutive bp) which is at least 95 % identical to a gene. According to a specific embodiment, the nucleic acid sequence of the non-transcribable polynucleotide trigger, for example dsRNA, molecule comprises a second nucleic acid segment at least 17 bp in length (over at least 17 consecutive bp) which is 100 % identical to a gene.

According to a specific embodiment, the first nucleic acid segment and the second nucleic acid segment overlap (by at least 5 %, 10 %, 20 %, 40 %, 50 % or more). According to a specific embodiment, the overlap is by 5-99 %, 5-95 %, 5-90 %, 5-80 %, 5-70 %, 5-60 %. According to a specific embodiment, the first nucleic acid segment and the second nucleic acid segment are in no overlap.

In some embodiments, the nucleic acid sequence of the non-transcribable polynucleotide trigger, for example dsRNA, molecule is selected having a homology level to a gene of an insect sufficient to induce degradation of the gene of the insect, wherein the phytopathogenic organism depends on the plant for growth and wherein the degradation induces a growth arrest or death of the insect.

Thus, the non-transcribable polynucleotide trigger, for example dsRNA, molecule exhibits at least 80 %, 85 %, 88 %, 90 %, 91 %, 92 %, 93 %, 94 %, 95 %, 96 %, 97 %, 98 %, 99 % or even 100 % identity to the gene of the insect.

In some embodiments, the non-transcribable polynucleotide trigger, for example dsRNA, molecule can be designed for specifically targeting a target gene of interest. It will be appreciated that the non-transcribable polynucleotide trigger, for example dsRNA, molecule can be used to down-regulate one or more target genes of the pinsect. If a number of target genes are targeted, a heterogenic composition which comprises a plurality of non-transcribable polynucleotide trigger, for example dsRNA, molecules for targeting a number of target genes is used. Alternatively said plurality of non-transcribable polynucleotide trigger, for example dsRNA molecules are separately applied to the seeds (but not as a single composition).

In one embodiment, the dsRNA may comprise a target sequence in an intron, exon, 3' UTR, 5' UTR, or a regulatory element of a target gene, or combinations thereof. In one embodiment, the dsRNA of the present application may comprise a target site residing in a promoter.

The dsRNA may be synthesized using any method known in the art, including either enzymatic syntheses or solid-phase syntheses. These are especially useful in the case of short polynucleotide sequences with or without modifications as explained above. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example: Sambrook, J. and Russell, D. W. (2001), "Molecular Cloning: A Laboratory Manual"; Ausubel, R. M. et al., eds. (1994, 1989), "Current Protocols in Molecular Biology," Volumes I-III, John Wiley & Sons, Baltimore, Maryland; Perbal, B. (1988), "A Practical Guide to Molecular Cloning," John Wiley & Sons, New York; and Gait, M. J., ed. (1984), "Oligonucleotide Synthesis"; utilizing solid-phase chemistry, *e.g.,* cyanoethyl phosphoramidite followed by deprotection, desalting, and purification by, for example, an automated trityl-on method or HPLC.

As mentioned, the naked dsRNA molecule is directly contacted with the seed.

The seed may be of any plant, such as of the Viridiplantae super family including monocotyledon and dicotyledon plants. Other plants are listed herein below. According to an embodiment of the invention, the cells of the plant comprise RNA dependent RNA polymerase activity and the target RNA molecule of the dsRNA to ensure amplification of the dsRNA.

The term "plant" as used herein encompasses whole plants, ancestors and progeny of the plants and plant parts, including seeds, shoots, stems, roots (including tubers), and isolated plant cells, tissues and organs. The plant may be in any form including suspension cultures, embryos, meristematic regions, callus tissue, leaves, gametophytes, sporophytes, pollen, and microspores. It will be appreciated, that the plant or seed thereof may be transgenic plants.

As used herein the phrase "plant cell" refers to plant cells which are derived and isolated from disintegrated plant cell tissue or plant cell cultures. Plant cells may be reproductive cells (*i.e.,* cells from a tissue contributing directly to the sexual reproduction of a plant) or non-reproductive cells (*i.e.,* cells from a tissue not involved in the sexual reproduction of a plant). Plant cells may be cells that are capable of regenerating into a whole plant or cells that cannot regenerate into a whole plant, for example, enucleated mature sieve tube cells.

As used herein the phrase "plant cell culture" refers to any type of native (naturally occurring) plant cells, plant cell lines and genetically modified plant cells, which are not assembled to form a complete plant, such that at least one biological structure of a plant is not present. Optionally, the plant cell culture of this aspect of the present invention may comprise a particular type of a plant cell or a plurality of different types of plant cells. It should be noted that optionally plant cultures featuring a particular type of plant cell may be originally derived from a plurality of different types of such plant cells.

Any commercially or scientifically valuable plant is envisaged in accordance with some embodiments of the invention. Plants that are particularly useful in the methods of the invention include all plants which belong to the super family Viridiplantae, in particular monocotyledonous and dicotyledonous plants including a fodder or forage legume, ornamental plant, food crop, tree, or shrub selected from the list comprising *Acacia spp., Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chacoomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Dibeteropogon amplectens, Dioclea spp, Dolichos spp., Dorycnium rectum, Echinochloa pyramidalis, Ehraffia spp., Eleusine coracana, Eragrestis spp., Erythrina spp., Eucalyptus spp., Euclea schimperi, Eulalia vi*/*losa, Pagopyrum spp., Feijoa sellowlana, Fragaria spp., Flemingia spp, Freycinetia banksli, Geranium thunbergii, GinAgo biloba, Glycine javanica, Gliricidia spp, Gossypium hirsutum, Grevillea spp., Guibourtia coleosperma, Hedysarum spp., Hemaffhia altissima, Heteropogon contoffus, Hordeum vulgare, Hyparrhenia rufa, Hypericum erectum, Hypeffhelia dissolute, Indigo incamata, Iris spp., Leptarrhena pyrolifolia, Lespediza spp., Lettuca spp., Leucaena leucocephala, Loudetia simplex, Lotonus bainesli, Lotus spp., Macrotyloma axillare, Malus spp., Manihot esculenta, Medicago saliva, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativam, Podocarpus totara,* *Pogonarthria fleckii, Pogonaffhria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys vefficillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, maize, wheat, barley, rye, oat, peanut, pea, lentil and alfalfa, cotton, rapeseed, canola, pepper, sunflower, tobacco, eggplant, eucalyptus, a tree, an ornamental plant, a perennial grass and a forage crop. Alternatively algae and other non-Viridiplantae can be used for the methods of the present invention.

According to some embodiments of the invention, the plant used by the method of the invention is a crop plant including, but not limited to, cotton, Brassica vegetables, oilseed rape, sesame, olive tree, palm oil, banana, wheat, corn or maize, barley, alfalfa, peanuts, sunflowers, rice, oats, sugarcane, soybean, turf grasses, barley, rye, sorghum, sugar cane, chicory, lettuce, tomato, zucchini, bell pepper, eggplant, cucumber, melon, watermelon, beans, hibiscus, okra, apple, rose, strawberry, chili, garlic, pea, lentil, canola, mums, *Arabidopsis,* broccoli, cabbage, beet, quinoa, spinach, squash, onion, leek, tobacco, potato, sugarbeet, papaya, pineapple, mango, *Arabidopsis* thaliana, and also plants used in horticulture, floriculture or forestry, such as, but not limited to, poplar, fir, eucalyptus, pine, an ornamental plant, a perennial grass and a forage crop, coniferous plants, moss, algae, as well as other plants listed in World Wide Web (dot) nationmaster (dot) com/encyclopedialPlantae.

According to a specific embodiment, the plant is selected from the group consisting of corn, rice, wheat, tomato, cotton and sorghum.

According to a specific embodiment, the seed is an uncoated or fresh seed that hasn't been subjected to chemical/physical treatments.

In some embodiments, washing of the seeds is effected for 30 minutes to 4 hours. Other examples of wash ranges are 1 minute to 10 minutes, 10 minutes to 30 minutes. According to some embodiments, washing of the seeds can be as short as 5, 10, 20, 30, 45, or 60 seconds. The wash solution may include a weak detergent such as Tween-20. The concentration of the detergent may be 0.01-0.2% or 0.2-1%. According to another embodiment, the detergent concentration can be about 0.001%, 0.005%, 0.01%, 0.05%, 0.1%, 0.5%, 1% or higher.

The seed is subjected to priming or washing prior to contacting with the dsRNA.

As used herein the term "priming" refers to controlling the hydration level within seeds so that the metabolic activity necessary for germination can occur but radicle emergence is prevented. Different physiological activities within the seed occur at different moisture levels (Leopold and Vertucci, 1989; Taylor, 1997). The last physiological activity in the germination process is radicle emergence. The initiation of radicle emergence requires a high seed water content. By limiting seed water content, all the metabolic steps necessary for germination can occur without the irreversible act of radicle emergence. Prior to radicle emergence, the seed is considered desiccation tolerant, thus the primed seed moisture content can be decreased by drying. After drying, primed seeds can be stored until time of sowing.

Several different priming methods are used commercially. Among them, liquid or osmotic priming and solid matrix priming appear to have the greatest following (Khan *et al.,* 1991).

According to an embodiment of the invention, priming is effected in the presence of salt, a chelating agent, polyethylene glycol or a combination of same (e.g., chelating agent and salt).

Alternatively, priming is effected in the presence of water such as deionized water or double deionized water. According to a specific embodiment, the priming is effected in the presence of 100% ddW.

Several types of seed priming are commonly used:
Osmopriming (osmoconditioning) - is the standard priming technique. Seeds are incubated in well aerated solutions with a low water potential, and afterwards washes and dried. The low water potential of the solutions can be achieved by adding osmotica like mannitol, polyethyleneglycol (PEG) or salts like KCI.

Hydropriming (drum priming) - is achieved by continuous or successive addition of a limited amount of water to the seeds. A drum is used for this purpose and the water can also be applied by humid air. 'On-farm steeping' is a cheap and useful technique that is practiced by incubating seeds (cereals, legumes) for a limited time in warm water.

Matrixpriming (matriconditioning) - is the incubation of seeds in a solid, insoluble matrix (vermiculite, diatomaceous earth, cross-linked highly water-absorbent polymers) with a limited amount of water. This method confers a slow imbibition.

Pregerminated seeds - is only possible with a few species. In contrast to normal priming, seeds are allowed to perform radicle protrusion. This is followed by sorting for specific stages, a treatment that reinduces desiccation tolerance, and drying. The use of pregerminated seeds causes rapid and uniform seedling development.

Thus, according to one embodiment, the seeds are primed seeds.

Of note, it may be possible that the seeds are treated with water (double-distilled water, ddW), prior to contacting with the dsRNA without effecting any priming on the seeds. For instance, treatment for a short while with water (*e.g.,* 30 seconds to 1 hour, 30 seconds to 0.5 hour, 30 seconds to 10 minutes, 30 seconds to 5 minutes or 45 seconds to 5 minutes). According to some embodiments, treatment with water can be as short as 5, 10, 20, or 30 seconds.

It will be appreciated that the non-transcribable polynucleotide trigger, for example dsRNA, molecule can be comprised in water (*e.g.,* tap water, distilled water or double distilled water) *i.e.,* free of any of the above mentioned priming effective concentration of salts, a chelating agents, polyethylene glycol or combinations of same (*e.g.,* chelating agent and salt). In some embodiments, the non-transcribable polynucleotide trigger, for example dsRNA, molecule is provided to the seed in a buffer solution, such as EDTA.

A non-limiting method of introducing the dsRNA into the seed is provided in Example 1, which is considered as an integral part of the specification.

The temperature at the washing/priming and drying steps may be the same or differ.

According to one embodiment, the washing/priming is effected at 4-28 °C.

According to one embodiment, the priming/washing solution or the dsRNA containing solution is devoid of a solid carrier.

According to one embodiment, the priming/washing solution or the dsRNA containing solution is devoid of a transferring agent such as a surfactant or a salt.

According to a further embodiment of the invention, the seeds subject to contacting with the dsRNA molecule are washed in order to remove agents, to which the seeds have been subjected, such as a pesticide, a fungicide, an insecticide, a fertilizer, a coating agent and a coloring agent.

Thus, according to one embodiment, the seeds (prior to treatment with dsRNA) are substantially free (*i.e.*, do not comprise effective amounts) of pesticide, a fungicide, an insecticide, a fertilizer, a coating agent and a coloring agent.

The seeds are then subjected to drying.

According to one embodiment, the drying is effected at 20-37 °C, 20-30 °C, 22-37 °C, 15-22 °C or 20-25 °C for 10-20 hours, 10-16 hours or even 2-5 hours.

Various considerations are to be taken when calculating the concentration of the dsRNA in the contacting solution.

These are dependent on at least one of seed size, seed weight, seed volume, seed surface area, seed density and seed permeability.

For example, related to seed size, weight, volume and surface area, it is estimated that corn seeds will require longer treatment than *Arabidopsis* and tomato seeds. Regarding permeability and density, it is estimated that wheat seeds will require longer treatments at higher concentrations than tomato seeds.

Examples of concentrations of dsRNA in the treating solution include, but are not limited to, 0.01-0.3 µg/µl, 0.01-0.15 µg/µl, 0.04-0.15 µg/µl, 0.1-100 µg/µl; 0.1-50 µg/µl, 0.1-10, µg/µl, 0.1-5 µg/µl, 0.1-1 µg/µl, 0.1-0.5 µg/µl, 0.15-0.5 µg/µl , 0.1-0.3 µg/µl, 0.01-0.1 µg/µl, 0.01-0.05 µg/µl, 0.02-0.04 µg/µl , 0.001-0.02 µg/µl. According to a specific embodiment, the concentration of the dsRNA in the treating solution is 0.01-0.15 or 0.04-0.15 µg/µl.

In one embodiment, the dsRNA concentration in the treating solution is 0.01-0.3 µg/ml, 0.01-0.15 µg/ml, 0.04-0.15 µg/ml, 0.1-100 µg/ml; 0.1-50 µg/ml, 0.1-10 µg/ml, 0.1-5 µg/ml, 0.1-1 µg/ml, 0.1-0.5 µg/ml, 0.15-0.5 µg/ml , 0.1-0.3 µg/ml, 0.01-0.1 µg/ml, 0.01-0.05 µg/ml, 0.02-0.04 µg/ml , or 0.001-0.02 µg/ml.

In another embodiment, the dsRNA concentration in the treating solution is about 5-10 µg/ml, 10-15 µg/ml, 15-20 µg/ml, 20-25 µg/ml; 25-30 µg/ml, 30-35 µg/ml, 35-40 µg/ml, 40-45 µg/ml, 45-50 µg/ml, 50-55 µg/ml, 55-60 µg/ml, 60-65 µg/ml, 65-70 µg/ml, 70-75 µg/ml, 75-80 µg/ml, 80-85 µg/ml, 85-90 µg/ml, 90-95 µg/ml, 95-100 µg/ml, 100-105 µg/ml, 105-110 µg/ml, 110-115 µg/ml, 115-120 µg/ml, 120-125 µg/ml; 125-130 µg/ml, 130-135 µg/ml, 135-140 µg/ml, 140-145 µg/ml, 145-150 µg/ml, 150-155 µg/ml, 155-160 µg/ml, 160-165 µg/ml, 165-170 µg/ml, 170-175 µg/ml, 175-180 µg/ml, 180-185 µg/ml, 185-190 µg/ml, 190-195 µg/ml, 195-200 µg/ml, 200-210 µg/ml, 210-220 µg/ml, 220-230 µg/ml, 230-240 µg/ml, 240-250 µg/ml, 250-260 µg/ml, 260-270 µg/ml, 270-280 µg/ml, 280-290 µg/ml, 290-300 µg/ml, 300-310 µg/ml, 310-320 µg/ml, 320-330 µg/ml, 330-340 µg/ml, 340-350 µg/ml, 350-360 µg/ml, 360-370 µg/ml, 370-380 µg/ml, 380-390 µg/ml, 390-400 µg/ml, 400-410 µg/ml, 410-420 µg/ml, 420-430 µg/ml, 430-440 µg/ml, 440-450 µg/ml, 450-460 µg/ml, 460-470 µg/ml, 470-480 µg/ml, 480-490 µg/ml, or about 490-500 µg/ml.

In another embodiment, the dsRNA concentration in the treating solution is 0.0001-3 µg/µl, 0.0001-2.5 µg/µl, 0.0001-2 µg/µl, 0.0001-1.5 µg/µl, 0.0001-1 µg/µl, 0.0001-0.9 µg/µl, 0.0001-0.8 µg/µl, 0.0001-0.7 µg/µl, 0.0001-0.6 µg/µl, 0.0001-0.5 µg/µl, 0.0001-0.4 µg/µl, 0.0001-0.3 µg/µl, 0.0001-0.2 µg/µl, 0.0001-0.1 µg/µl, 0.0001-0.05 µg/µl, 0.0001-0.02 µg/µl, 0.0001-0.01 µg/µl, 0.0001-0.005 µg/µl, 0.0001-0.001 µg/µl, or 0.0001-0.0005 µg/µl.

In another embodiment, the dsRNA concentration in the treating solution is 0.0001-3 µg/µl, 0.0005-3 µg/µl, 0.001-3 µg/µl, 0.005-3 µg/µl, 0.01-3 µg/µl, 0.05-3 µg/µl, 0.1-3 µg/µl, 0.2-3 µg/µl, 0.3-3 µg/µl, 0.4-3 µg/µl, 0.5-3 µg/µl, 0.6-3 µg/µl, 0.7-3 µg/µl, 0.8-3 µg/µl, 0.9-3 µg/µl, 1-3 µg/µl, or 2-3 µg/µl.

In another embodiment, the dsRNA concentration in the treating solution is 0.0001-3 µg/µl, 0.0005-2.5 µg/µl, 0.001-2 µg/µl, 0.005-1.5 µg/µl, 0.01-1 µg/µl, 0.05-0.5 µg/µl, 0.1-0.4 µg/µl, or 0.2-0.3 µg/µl.

According to a specific embodiment, the contacting with the dsRNA is effected in the presence of a chelating agent such as EDTA or another chelating agent such as DTPA (0.01-0.1 mM).

In some embodiments, the treating solution may comprise a transferring agent such as a surfactant or a salt. Examples of such transferring agents include but are not limited salts such as sodium or lithium salts of fatty acids (such as tallow or tallowamines or phospholipids lipofectamine or lipofectin (1-20 nM, or 0.1-1 nM)) and organosilicone surfactants. Other useful surfactants include organosilicone surfactants including nonionic organosilicone surfactants, e.g., trisiloxane ethoxylate surfactants or a silicone polyether copolymer such as a copolymer of polyalkylene oxide modified heptamethyl trisiloxane and allyloxypolypropylene glycol methylether (commercially available as SilwetTM L-77 surfactant having CAS Number 27306-78-1 and EPA Number: CAL.REG.NO. 5905-50073-AA, currently available from Momentive Performance Materials, Albany, N.Y.).

In some embodiments, the treating solution may comprise a physical agent. Examples of physical agents include: (a) abrasives such as carborundum, corundum, sand, calcite, pumice, garnet, and the like, (b) nanoparticles such as carbon nanotubes and (c) a physical force. Carbon nanotubes are disclosed by Kam et al. (2004) J. Am. Chem. Soc., 126 (22):6850-6851, Liu et al. (2009) Nano Lett., 9(3):1007-1010, and Khodakovskaya et al. (2009) ACS Nano, 3(10):3221-3227. Physical force agents can include heating, chilling, the application of positive pressure, or ultrasound treatment. Agents for laboratory conditioning of a plant to permeation by polynucleotides include, *e.g.,* application of a chemical agent, enzymatic treatment, heating or chilling, treatment with positive or negative pressure, or ultrasound treatment. Agents for conditioning plants in a field include chemical agents such as surfactants and salts.

Contacting of the seeds with the dsRNA can be effected using soaking.

As used herein "an effective amount" refers to an amount of dsRNA which is sufficient to down regulate the target gene by at least 20%, 30%, 40%, 50%, or more, say 60%, 70%, 80%, 90% or more even 100%. The effective amount can be a result of the formation of amplification in the plant or the phytopathogen.

Contacting may be effected by soaking (*i.e.*, inoculation) so that shaking the seeds with the treating solution may improve penetration and soaking and therefore reduce treatment time. Shaking is typically performed at 50-150 RPM and depends on the volume of the treating solution. Shaking may be effected for 4-24 hours (1-4 hours, 10 minutes to 1 hour or 30 seconds to 10 minutes). The present teachings further envisage short incubation time such as up to 10 minutes. Examples include but are not limited to 30 seconds to 7 minutes, to 30 seconds to 5 minutes, to 30 seconds to 3 minutes, to 30 seconds to 2 minutes, to 30 seconds to 1 minute, 1 minute to 10 minutes or to 1 minute to 5 minutes.

In one embodiment, the incubation time may be between 1 and 60, between 2 and 60, between 5 and 60, between 10 and 60, between 20 and 60, between 30 and 60, between 40 and 60, between 50 and 60, between 1 and 50, between 1 and 40, between 1 and 30, between 1 and 20, between 1 and 10, between 1 and 5, between 5 and 50, between 10 and 40, and between 20 and 30 seconds.

In another embodiment, the incubation time may be between 1 and 60, between 2 and 60, between 5 and 60, between 10 and 60, between 20 and 60, between 30 and 60, between 40 and 60, between 50 and 60, between 1 and 50, between 1 and 40, between 1 and 30, between 1 and 20, between 1 and 10, between 1 and 5, between 5 and 50, between 10 and 40, and between 20 and 30 minutes.

Following contacting, preferably prior to breaking of seed dormancy and embryo emergence, the seeds may be subjected to treatments (e.g., coating) with the above agents (*e.g.,* pesticide, fungicide etc.).

After contacting with the treatment solution, the seeds may be subjected to drying for up to 30 hours at 25-37 °C. For example, the seeds may be dried for 16 hours at 30 °C.

As used herein the term "isolated" refers to separation from the natural physiological environment. In the case of seed, the isolated seed is separated from other parts of the plant. In the case of a nucleic acid molecule (*e.g.,* dsRNA) separated from the cytoplasm.

The dsRNA is not expressed from the plant genome, thereby not being an integral part of the genome.

Methods of measuring the localization of RNA molecules in the seed are well known in the art. The use of siGlo as described in the Examples section is an example for such.

As used herein the term "higher" refers to at least about 3%, 5%, 7%, 10%, 15%, 20%, 25%, 30%, 50%, 60%, 70%, 80%, 90% or even a few folds higher.

According to an alternative or an additional embodiment, there is provided an isolated seed comprising an exogenous dsRNA, wherein the plant seed is devoid of a heterologous promoter for driving expression of said exogenous dsRNA, wherein a spatial distribution of said exogenous dsRNA and/or siRNA maturing there from is altered in the seed as compared to same in a transgenic seed recombinantly expressing said exogenous dsRNA.

The term "recombinantly expressing" refers to an expression from a nucleic acid construct.

Methods of qualifying successful introduction of the dsRNA include but are not limited to, RT-PCR (*e.g.,* quantifying the level of the target gene or the naked dsRNA), phenotypic analysis such as biomass, vigor, yield and stress tolerance, root architecture, leaf dimensions, grain size and weight, oil content, cellulose, as well as cell biology techniques.

According to some embodiments, an alteration of the expression level of the plant ortholog of the insect pest gene targeted by the seed treatment, as described herein, is observed. See for instance Examples 29 and 30 of the Examples section which follows.

Seeds may be stored for 1 day to several months prior to planting (*e.g.,* at 4-10 °C).

The resultant seed can be germinated in the dark so as to produce a plant.

As used herein "devoid of a heterologous promoter for driving expression of the dsRNA" means that the plant or plant cell doesn't include a cis-acting regulatory sequence (*e.g.,* heterologous) transcribing the dsRNA in the plant. As used herein the term "heterologous" refers to exogenous, not-naturally occurring within the native plant cell (such as by position of integration, or being non-naturally found within the plant cell). Thus the isolated seed in the absence of a heterologous promoter sequence for driving expression of the dsRNA in the plant, comprises a homogenic (prior to amplification) or heterogenic (secondary siRNAs, following amplification) population of plant non-transcribable dsRNA.

Examples 7 and 8-25 of the Examples section which follows, describes implementation the present teachings towards conferring resistance to *Spodoptera littoralis.* Examples 26 and 27 of the Examples section which follows, describes implementation the present teachings towards conferring resistance to *Coleopteran* pests.

As used herein the term "improving" or "increasing" refers to at least about 2%, at least about 3%, at least about 4%, at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, at least about 90% or greater increase in NUE, in tolerance to stress, in yield, in biomass or in vigor of a plant, as compared to a native or wild-type plants [*i.e.*, isogenic plants (not grown from seeds treated with dsRNA) of the present embodiments].

The target gene of the dsRNA may not be an endogenous plant gene but rather a gene exogenous to the plant, such as a gene of an insect which feeds on the plant or depends thereon for growth/replication and/or survival. In some embodiments, the target gene is an essential gene of an insect pest.

As used herein, the term "phytopathogen" refers to an organism that benefits from an interaction with a plant, and has a negative effect on that plant. The term "phytopathogen" includes insects, arachnids, crustaceans, fungi, bacteria, viruses, nematodes, flatworms, roundworms, pinworms, hookworms, tapeworms, trypanosomes, schistosomes, botflies, fleas, ticks, mites, and lice and the like that may ingest or contact one or more cells, tissues, or fluids produced by a plant.

The methods described herein can be used to generate a plant that is resistant to one or more phytopathogens, wherein the phytopathogen is an insect pest. When an insect is the target pest for the present invention, such pests include but are not limited to: from the order *Lepidoptera,* for example, *Acleris spp., Adoxophyes spp., Aegeria spp., Agrotis spp., Alabama argillaceae, Amylois spp., Anticarsia gemmatalis, Archips spp, Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantiria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia Nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni* and *Yponomeuta spp.;* from the order *Coleoptera,* for example, *Agriotes spp., Anthonomus spp., Atomaria linearis, Chaetocnema tibialis, Cosmopolites spp., Curculio spp., Denrmestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp.* and *Trogoderma spp.;* from the order *Orthoptera,* for example, *Blatta spp., Blattella spp., Gryllotalpa spp., Leucophaea maderae, Locusta spp., Periplaneta ssp., and Schistocerca spp.;* from the order *Isoptera,* for example, *Reticulitemes ssp;* from the order *Psocoptera,* for example, *Liposcelis spp.;* from the order *Anoplura,* for example, *Haematopinus spp., Linognathus spp., Pediculus spp., Pemphigus spp.* and *Phylloxera spp.;* from the order *Mallophaga,* for example, *Damalinea spp.* and *Trichodectes spp.;* from the order *Thysanoptera,* for example, *Franklinella spp., Hercinothrips spp., Taeniothrips spp., Thrips palmi, Thrips tabaci* and *Scirtothrips aurantii;* from the order *Heteroptera,* for example, *Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp., Triatoma spp., Miridae family spp.* such as *Lygus hesperus* and *Lygus lineoloris, Lygaeidae family spp.* such as *Blissus leucopterus,* and *Pentatomidae family spp.;* from the order *Homoptera,* for example, *Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lacanium corni, Lepidosaphes spp., Macrosiphus spp., Myzus spp., Nehotettix spp., Nilaparvata spp., Paratoria spp., Pemphigus spp., Planococcus spp., Pseudaulacaspis spp., Pseudococcus spp., Psylla ssp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae* and *Unaspis citri;* from the order *Hymenoptera,* for example, *Acromyrmex, Atta spp., Cephus spp., Diprion spp., Diprionidae, Gilpinia polytoma, Hoplocampa spp., Lasius spp., Monoimorium pharaonis, Neodiprion spp, Solenopis spp.* and *Vespa ssp.;* from the order *Diptera,* for example, *Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomysa spp., Lucilia spp., Melanagromyza spp., Musca ssp., Oestrus spp., Orseolia spp., Oscinella frit, Pegomyia hyoscyami, Phorbia spp., Rhagoletis pomonella, Sciara spp., Stomoxys spp., Tabanus spp., Tannia spp.* and *Tipula spp.,* from the order *Siphonaptera,* for example, *Ceratophyllus spp. und Xenopsylla cheopis* and from the order *Thysanura,* for example, *Lepisma saccharina.* Thus, according to one embodiment, there is provided a method of inhibiting expression of a target gene in an insect, the method comprising providing (*e.g.,* feeding or contacting under infecting conditions) to the insect the plant as described herein (at least part thereof includes the naked dsRNA), thereby inhibiting expression of a target gene in the insect. In some embodiments, the target gene is an "essential gene." As used herein, the term "essential gene" refers to a gene of an insect that is essential for its survival or reproduction. In some embodiments, the target gene is expressed in the insect gut, for example, V-ATPase. In some embodiments, the target gene is involved in the growth, development, and reproduction of an insect. Examples of such genes include, but are not limited to, CHD3 gene and a beta-tubulin gene.

Several embodiments relate to a method of inhibiting expression of a target gene in an insect pest, the method comprising providing (*e.g.,* feeding) to the insect pest a plant grown from a seed treated with an exogenous dsRNA as described herein, thereby inhibiting expression of the target gene in the insect pest. Insects that may cause damage and disease in plants belong to three categories, according to their method of feeding: chewing, sucking and boring. Major damage is caused by chewing insects that eat plant tissue, such as leaves, flowers, buds and twigs. Examples from this large insect category include beetles and their larvae (grubs), web-worms, bagworms and larvae of moths and sawflies (caterpillars). By comparison, sucking insects insert their mouth parts into the tissues of leaves, twigs, branches, flowers or fruit and suck out the plant's juices. Typical examples of sucking insects include but are not limited to aphids, mealy bugs, thrips and leaf-hoppers. Damage caused by these pests is often indicated by discoloration, drooping, wilting and general lack of vigor in the affected plant.

Several embodiments relate to a method of providing resistance to an insect pest, the method comprising growing a plant from a seed treated with an exogenous dsRNA as described herein. In some embodiments, the insect pest is selected from the orders *Coleoptera, Lepidoptera, Diptera, Orthoptera, Heteroptera, Ctenophalides, Arachnidiae,* and *Hymenoptera.* In some embodiments, the insect pest is a beetle or larvae. According to a specific embodiment, the phytopathogen is prodentia of the family *Noctuidae e.g., Spodoptera littoralis.*

Insect pests causing plant disease include those from the families of, for example, *Apidae, Curculionidae, Scarabaeidae, Tephritidae, Tortricidae,* amongst others.

The target gene of the insect encodes a product essential to the viability and/or infectivity of the insect, therefore its down-regulation (by the naked dsRNA) results in a reduced capability of the insectto survive and infect host cells. Hence, such down-regulation results in a "deleterious effect" on the maintenance viability and/or infectivity of the insect, in that it prevents or reduces the insect's ability to feed off and survive on nutrients derived from host cells. By virtue of this reduction in the insect's viability and/or infectivity, resistance and/or enhanced tolerance to infection by an insect is facilitated in the cells of the plant. Genes in the insect may be targeted at the mature (adult), immature (juvenile) or embryo stages.

Examples of genes essential to the viability and/or infectivity of the insect are provided herein. Such genes may include genes involved in development and reproduction, *e.g.* transcription factors (see, *e.g.* Xue *et al.,* 1993; Finney *et al.,* 1988), cell cycle regulators such as wee-1 and ncc-1 proteins (see, *e.g.* Wilson *et al.,* 1999; Boxem *et al.,* 1999) and embryo-lethal mutants (see, *e.g.* Schnabel *et al.,* 1991); proteins required for modeling such as collagen, ChR3 and LRP-1 (see, *e.g.* Yochem *et al.,* 1999; Kostrouchova *et al.,* 1998; Ray *et al.,* 1989); genes encoding proteins involved in the motility/nervous system, *e.g.* acetycholinesterase (see, *e.g.* Piotee *et al.,* 1999; Talesa *et al.,* 1995; Arpagaus *et al.,* 1998), ryanodine receptor such as *unc-68* (see, *e.g.* Maryon *et al.,* 1998; Maryon *et al.,* 1996) and glutamate-gated chloride channels or the avermeetin receptor (see, *e.g.,* Cully *et al.,* 1994; Vassilatis *et al.,* 1997; Dent *et al.,* 1997); hydrolytic enzymes required for deriving nutrition from the host, *e.g.* serine proteinases such as HGSP-1 and HGSP-III (see, *e.g.* Lilley *et al.,* 1997); parasitic genes encoding proteins required for invasion and establishment of the feeding site, *e.g.* cellulases (see, *e.g.* de Boer *et al.,* 1999; Rosso *et al.,* 1999) and genes encoding proteins that direct production of stylar or amphidial secretions such as sec-1 protein (see, *e.g.* Ray *et al.,* 1994; Ding *et al.,* 1998); genes encoding proteins required for sex or female determination, *e.g.* tra-1, tra-2 and egl-1, a suppressor of ced9 (see, *e.g.* Hodgkin, 1980; Hodgkin, 1977; Hodgkin, 1999; Gumienny *et al.,* 1999; Zarkower *et al.,* 1992); and genes encoding proteins required for maintenance of normal metabolic function and homeostasis, *e.g.* sterol metabolism, embryo lethal mutants (see, *e.g.* Schnabel *et al.,* 1991) and trans-spliced leader sequences (see, *e.g.* Ferguson et al, 1996), pos-1, cytoplasmic Zn finger protein; pie-1, cytoplasmic Zn finger protein; mei-1, ATPase; dif-1, mitochondrial energy transfer protein; rba-2, chromatin assembly factor; skn-1, transcription factor; plk-1, kinase; gpb-1, G-protein B subunit; par-1, kinase; bir-1, inhibitor of apoptosis; mex-3, RNA-binding protein, unc-37, G-protein B subunit; hlh-2, transcription factor; par-2, dnc-1, dynactin; par-6, dhc-1, dynein heavy chain; and pal-1, homeobox.

Several embodiments relate to a method of conferring insect resistance on a plant, the method comprising contacting a seed with an exogenous dsRNA molecule comprising a sequence that is essentially identical or essentially complementary to at least 18 contiguous nucleotides of a gene of an insect, and growing a plant from the seed. As used herein, a "insect resistance" trait is a characteristic of a plant that causes the plant host to be resistant to attack from an insect that typically is capable of inflicting damage or loss to the plant. Not wishing to be bound by a particular theory, once the insect is provided with the plant material produced from a seed comprising the naked dsRNA, expression of the gene within the target insect is suppressed, and the suppression of expression of the gene in the target insect results in the plant being resistant to the insect.

In the embodiments described herein, the target gene can encode an essential protein or transcribe an non-coding RNA which, the predicted function is for example selected from the group consisting of ion regulation and transport, enzyme synthesis, maintenance of cell membrane potential, amino acid biosynthesis, amino acid degradation, development and differentiation, infection, penetration, development of appressoria or haustoria, mycelial growth, melanin synthesis, toxin synthesis, siderophore synthesis, sporulation, fruiting body synthesis, cell division, energy metabolism, respiration, and apoptosis, among others.

To substantiate the anti-pest activity, the present teachings also contemplate observing death or growth inhibition and the degree of host symptomatology following said providing.

In some embodiments, a seed comprising an exogenous dsRNA as described herein is treated with a non-polynucleotide pesticide. It is believed that the combination of a plant exhibiting bioactivity against a target pest as a result of treating the seed from which the plant is grown with an exogenous dsRNA coupled with treatment of the seed with certain chemical or protein pesticides provides unexpected synergistic advantages to seeds having such treatment, including unexpectedly superior efficacy for protection against damage to the resulting plant by the target pest. The seeds of the present embodiments are believed to have the property of decreasing the cost of pesticide use, because less of the pesticide can be used to obtain a required amount of protection than if the innovative composition and method is not used. Moreover, because less pesticide is used it is believed that the subject method is therefore safer to the operator and to the environment, and is potentially less expensive than conventional methods.

When it is said that some effects are "synergistic," it is meant to include the synergistic effects of the combination on the pesticidal activity (or efficacy) of the combination of the bioactivity of a plant grown from a dsRNA treated seed and the pesticide. However, it is not intended that such synergistic effects be limited to the pesticidal activity, but that they should also include such unexpected advantages as increased scope of activity, advantageous activity profile as related to type and amount of damage reduction, decreased cost of pesticide and application, decreased pesticide distribution in the environment, decreased pesticide exposure of personnel who produce, handle and plant seeds, and other advantages known to those skilled in the art.

The seeds can be packed in a seed containing device which comprises a plurality of seeds at least some of which (*e.g.,* 5%, 10% or more) containing an exogenous naked dsRNA, wherein the seed is devoid of a heterologous promoter for driving expression of the dsRNA.

The seed containing device can be a bag, a plastic bag, a paper bag, a soft shell container or a hard shell container.

Also described herein is a solution for treating seeds comprising a non-transcribable polynucleotide trigger, for example dsRNA, molecule comprising a sequence that is essentially complementary or essentially identical to at least 18 contiguous nucleotides of a target gene. In some embodiments, the solution may further comprise buffer, for example, EDTA. As used herein "solution" refers to homogeneous mixtures and non-homogeneous mixtures such as suspensions, colloids, micelles, and emulsions. In some embodiments, the solution may be provided in a kit. In some embodiments, the kit may further comprise one or more of seeds, containers, priming solution, and seed growth medium.

Reagents of the present invention can be packed in a kit including the non-transcribable polynucleotide trigger, for example dsRNA, molecule, instructions for introducing the non-transcribable polynucleotide trigger, for example dsRNA, molecule into the seeds and optionally a priming solution.

Compositions as herein described may, if desired, be presented in a pack or dispenser device, which may contain one or more dosage forms containing the active ingredient. The pack may, for example, comprise metal or plastic foil, such as a blister pack. The pack or dispenser device may be accompanied by instructions for introduction to the seed.

The non-transcribable polynucleotide trigger, for example dsRNA, molecule and priming solution may be comprised in separate containers.

As used herein the term "about" refers to ± 10%.

The terms "comprises," "comprising," "includes," "including," "having" and their conjugates mean "including but not limited to."

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a," "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the agronomic, chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following Examples. The following Examples are presented for the purposes of illustration.

### EXAMPLES

Reference is now made to the following Examples, which together with the above descriptions illustrate the invention.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996. Other general references are provided throughout this document. The procedures therein are believed to be well known in the art and are provided for the convenience of the reader.

### EXAMPLE 1: PROTOCOLS FOR dsRNA PRODUCTION AND SEED

### TREATMENT (FOR REFERENCE ONLY)

### Generating the dsRNA/siRNA Sequences

The dsRNA sequences were custom-created for each gene using *in vitro* transcription of PCR products. Part of the mRNA, including either the ORF, 3' UTR or 5' UTR for which dsRNA to be produced was PCR-amplified using gene-specific primers, which contain the sequence of the T7 promoter on either side. This product was used as a template for dsRNA production using commercial kits such as the MaxiScript dsRNA kit (Life Technologies) or T7 High Yield RNA Synthesis kit (NEB). Next, the sample is treated with DNase Turbo at 37 °C for 15-30 min followed by phenol treatment and nucleic acid precipitation. Next, one of two different reactions is carried out: (1) dsRNA is ready to use, or (2) processing of the dsRNA with Dicer (Shortcut RNase III (NEB)) to create small interfering RNAs (siRNA).

Either dsRNA or a combination of dsRNA and siRNA were used for seed treatments as described below.

General Seed Treatment Protocol for Gene Silencing using a dsRNA/siRNA Mixture.

Uncoated organic corn seeds were from variety "popcorn," uncoated organic whole grain rice seeds, organic soybean and wheat seeds were purchased from Nitsat Haduvdevan (Israel). Fresh tomato seeds were retrieved from M82 tomato fruits, which are propagated in-house. Uncoated or fresh plant seeds were washed with double distilled water (DDW) prior to treatment for four hours. Next, seeds were dried at 30 °C for 10-16 hours. Following the drying step, seeds were treated with a solution containing the dsRNA formulation, which is made of dsRNA at a final concentration of 40-150 µg/ml in 0.1mM EDTA. Treatment was performed by gently shaking the seeds in the solution for 24 hours in a dark growth chamber at 15 °C. Finally, seeds were washed twice briefly and planted on soil or dried for 0-30 hours and germinated at 25 °C in a dark growth chamber and planted in soil or planted directly in soil. Control seeds were treated in a similar way, with a formulation that lacked the dsRNA or with non-specific dsRNA.

### EXAMPLE 2: STABILITY OF THE DSRNA IN SEEDLINGS OF RICE, TOMATO AND SORGHUM (FOR REFERENCE ONLY)

As an example for an exogenous gene that is not present/expressed in plants, the ORFs encoding the replicase and coat protein of CGMMV (accession number AF417242) were used to as targets for dsRNA treatment of plant seeds using the protocol described in Example 1. Rice, tomato and sorghum seeds were washed for 4 hours at 20 °C, tomato and sorghum were dried at 30 °C and rice at 20 °C for overnight. Seeds were immediately treated at 15 °C with 132.7 µg/ml dsRNA (final concentration) for 39 hours for rice, 93.8 µg/ml dsRNA (final concentration) for 48 hours for tomato, and 75 µg/ml dsRNA (final concentration) for 40 hours for sorghum.

Briefly, the virus-derived ORFs were amplified by PCR with specifically designed forward and reverse primers that contain the T7 sequence (5'-TAATACGACTCACTATAGGG-3', SEQ ID NO: 1) at their 5' (see Table 1, below). PCR products were purified from agarose gel and since they carry T7 promoters at both ends they were used as templates for T7-dependent in-vitro transcription, resulting in dsRNA product of the CGMMV genes. PCR on a housekeeping gene, tubulin, was used as a positive control (forward primer 5'-GGTGCTCTGAACGTGGATG-3' (SEQ ID NO: 2), and reverse primer 5'-CATCATCGCCATCCTCATTCTC-3'(SEQ ID NO: 3)).

**Table 1: PCR primers served as Templates for in vitro Transcription and detection of CGMMV and CGMMV dsRNA products.**

| Virus Name | Product Name | Product Sequence/SEQ ID NO: | Forward primer/SEQ ID NO: | Reverse primer/SEQ ID NO: |
|---|---|---|---|---|
| 1) CGMMV (NCBI Accession number AF417242) | CGMW dsRNA product 1 | TAATACGACTCACTATAGGGGGTAAGCG GCATTCTAAACCTCCAAATCGGAGGTTG GACTCTGCTTCTGAAGAGTCCAGTTCTGT TTCTTTTGAAGATGGCTTACAATCCGATC ACACCTAGCAAACTTATTGCGTTTAGTG CTTCTTATGTTCCCGTCAGGACTTTACTT AATTTTCTAGTTGCTTCACAAGGTACCGC TTTCCAGACTCAAGCGGGAAGAGATTCT TTCCGCGAGTCCCTGTCTGCGTTACCCTC GTCTGTCGTAGATATTAATTCTAGATTCC | TAATACGACT CACTATAGGG GGTAAGCGGC ATTCTAAACC/ (SEQ ID NO:5) | Set 1: |
| | | | | TAATACGACTCA CTATAGGGGAAG ACCCTCGAAACT AAGC/(SEQ ID NO:4) |
| | | | CTTCTTATGTT CCCGTCAGG/ (SEQ ID NO:7) | Set 2: |
| | | | | ACTCAGCAGTCG TAGGATTG/(SEQ ID NO:6) |
| | | CAGATGCGGGTTTTTACGCTTTCCTCAAC GGTCCTGTGTTGAGGCCTATCTTCGTTTC GCTTCTCAGCTCCACGGATACGCGTAAT AGGGTCATTGAGGTTGTAGATCCTAGCA ATCCTACGACTGCTGAGTCGCTTAACGC CGTAAAGCGTACTGATGACGCGTCTACG GCCGCTAGGGCTGAGATAGATAATTTAA TAGAGTCTATTTCTAAGGGTTTTGATGTT TACGATAGGGCTTCATTTGAAGCCGCGT TTTCGGTAGTCTGGTCAGAGGCTACCAC CTCGAAAGCTTAGTTTCGAGGGTCTTCC CCTATAGTGAGTCGTATTA/(SEQ ID NO:8) | | |
| | CGMW dsRNA product 2 | TAATACGACTCACTATAGGGGCTTTACC GCCACTAAGAACTCTGTACACTCCCTTG CGGGTGGTCTGAGGCTTCTTGAATTGGA ATATATGATGATGCAAGTGCCCTACGGC TCACCTTGTTATGACATCGGCGGTAACT ATACGCAGCACTTGTTCAAAGGTAGATC ATATGTGCATTGCTGCAATCCGTGCCTA GATCTTAAAGATGTTGCGAGGAATGTGA TGTACAACGATATGATCACGCAACATGT ACAGAGGCACAAGGGATCTGGCGGGTG CAGACCTCTTCCAACTTTCCAGATAGAT GCATTCAGGAGGTACGATAGTTCTCCCT GTGCGGTCACCTGTTCAGACGTTTTCCA AGAGTGTTCCTATGATTTTGGGAGTGGT AGGGATAATCATGCAGTCTCGTTGCATT CAATCTACGATATCCCTTATTCTTCGATC GGACCTGCTCTTCATAGGAAAAATGTGC GAGTTTGTTATGCAGCCTTTCATTTCTCG GAGGCATTGCTTTTAGGTTCGCCTGTAG GTAATTTAAATAGTATTGGGGCTCAGTT TAGGGTCGATGGTGATGCCCTATAGTGA GTCGTATTA/(SEQ ID NO:11) | TAATACGACT CACTATAGGG GCTTTACCGC CACTAAGAAC /(SEQ ID NO:10) | Set 3: |
| | | | | TAATACGACTCA CTATAGGGCATC ACCATCGACCCT AAAC /(SEQ ID NO:9) |

| | | | | |
|---|---|---|---|---|
| dsRNA homologous to green mottle mosaic virus is stable in rice seedlings. Rice seeds were treated at 15 °C with 132.7 µg/ml dsRNA (final concentration) for 39 hours and dsRNA was detected. At one week post germination, dsRNA was detectable in 9 out of 10 seedlings. Detection of tubulin cDNA served as a positive control for the cDNA quality. At two weeks post germination, dsRNA is detectable in 10 out of 10 seedlings. At 3 weeks post germination, dsRNA homologous to green mottle mosaic virus is detected in 5 out of 5 samples in rice seedlings | | | | |

Tomato seeds were treated at 15 °C with 93.8 µg/ml dsRNA (final concentration) for 48 hours and sorghum seeds treated at 5 µg/ml dsRNA (final concentration) for 40 hours. CGMMV dsRNA was detected by RT-PCR in 5 out of 13 tomato seedlings tested at 10 day post-germination and 3 out of four sorghum seedlings 4 weeks after germination.

The exogenous dsRNA was found to be stable for at least three weeks in rice seedlings and at least 10 days in tomato seedlings and four weeks in Sorghum plants.

### EXAMPLE 3: THE dsRNA IS NOT INTEGRATED INTO THE GENOME OF RICE (FOR REFERENCE ONLY)

Rice seeds were treated with an exogenous dsRNA as in Example 2. Plants were germinated and grown for five weeks, DNA was extracted and PCR reactions were performed to demonstrate that the dsRNA did not integrate into the Rice's genome. Two sets of primers that gave a positive reaction when checked on the RNA level were used, set 1 (see Table 2) of primers were the set of primers used to amplify the template (all the dsRNA sequence). Set 2 (see Table 3) are the primers that were used in the PCR above. A Rice endogenous housekeeping gene (tubulin) was used as a positive control for the PCR reaction (see Table 2).

Three different DNA PCR reactions were carried out on dsRNA treated and untreated plants. No amplified DNA corresponding to CGMMV was detected in any treated or untreated plant.

**Table 2: Tubulin Primers Used for PCR Amplification.**

| Primer Name and Direction | Primer Sequence/(SEQ ID NO:) | Primer Length |
|---|---|---|
| osa_TubA1_736F | GGTGCTCTGAACGTGGATG (SEQ ID NO: 12) | 19 |
| osa_TubA1_1342R | CATCATCGCCATCCTCATTCTC (SEQ ID NO: 13) | 22 |

### EXAMPLE 4: EXOGENOUS dsRNA MOLECULES ARE HIGHLY STABLE IN SOLUTION AND DO NOT GET INCORPORATED INTO THE GENOME OF TREATED PLANTS (FOR REFERENCE ONLY)

Corn seeds were treated using the protocol described in Example 1, seeds were washed for 4 h at 20 °C, dried at 30 °C overnight and immediately treated with 40 µg/ml dsRNA (final concentration) directed against the β-glucuronidase (GUS) reporter gene for 60 hours at 15 °C, dried and were germinated. Leaves and roots were harvested from control and dsGUS-treated plants 7 and 15 days following germination. RNA was extracted from the harvested tissues and RT-PCR with specific GUS primers was run (Table 3). In addition, a corn endogenous housekeeping gene (ubiquitin) was used as a positive control for the PCR reaction. The GUS dsRNA molecules were found to be extremely stable in the treated seeds, and can be detected in corn plants 7 and 15 days post germination of the seeds.

GUS dsRNA can is detected in corn seedlings by RT-PCR at 7 and 15 days after germination according to an aspect of the present disclosure. At one week, GUS dsRNA is detected in shoots of nine of eleven corn seedlings tested. GUS dsRNA is not detected in untreated plants. At 1 week post-germination, GUS dsRNA is detected in five of five treated corn seedlings' roots 1 week post germination. At 15 days post germination, GUS dsRNA is detected in corn seedlings' roots.

GUS dsRNA molecules do not get incorporated in the genome of treated corn plants one week after germination as determined by agarose gel electrophoresis of DNA PCR reactions on GUS sequence.

**Table 3: Primers for PCR Amplification of GUS and Ubiquitin Genes and GUS dsRNA product.**

| Primer Length | Primer Sequence/SEQ ID NO: | Primer Name |
|---|---|---|
| GUS_T7_For | TAATACGACTCACTATAGGGAGATCGACGGCCTGTGGGCATTC /(SEQ ID NO:15) | |
| GUS_T7_Rev | TAATACGACTCACTATAGGGAGCATTCCCGGCGGGATAGTCTG /(SEQ ID NO:16) | 43 |
| GUS208For | CAGCGCGAAGTCTTTATACC/(SEQ ID NO:17) | 43 |
| GUS289Rev | CTTTGCCGTAATGAGTGACC/(SEQ ID NO:18) | 20 |
| zmaUBQ-947F | CCATAACCCTGGAGGTTGAG/(SEQ ID NO:19) | 20 |
| zmaUBQ1043R | ATCAGACGCTGCTGGTCTGG/(SEQ ID NO:20) | 20 |
| GUS dsRNA product | TAATACGACTCACTATAGGGAGATCGACGGCCTGTGGGCATTC AGTCTGGATCGCGAAAACTGTGGAATTGATCAGCGTTGGTGG GAAAGCGCGTTACAAGAAAGCCGGGCTATTGCTGTGCCAGGC AGTTTTAACGATCAGTTCGCCGATGCAGATATTCGTAATTATG CGGGCAACGTCTGGTATCAGCGCGAAGTCTTTATACCGAAAG GTTGGGCAGGCCAGCGTATCGTGCTGCGTTTCGATGCGGTCAC TCATTACGGCAAAGTGTGGGTCAATAATCAGGAAGTGATGGA GCATCAGGGCGGCTATACGCCATTTGAAGCCGATGTCACGCC GTATGTTATTGCCGGGAAAAGTGTACGTATCACCGTTTGTGTG AACAACGAACTGAACTGGCAGACTATCCCGCCGGGAATGCTC CCTATAGTGAGTCGTATTA/(SEQ ID NO:21) | |

### EXAMPLE 5: FLUORESCENCE MICROSCOPY OF siRNA SEQUENCES IN VARIOUS PLANT SEEDS (FOR REFERENCE ONLY)

Plant seeds as per the protocol described in Example 1. Seeds were washed for 4 h at 20 °C, dried at 25 °C and were immediately treated with a fluorescent siRNA (siGLO, 2µM final concentration, Thermo Scientific) at 15 °C for 24 h. The quality of the siGLO before application to a plant seed was verified by gel electrophoresis analysis Bands c corresponding to the expected size of 20-24 bp of the fluorescent siRNA molecules was detected.

Fluorescent pictures of the seeds were taken 24-48 hours post treatment using an Olympus microscope at the lowest objective magnification (5X for bigger seeds such as rice and tomato seeds, and 10X for smaller seeds such as *Arabidopsis* seeds). To eliminate the possibility of non-specific auto-fluorescence, dsRNA-treated seeds are compared to control untreated seeds. Penetration of fluorescent siRNA molecules into plant seeds was observed at 24 hours after seed treatment with siRNA at 2 µM final concentration in *Arabidopsis* seeds, rice seeds, and tomato seeds.

Penetration of fluorescent siRNA molecules into rice seeds was observed at 24 hours following treatment with siGLO dsRNA.

In order to evaluate the distribution efficiency of the fluorescent siRNA inside the seeds, different plant seeds were cut into slices and imaged with a fluorescent microscope 48 hours after treatment. Each treated seed was imaged alongside a control untreated seed. Light and fluorescent images were taken where applicable for rice, tomato, cucumber, bean, sorghum and wheat seed samples.

Penetration of fluorescent siRNA molecules into rice seeds was observed at 48 hours following treatment with siGLO dsRNA. siGLO-treated and control rice seeds were sliced to view the interior distribution of the fluorescent dsRNA using a fluorescent microscope and fluorescent siRNA molecules detected in the treated seed. Fluorescent siGLO RNA is detected in the endosperm and the embryo.

Penetration of fluorescent siRNA molecules into tomato seeds was observed at 48 hours following treatment with siGLO dsRNA. siGLO-treated and control tomato seeds were sliced to view the interior distribution of the fluorescent dsRNA using a fluorescent microscope. Fluorescent siGLO RNA is detected in the endosperm and the embryo.

Penetration of fluorescent siRNA molecules into cucumber seeds was observed at 48 hours following treatment with siGLO dsRNA. siGLO-treated and control cucumber seeds were sliced to view the interior distribution of the florescent dsRNA using a fluorescent microscope. Fluorescent siGLO RNA is detected in the endosperm and the embryo.

Penetration of fluorescent siRNA molecules is detected in sliced seeds of various plant species, including bean, tomato, sorghum and wheat, 48 hours following treatment with siGLO dsRNA. siGLO-treated and control seeds were sliced to view the interior distribution of the fluorescent dsRNA using a fluorescent microscope. Light images were also taken for each seed and are shown alongside the fluorescent image of the seed for reference.

Figure 1 presents fluorescent images of siGLO-treatment of rice seeds over a 24 hour period. The effect of incubation time with siGLO dsRNA on fluorescence intensity, indicating quantity and quality of dsRNA penetration, was tested. Control seeds that were left untreated (1), were imaged along with seeds treated with siGLO dsRNA for four different incubation times; 10 min (2), 3.5 hours (3), 5.5 hours (4), and 24 hours (5).

It is clear that the siRNA is distributed at various levels between the embryo and the endosperm. Accordingly, dsRNA molecules enter the embryo directly. Though not to be limited by any particular theory, the dsRNA molecules are carried by the water-based solution used for the seed treatment. The dsRNA molecules enter the endosperm as part of the endosperm's water-absorption process. These molecules then are transferred to the embryo as it develops as part of the endosperm to embryo nutrient flow during germination and seed development.

These present findings suggest the RNA molecules used to treat the seeds both penetrate the embryo and function in the embryo as it develops and also penetrate the endosperm and feed the embryo following germination.

### EXAMPLE 6: TIME COURSE EXPERIMENT WITH siGLO TREATMENT (FOR REFERENCE ONLY)

A time course experiment was performed on rice seeds to monitor the kinetics of siGLO penetration into the seeds following the seed treatment (Figure 1). The results indicate that the siRNA efficiently penetrates the plant seeds using the protocol described in Example 1.

### EXAMPLE 7: SEED TREATMENT AGAINST SPODOPTERA LITTORALIS GENES

*Spodoptera littoralis* (or *Prodenia littoralis*), also known as the African Cotton Leafworm or Egyptian Cotton Leafworm, is a moth found widely in Africa and Mediterranean Europe. It is a common pest on vegetables, fruits, flowers and other crops.

RNA was extracted for dsRNA production from *Spodoptera littoralis* larvae, and a cDNA library was prepared from 0.5 µg total RNA. Several genes (ATPase, NADPH Cytochrome P450 oxidoreductase (herein referred to as NADPH), inhibitor of apoptosis (IAP) and Chitin Synthase) were selected to test the effect of feeding S. litoralis with plants grown from seeds treated with dsRNA directed against these genes (see Table 4). Corn seeds were washed for 4 h, dried at 30°C and immediately were treated with dsRNA molecules at a final concentration of 40µg/ml (for IAP and ATPase), 80µg/ml (for NADPH, 40µg/ml for each dsRNA sequence, see Table 4), or a mix solution (80µg/ml final) containing all three genes (20µg/ml for each of the four dsRNA sequences), for 24 hours. Fresh tomato seeds were not washed and immediately treated with dsRNA molecules at a final concentration of 66µg/ml (for IAP), 133µg/ml (for NADPH), or a mix solution (80µg/ml final) containing dsRNA targeting these two genes, for 48 hours. Treated seeds were germinated and grown into plants. Control seeds which were not treated with dsRNA directed against S. *littoralis* genes but were incubated with a similar solution, either not containing dsRNA or containing dsRNA directed against an unrelated gene, such as GUS, were germinated and grown alongside the treated plants. The leaves of treated and control plants were placed in petri dishes and used as sole food source for S. *littoralis* (typically, about 5 caterpillars per plate). Total body weight of the caterpillars was recorded at the beginning of each experiment, and was tracked throughout. New leaves were supplemented as needed and their weight was recorded as well. Body weight gain of the caterpillars was calculated and used as an indicator to their well-being and survivability.

**Table 4 Sequences of Spodoptera littoralis Genes for Down regulation and Primers used for dsRNA Molecules Generation.**

| Gene Name | Organism | SEQ ID NO |
|---|---|---|
| NADPH | *Spodoptera littoralis* NADPH cytochrome P450 oxidoreductase mRNA, complete cds (JX310073.1) | 21 |
| ATPase | *Spodoptera littoralis* H(+)-ATPase B subunit mRNA, partial cds (AY169409.1) | 22 |
| IAP | *Spodoptera littoralis* mRNA for inhibitor of apoptosis (iap gene) (AM709785.1) | 23 |

| Chitin synthase | *Spodoptera exigua* chitin synthase A mRNA, complete cds (DQ062153) | 24 |
|---|---|---|
| NADPH dsRNA#1 | *Spodoptera littoralis* | 25 |
| NADPH dsRNA#2 | *Spodoptera littoralis* | 26 |
| NADPH dsRNA#1 frwd | *Spodoptera littoralis* | 27 |
| NADPH dsRNA#1 rev | *Spodoptera littoralis* | 28 |
| NADPH dsRNA#2 frwd | *Spodoptera littoralis* | 29 |
| NADPH dsRNA#2 rev | *Spodoptera littoralis* | 30 |
| ATPase dsRNA#1 | *Spodoptera littoralis* | 31 |
| ATPase dsRNA#1 frwd | *Spodoptera littoralis* | 32 |
| ATPase dsRNA#1 rev | *Spodoptera littoralis* | 33 |
| IAP dsRNA#1 | *Spodoptera littoralis* | 34 |
| IAP dsRNA#1 frwd | *Spodoptera littoralis* | 35 |
| IAP dsRNA#1 rev | *Spodoptera littoralis* | 36 |
| Chitin synthase dsRNA#1 | *Spodoptera exigua* | 37 |
| Chitin synthase dsRNA#2 | *Spodoptera exigua* | 38 |
| Chitin synthase dsRNA#1 frwd | *Spodoptera exigua* | 39 |
| Chitin synthase dsRNA#2 frwd | *Spodoptera exigua* | 40 |
| Chitin synthase dsRNA#2 rev | *Spodoptera exigua* | 41 |
| Chitin synthase dsRNA#1 rev | *Spodoptera exigua* | 42 |

### Experiment 1

*Spodoptera littoralis* leafworms were placed in petri dishes with corn leaves from germinated control or dsRNA-treated seeds and were monitored daily for consumption of leaves and for body weight gain. Data for S. *littoralis* body weight gain after 24 hours, 48 hours and 5 days are shown in Table 5 respectively. A negative effect on body weight gain of the worms feeding on any dsRNA-treated leaves compared to worms feeding on control untreated leaves is noted. Body weight gain of S. *littoralis* fed on the control leaves was normalized to a value of '1'.

### Experiment 2

In this experiment, dsRNA molecules for silencing of the *S. littoralis* NADPH or IAP genes were used to treat corn seeds. Leaves from seedlings grown from these seeds, as well as control leaves, were used as a food source for 5 *Spodoptera littoralis* leafworms in a single petri dish (two plates for each treatment). Control leaves were treated with dsRNA directed against the GUS gene. Body weight gain was recorded for control and treated groups 48 hours from beginning of the experiment (Table 5). The strongest effect on body weight gain was seen in worms feeding on NADPH-dsRNA treated leaves. Body weight gain of *S. littoralis* fed on the control leaves was normalized to a value of'1'.

### Experiment 3

In this experiment, dsRNA molecules for silencing of the *S. littoralis* NADPH or IAP genes were used to treat tomato seeds. An additional treatment was also included, where seeds were treated with a mix solution containing the dsRNA molecules targeted against both genes. Leaves from seedlings grown from these seeds, as well as control leaves, were used as a food source for 5 *Spodoptera littoralis* leafworms in a single petri dish. Body weight gain was recorded for control and treated groups 72 hours after treatment is presented in Table 5. Body weight gain of *S. littoralis* fed on the control leaves was normalized to a value of'1'.

### Experiment 4

In this experiment, dsRNA molecules for silencing of the *S. littoralis* NADPH, IAP or ATPase genes were used to treat corn seeds. An additional treatment was also included, where seeds were treated with a mix solution containing the dsRNA molecules targeted against all three genes. Leaves from seedlings grown from these seeds, as well as control leaves, were used as a food source for 5 *Spodoptera littoralis* leafworms in a single petri dish. On day 4, the treated corn leaves were replaced with untreated lettuce leaves as the only food source. Body weight gain was recorded for control and treated groups for up to 8 days. The body weight of all worms at 24 hours was used as a reference point and body weight gain of *S. littoralis* fed on the control leaves was normalized to a value of '1'. Data of relative body weight gain of worms feeding on control or treated corn leaves is presented in Table 5.

**Table 5: Spodoptera littoralis body weight gain after twenty four hours on dsRNA treated leaves**

| Expt. | Time | control | NADPH | IAP | Mix | ATPase | gus |
|---|---|---|---|---|---|---|---|
| | 24 hours | 1.0 | 0.64 | 0.38 | n/a | 0.8 | n/a |
| 1 | 48 hours | 1.0 | 0.69 | 0.57 | n/a | 0.7 | n/a |
| 1 | 5 days | 1.0 | 0.36 | 0.84 | na/ | 0.94 | n/a |
| 2 | 48 hours | 1.0 | 0.55 | 0.9 | | | 1.0 |
| 3 | 48 hours | 1 | 0.55 | 0.9 | | | |
| 3 | 72 hours | 1 | 0.95 | 0.91 | 0.90 | | |
| 4 | 5 days¹ | 1.0 | 0.76 | 0.73 | 0.99 | 1.11 | |
| 4 | 7 days² | 1.0 | 0.88 | 0.87 | 0.89 | 0.91 | |
| 4 | 8 days³ | 1.0 | 0.9 | 0.78 | 0.97 | 1.12 | |

| | | | | | | | |
|---|---|---|---|---|---|---|---|
| ¹ four days of treated corn and 1 day of lettuce; ² four days of treated corn and 3 days of lettuce; ³ four days of treated corn and 4 days of lettuce | | | | | | | |

### EXAMPLE 8: GENERATION OF dsRNA MOLECULES FOR SILENCING A TARGET GENE OF A PHYTOPATHOGEN

dsRNAs encoding *S. littoralis* genes were analyzed against the corn and tomato genomes (Figures 2 and 3 respectively) using BLAST searches with the following parameters: Expect threshold - 10; Word size - 11; Match/Mismatch score 2,-3; Gap costs: Existence:5 Extension:2; Max matches in a query range: 0. BLAST searches were performed against the databases of corn (*Zea mays* - taxid:4577) and tomato (*Solanum lycopersicum* taxid:4081) sequences that meet the teachings of the present invention are presented.

### EXAMPLE 9: SEED TREATMENT AGAINST SPODOPTERA LITTORALIS NADPH GENE

Corn seeds (var. 01DKD2) were treated with dsRNA molecules (SEQ ID No. 26) having a nucleotide sequence that is essentially identical or essentially complementary to at least 18 contiguous nucleotides of the *S. littoralis* NADPH gene according to the protocol described in Example 1. A final concentration of 80 µg/ml dsRNA diluted with 0.1mM EDTA was used. Treatment was performed by gently shaking the seeds in the solution for 3.5 hours in a dark growth chamber at 15 °C. After treatment, seeds were planted in soil and grown at about 25 °C with 16 hours photoperiod. The plants were watered with tap water as necessary. Seeds that were treated with GFP dsRNA (SEQ ID No. 124), or with a similar solution not containing dsRNA (EDTA control), were germinated and grown alongside the treated plants as a control.

28 days after seed treatment, the leaves of treated and control plants were placed in petri dishes and used as sole food source for *S. littoralis.* For each plant, 15 larvae were used (5 larvae per plate, three plates per plant). Five plants from each seed treatment (NADPH, GFP and EDTA) were tested. New leaves were supplemented as needed. Body weight of each larva was recorded 12 days after the beginning of feeding and was used as an indicator of their well-being and survivability. A significant (one-way ANOVA, p-value=8.36×10⁻⁵) negative effect on the body weight of the larvae fed on NADPH dsRNA-treated plants compared to larvae fed on control plants was observed. See Table . The average weight of larvae fed on NADPH-treated plants was 23% and 20% lower than the average weight of larvae fed on GFP and EDTA-treated plants, respectively.

**Table 6: Spodoptera littoralis average weight (mg) after 12 days of feeding on treated plants.**

| Sample | 1 | 2 | 3 | 4 | 5 | Average | std. dev. |
|---|---|---|---|---|---|---|---|
| EDTA | 40.8 | 46.7 | 45.3 | 38.9 | 47.1 | 43.8 | 3.7 |
| GFP | 42.9 | 41 | 47.3 | 48.9 | 49.2 | 45.9 | 3.7 |
| sI-NADPH #1 | 38.5 | 26.8 | 32.5 | 35.2 | 43.2 | 35.2 | 6.2 |

73 days after seed treatment, the leaves of treated and control plants were used again as sole food source for *S. littoralis.* Five plants from each group were included in the feeding experiment. The leaves of each plant were placed in five petri dishes containing five larvae each, summing to 25 larvae per plant and 125 larvae per group. Seven days into the experiment, an unusual large number of larvae were found dead in the EDTA control group. Due to the large number of deaths in the control group, the effect of feeding plant tissue collected 73 days after dsRNA seed treatment on S. *littoralis* well-being and survivability was not analyzed further.

The expression levels of NADPH in subsets of larvae fed on plants grown from seeds treated with dsRNA molecules targeting NADPH or GFP (28 days after seed treatment) were determined.

**Table 7: Larvae from which RNA was extracted.**

| Leaf source | | | Weight (mg) |
|---|---|---|---|
| Treatment | Plant number | Repeat | |
| NADPH | 2 | 2 | 22 |
| | | | 29 |
| | | | 15 |
| | | | 35 |
| | | | 18 |
| | 3 | 3 | 36 |
| | | | 28 |
| | | | 32 |
| | | | 30 |
| | | | 39 |
| | | 2 | 29 |
| | | | 28 |
| GFP | 1 | 2 | 37 |
| | | | 49 |
| | | | 33 |
| | | | 27 |
| | | | 37 |
| | 2 | 3 | 28 |
| | | | 40 |
| | | | 26 |
| | | | 39 |
| | | | 31 |
| | | 2 | 34 |
| | | | 39 |

Total RNA was extracted from the larvae and cDNA was prepared using oligo-dT primers and the expression level of *S. littoralis* NADPH mRNA was determined in treated and control larvae by real-time PCR (RT-PCR) with SYBR Green (Quanta BioSciences), using the house-keeping genes Actin and EF1α as normalizers. The sequences of the primers used in the RT-PCR analysis are shown in Table .

**Table 8: Primers Used for RT-PCR Analysis for Expression Level of NADPH.**

| Primer Name and Direction | Primer Sequence | SEQ ID No. |
|---|---|---|
| NADPH_F | ATGGCTGTTGACGTAAGG | 125 |
| NADPH_R | TGCAGCTTCAGCTTCTGTG | 126 |
| EF1α_F | ACCGTCGTACTGGTAAATCC | 127 |
| EF1α_R | TGGCGGCATCTCCAGATTTG | 128 |
| Actin_F | CTGGTCGTACCACCGGTAT | 129 |
| Actin_R | GCAGAGCGTAACCTTCGTAG | 130 |

| | | |
|---|---|---|
| No significant change in NADPH expression levels (Wilcoxon rank-sum test, p-value>0.05) was observed by RT-PCR analysis in larvae fed on plants grown from seeds treated with dsRNA molecules targeting NADPH or GFP (28 days after seed treatment). | | |

### EXAMPLE 10: SEED TREATMENT AGAINST SPODOPTERA LITTORALIS ATPase GENE

Corn seeds were treated according to the protocol described in Example 1 with dsRNA molecules having a nucleotide sequence that is essentially identical or essentially complementary to at least 18 contiguous nucleotides of the *S. littoralis* ATPase gene (SEQ ID No. 31). Briefly, seeds were washed with double distilled water (DDW) prior to treatment for four hours. Next, seeds were dried at 30 °C over-night. Following the drying step, a final concentration of 53 µg/ml dsRNA diluted with 0.1mM EDTA was used. Treatment was performed by gently shaking the seeds in the solution for 26 hours in a dark growth chamber at 15 °C. After treatment, seeds were germinated on wet paper for seven days and then planted in soil and grown at about 25 °C with 16 hours photoperiod. The plants were watered with tap water as necessary. Seeds that were treated with a similar solution not containing dsRNA were germinated and grown alongside the treated plants as a control (EDTA control).

43 days after seed treatment, the leaves of treated and control plants were used as sole food source for *S. littoralis.* Plant number 1 served as a food source for 20 larvae placed in 130×70mm box. Plant number 2 served as a food source for 15 larvae placed in 124×95mm box. Plant number 3 served as a food source for 8 larvae placed in petri dish. The surface of all boxes and plates was covered with vermiculite, and new leaves were supplemented as needed. Mortality and body weight of the larvae were tracked throughout the experiment. Figure 4A shows mortality and Figure 4B shows the average weight of live *S. littoralis* larvae eight days after the beginning of feeding. While the larvae fed on plants 1 and 3 grown from ATPase dsRNA treated seeds gained comparable weight and showed similar mortality to that of the control group, the larvae fed on plant number 2 grown from an ATPase dsRNA treated seed were almost 3-fold smaller compared to the control group, which had a higher death rate.

To test the persistence of the effects of dsRNA seed treatment, the leaves of plant number 2 were collected 85 days after seed treatment and used as sole food source for S. *littoralis.* A total of 15 larvae, in three petri dishes containing five larvae each, were used. Figure 4C shows the percentage of dead larvae three days after the beginning of experiment. In the ATPase dsRNA-treated group 12 out of 15 larvae were dead, while no dead larvae were found in the control group.

The persistence of the effects of dsRNA seed treatment were further tested by collecting the leaves of plants number 1 and 2 at 91 days after seed treatment, and using the leaves as the sole food source for *S. littoralis.* A total of 15 larvae, in three petri dishes containing five larvae each, were fed on each plant. Four days into the experiment, both groups were fed also on plant number 3. Figure 4D shows the percentage of dead larvae seven days after the beginning of feeding, compared to the control group.

### EXAMPLE 11: SEED TREATMENT AGAINST SPODOPTERA LITTORALIS ATPase, IAP AND NADPH GENES

The plants described in this Example were treated with dsRNA molecules having a nucleotide sequence that is essentially identical or essentially complementary to at least 18 contiguous nucleotides of the *S. littoralis* ATPase, IAP or NADPH gene, and are the same plants described in Example 7.

67 days after seed treatment, leaves of the treated and control plants described in Example 7 were used as sole food source for *S. littoralis.* One plant from each treatment served as a food source for 10 larvae placed in a petri dish. The surface of the plates was covered with vermiculite. Figure 5 shows the percentage of dead larvae after seven days of feeding.

### EXAMPLE 12: SEED TREATMENT AGAINST SPODOPTERA LITTORALIS EF1α GENE

Corn seeds were treated with dsRNA molecules having a nucleotide sequence that is essentially identical or essentially complementary to at least 18 contiguous nucleotides of the S. *littoralis* EF1α gene (Table) according to the protocol described in Example 1. Briefly, corn seeds were washed with double distilled water (DDW) prior to treatment for four hours. Next, the seeds were dried at 30 °C over-night. Following the drying step, a final concentration of 132 µg/ml dsRNA diluted with 0.1mM EDTA was used. Treatment was performed by gently shaking the seeds in the solution for 26 hours in a dark growth chamber at 15 °C. After treatment, seeds were germinated on wet paper for seven days and then planted in soil and grown at about 25 °C with 16 hours photoperiod. The plants were watered with tap water as necessary. Seeds that were treated with a similar solution not containing dsRNA were germinated and grown alongside the treated plants as a control (EDTA control).

**Table 9: dsRNAs derived from the S. littoralis EF1α gene.**

| Sequence Name | Sequence | SEQ ID No.: |
|---|---|---|
| EF1α dsRNA #1 | CTAATACGACTCACTATAGGGAGAATGCCCTGGTTCAAGGGATGG AACGTTGAGCGCAAGGAAGGCAAGGCTGAAGGTAAATGCCTCATT GAGGCCCTCGACGCCATCCTGCCCCCTGCTCGCCCCACAGACAAG CCCCTGCGTCTTCCCCTCCAGGACGTATACAAAATCGGTGGTATTG GTACGGTGCCCGTAGGCAGAGTTGAAACTGGTATCCTCAAGCCTG GTACCATCGTCGTCTTCGCCCCCGCCAACATCACCACTGAAGTCAA GTCTGTGGAGATGCACCACGAAGCTCTCCAAGAGGCCGTACCCGG TGACAACGTTGGTTTCAACGTAAAGAACGTTTCCGTCAAGGAGTT GCGTCGTGGTTACGTCGCTGGTGACTCCAAGAACAACCCACCCAA GGGCGCCGCCGATTTCACAGCACAGGTCATCGTGCTCAACCACCC TGGTCAAATCTCAAACGGATACACACCTGTGCTGGATTGCCACAC AGCCCACATTGCCTGCAAGTTCGCTGTCTCCCTATAGTGAGTCGTA TTAG | 131 |
| EF1α dsRNA #2 | CTAATACGACTCACTATAGGGAGAGGCCCAGGAAATGGGTAAGG GTTCCTTCAAATACGCCTGGGTATTGGACAAACTGAAGGCTGAGC | 132 |
| | GTGAACGTGGTATCACCATTGATATTGCTCTGTGGAAGTTCGAAA CCGCTAAATACTATGTCACCATTATTGACGCTCCCGGACACAGAG ATTTCATCAAGAACATGATCACTGGAACCTCCCAGGCCGATTGCG CCGTACTCATTGTCGCCGCTGGTACCGGTGAATTCGAGGCTGGTAT CTCGAAGAACGGACAGACCCGTGAGCACGCTCTGCTCGCTTTCAC ACTCGGTGTCAAGCAGCTGATTGTGGGCGTCAACAAAATGGACTC CACTGAGCCCCCATACAGCGAATCCCGTTTCGAGGAAATCAAGAA GGAAGTGTCCTCCTACATCAAGAAGATCGGTTACAACCCAGCTGC TGTCGCTTTCGTACCCATTTCTGGCTGGCACGGAGTCTCCCTATAG TGAGTCGTATTAG | |

43 days after seed treatment, the leaves of treated and control plants were used as sole food source for *S. littoralis.* Plant number 1 served as a food source for 20 larvae placed in 130×170mm box. Plant number 2 served as a food source for 15 larvae placed in 124×95mm box. Plant number 3 served as a food source for 8 larvae placed in petri dish. The surface of all boxes and plates was covered with vermiculite, and new leaves were supplemented as needed. Mortality and body weight of the larvae were tracked throughout the experiment. Eight days after the beginning of the feeding experiment, eight larvae out of 43 were found dead in the EF1α treated group, and three out of 43 larvae were dead in the control group. Figure 6A shows the average weight of live *S. littoralis* larvae eight days after the beginning of feeding.

87 days after seed treatment, the leaves of plants number 2 and 3 were used for a second time as sole food source for *S. littoralis.* A total of 15 larvae, in three petri dishes containing five larvae each, were fed on each EF1α-treated plant, and on two control plants (plants number 3 and 6). Figure 6B shows the percentage of dead larvae five days after the beginning of experiment.

### EXAMPLE 13: SEED TREATMENT AGAINST SPODOPTERA LITTORALIS BETA ACTIN GENE

Corn seeds were treated with dsRNA molecules having a nucleotide sequence that is essentially identical or essentially complementary to at least 18 contiguous nucleotides of the *S. littoralis* Beta actin gene (Table ) according to the protocol described in Example 1. Seeds were washed with double distilled water (DDW) prior to treatment for four hours. Next, seeds were dried at 30 °C overnight. Following the drying step, a final concentration of 76 µg/ml dsRNA diluted with 0.1mM EDTA was used. Treatment was performed by gently shaking the seeds in the solution for 26 hours in a dark growth chamber at 15 °C. After treatment, seeds were germinated on wet paper for seven days and then planted in soil and grown at about 25 °C with 16 hours photoperiod. The plants were watered with tap water as necessary. Seeds that were treated with a similar solution not containing dsRNA were germinated and grown alongside the treated plants as a control (EDTA control).

**Table 10: dsRNA derived from the S. littoralis Beta actin gene.**

| Sequence Name | Sequence | SEQ ID No.: |
|---|---|---|
| Beta actin dsRNA #1 | CTAATACGACTCACTATAGGGAGAATGGCTCCG GCATGTGCAAGGCCGGTTTCGCCGGCGACGACG CGCCCCGCGCCGTCTTCCCATCCATCGTAGGTCG CCCTCGTCACCAGGGTGTGATGGTTGGTATGGGT CAGAAGGACTCCTACGTAGGCGATGAGGCCCAG AGCAAGAGAGGTATCCTCACCCTGAAGTACCCC ATCGAGCACGGTATCATCACCAACTGGGACGAC ATGGAGAAGATCTGGCACCACACCTTCTACAAC GAGCTGCGCGTCGCCCCTGAGGAACACCCAGTC CTCCTGACTGAGGCTCCCCTCAACCCTAAGGCCA ACAGGGAGAAGATGACCCAGATCATGTTTGAGA CCTTCAACTCCCCCGCCATGTACGTCGCCATCCA GGCTGTGCTCTCTCTGTACGCCTCTGGTCGTACC ACCGGTATCGTCCTGGACTCCGGTGATGGTGTCT CCCACACCGTTCTCCCTATAGTGAGTCGTATTAG | 133 |

43 days after seed treatment, the leaves of treated and control plants were used as sole food source for *S. littoralis.* Plant number 1 served as a food source for 20 larvae placed in 130×170mm box. Plant number 2 served as a food source for 15 larvae placed in 124×95mm box. Plant number 3 served as a food source for 8 larvae placed in petri dish. The surface of all boxes and plates was covered with vermiculite, and new leaves were supplemented as needed. Mortality and body weight of the caterpillars were tracked throughout the experiment. Eight days after the beginning of the feeding experiment, three larvae out of 43 were found dead in both the Beta-actin treated group and the control group. Figure 7 shows average weight of live *S. littoralis* larvae eight days after the beginning of feeding.

### EXAMPLE 14: SEED TREATMENT AGAINST SPODOPTERA LITTORALIS NADPH GENE

Corn seeds were treated with dsRNA molecules (SEQ ID No. 26) having a nucleotide sequence that is essentially identical or essentially complementary to at least 18 contiguous nucleotides of the *S. littoralis* NADPH gene according to the protocol described in Example 1. Briefly, seeds were washed with double distilled water (DDW) prior to treatment for four hours. Next, seeds were dried at 30 °C overnight. Following the drying step, a final concentration of 154 µg/ml dsRNA diluted with 0.1mM EDTA was used. Treatment was performed by gently shaking the seeds in the solution for 26 hours in a dark growth chamber at 15 °C. After treatment, seeds were germinated on wet paper for seven days and then planted in soil and grown at about 25 °C with 16 hours photoperiod. The plants were watered with tap water as necessary. Seeds that were treated with a similar solution not containing dsRNA were germinated and grown alongside the treated plants as a control (EDTA control).

43 days after seed treatment, the leaves of treated and control plants were used as sole food source for *S. littoralis.* Plant number 1 served as a food source for 20 larvae placed in 130×170mm box. Plant number 2 served as a food source for 15 larvae placed in 124×95mm box. Plant number 3 served as a food source for 8 larvae placed in petri dish. The surface of all boxes and plates was covered with vermiculite, and new leaves were supplemented as needed. Mortality and body weight of the larvae were tracked throughout the experiment. Eight days after the beginning of the feeding experiment, three larvae out of 43 were found dead in both the NADPH treated group and the control group. Figure 8A shows average weight of live *S. littoralis* larvae eight days after the beginning of feeding.

91 days after seed treatment, the leaves of plant number 2 were used for a second time as sole food source for *S. littoralis.* A total of 15 larvae, in three petri dishes containing five larvae each, were fed on the NADPH-treated plant. Additional 15 larvae, in three petri dishes containing five larvae each, were fed on control plant. Figure 8B shows the percentage of dead larvae seven days after the beginning of experiment. In the NADPH-treated group 9 out of 15 larvae were dead, while in the control group 2 out of 15 larvae were dead.

### EXAMPLE 15: SEED TREATMENT AGAINST SPODOPTERA LITTORALIS IAP, ATPase AND NADPH GENES

Corn seeds were treated according to the protocol described in Example 1 with dsRNA molecules (SEQ ID No. 34) having a nucleotide sequence that is essentially identical or essentially complementary to at least 18 contiguous nucleotides of the S. *littoralis* IAP gene or with a solution containing a mix of dsRNAs (SEQ ID Nos. 34, 25, 26, and 31) having a nucleotide sequence that is essentially identical or essentially complementary to at least 18 contiguous nucleotides of the *S. littoralis* IAP, NADPH and ATPase genes. These two solutions were first used for the seed treatment described in Example 7, and then re-used in the experiment described here. Seeds were washed with double distilled water (DDW) prior to treatment for four hours. Next, seeds were dried at 30 °C overnight. Treatment was performed by gently shaking the seeds in the solution for 24 hours in a dark growth chamber at 15 °C. After treatment, the seeds were dried overnight at 30 °C, planted in soil and grown at about 25 °C with 16 hours photoperiod. The plants were watered with tap water as necessary. Seeds that were treated with a similar solution (EDTA) not containing dsRNA were germinated and grown alongside the treated plants as a control.

27 days after seed treatment, the leaves of the treated and control plants were used as sole food source for *S. littoralis.* A total of 24 larvae, in three petri dishes containing eight larvae each, were used for each treatment. One repeat from the IAP treatment contained nine larvae. Each repeat was fed from one plant, and three days into the experiment a second plant from the same treatment was added to the plate. Mortality and body weight of the caterpillars were tracked throughout the experiment. Figures 9A-B shows average weight of live *S. littoralis* larvae six days after the beginning of the feeding experiment.

### EXAMPLE 16: SEED TREATMENT AGAINST SPODOPTERA LITTORALIS EF1α GENE

Corn seeds were treated according to the protocol described in Example 1 with two dsRNA molecules having a nucleotide sequence that is essentially identical or essentially complementary to at least 18 contiguous nucleotides of the *S. littoralis* EF1α gene (Table ). Briefly, seeds were washed with double distilled water (DDW) prior to treatment for four hours. Next, seeds were dried at 30°C overnight. Two dsRNA sequences (SEQ ID No. 131 and SEQ ID No. 132) were used separately in two different seed treatments; each at a final concentration of 67 µg/ml dsRNA diluted with 0.1mM EDTA. Treatment was performed by gently shaking the seeds in the solution for 24 hours in a dark growth chamber at 15 °C. After treatment, seeds were dried at 30 °C overnight and then planted in soil and grown at about 25 °C with 16 hours photoperiod. The plants were watered with tap water as necessary. Seeds that were treated with dsRNA molecules having a nucleotide sequence that is essentially identical or essentially complementary to at least 18 contiguous nucleotides of the corn DWF1 gene (Table ) (44µg/ml for DWF1#1 (SEQ ID No. 134) and 51µg/ml for DWF1#2 (SEQ ID No. 135)) were germinated and grown alongside the treated plants as two separated controls.

**Table 11: Control dsRNAs derived from the corn DWF gene.**

| Sequence Name | Sequence | SEQ ID No.: |
|---|---|---|
| DWF1 dsRNA #1 | CTAATACGACTCACTATAGGGAGTGTCAACATGGGTCAGAT AACCAGAGCTACCTGCCCAATGAACCTTGCCCTTGCGGTCG TCGCCGAGCTCGACGACCTCACTGTTGGTGGGCTGATCAAC GGTTACGGCATCGAGGGGAGCTCTCACCTCTATGGCCTTTTC TCCGACACGGTTGTCGCGATGGAGGTTGTTCTCGCAGATGG CCGGGTCGTCAGAGCCACCAAGGACAACGAGTACTCTGACC TTTTCTATGGAATTCCCTGGTCCCAGGGAACACTGGGGTTCC TTGTCTCTGCAGAGATCAAGCTGATCCCCATCAAGGAGTAC ATGAAGCTCACCTACACTCCAGTCAAGGGGGGTCTAAAGGA GATCGCGCAGGCCTACGCGGATTCTTTCGCTCCGAGGGACG GTGACCCGGCAAAGGTCCCTGACTTTGTTGAAGGGATGGTG TACACAGAGAGCGAGGGTGTCATGATGACGGGCGTGTACGC TTCGAAAGAAGAGGCGAAGAAGAAGGGCAACAAGATCAAC TGCGTGGGGTGGTGGTTTAAGCCCTGGTTCTACCTCTCCCTA TAGTGAGTCGTATTAG | 134 |
| DWF1 dsRNA #2 | CTAATACGACTCACTATAGGGAGAGCGAGTTTGTGGAGTAC ATCCCGACGAGGGAGTACTACCACCGGCACACCCGGTGCCT GTACTGGGAGGGGAAGCTGATCCTGCCCTTCGGCGACCAGT TCTGGTTCAGGTTCCTGCTGGGCTGGCTGATGCCACCGAAG GTGTCCCTGCTGAAGGCGACCCAGGGCGAGGCTATCAGGAA CTACTACCACGACAACCATGTGATCCAGGACATGCTGGTGC CGCTGTACAAGGTTGGGGATGCGCTGGAGTTCGTGCACCGC GAGATGGAGGTGTATCCTCTGTGGCTGTGCCCTCACCGGCT GTACAAGCTGCCGGTGAAGACGATGGTGTACCCGGAGCCTG GGTTCGAGCACCAGCACAGGCAGGGCGACGCGAGCTACGC ACAGATGTTCACGGACGTGGGCGTGTACTACGCCCCCGGGG CGGTGCTGAGGGGGGAGGAGTTCAACGGCGCGGAGGCTGT GCACAGGCTGGAGCAGTGGCTGATCGAGAACCACAGCTAC CAGCCGCAGTACGCGGTGTCGGAGCTGAACGAGAAGGACT CCTGTCTCCCTATAGTGAGTCGTATTAG | 135 |

35 days after seed treatment, the leaves of the treated and control plants were used as sole food source for *S. littoralis.* Two plants from each treatment and from the DWF1#1 control were included in the feeding experiment. The leaves of each plant were placed in two petri dishes containing 10 larvae each, summing to 40 larvae fed on each seed treatment. Mortality and body weight of the larvae were tracked throughout the experiment. Nine days after the beginning of the feeding experiment, four larvae out of 40 were found dead in the EF1α #2 treated group and in the control group. Six larvae out of 40 were found dead in the EF1α #1 treated group. Figure 10A shows average weight of live *S. littoralis* larvae nine days after the beginning of the feeding experiment.

36 days after seed treatment, other plants from the same EF1α#1 and DWF1#1 seed treatment were used as sole food source for *S. littoralis.* Fifteen plants from the treatment were included in the feeding experiment. The leaves of each plant were placed in three petri dishes containing 5 larvae each, summing to 15 larvae per plant and 225 larvae total. Two days into the experiment, plant number 15 was replaced by plant number 8. Two plants from the control group were included in the feeding experiment. The leaves of each control plant were placed in three petri dishes containing 5 larvae each, summing to 15 larvae per plant and 30 larvae total. Body weight of the larvae was tracked throughout the experiment. Figure 10B shows average weight *of S. littoralis* larvae after five days of feeding.

71 days after seed treatment, the leaves of the EF1α #2 treated and DWF1#2 control plants were used as sole food source for *S. littoralis.* Ten plants from the treatment were included in the feeding experiment, from which two plants were tested for the second time (see Figure 10A) and eight plants were tested for the first time. The leaves of each plant were placed in three petri dishes containing five larvae each, summing to 15 larvae per plant and 150 larvae total. Two plants from the control group, that were not tested previously, were included in the feeding experiment. The leaves of each control plant were placed in three petri dishes containing five larvae each, summing to 15 larvae per plant and 30 larvae total. Eight days into the experiment, an unusually large number of larvae were found dead in both treatment and control groups. Therefore, this time point was not analyzed further.

### EXAMPLE 17: SEED TREATMENT AGAINST SPODOPTERA LITTORALIS ATPase GENE

Corn seeds were treated with dsRNA molecules (SEQ ID No. 31) having a nucleotide sequence that is essentially identical or essentially complementary to at least 18 contiguous nucleotides of the *S. littoralis* ATPase gene according to the protocol described in Example 1. Briefly, seeds were washed with double distilled water (DDW) prior to treatment for four hours. Next, seeds were dried at 30 °C overnight. Following the drying step, a final concentration of 145 µg/ml dsRNA diluted with 0.1mM EDTA was used. The dsRNA solution contained a mixture of un-treated dsRNA molecules and phenol-treated dsRNA molecules as described in Example 1. Treatment was performed by gently shaking the seeds in the solution for 24 hours in a dark growth chamber at 15 °C. After treatment, seeds were dried at 30 °C overnight and then planted in soil and grown at about 25 °C with 16 hours photoperiod. The plants were watered with tap water as necessary. Seeds that were treated with 67 µg/ml dsRNA (SEQ ID No. 20) derived from GUS sequence were germinated and grown alongside the treated plants as a control.

56 days after seed treatment, the leaves of treated and control plants were used as sole food source for *S. littoralis.* Ten plants from the treatment were included in the feeding experiment. The leaves of each plant were placed in three petri dishes containing five larvae each, except for plants number 3 and 14 that were placed together in the same plates. A total of 15 larvae per plant and 135 larvae total were tested. Two plants from the control group were included in the feeding experiment. The leaves of each control plant were placed in three petri dishes containing five larvae each, summing to 15 larvae per plant and 30 larvae total. After twelve days of feeding, 12 out of 135 larvae were found dead in the ATPase treated group and 21 out of 30 larvae were found dead in the control group. Figure 11A shows the percentage of dead larvae 12 days after the beginning of experiment.

57 days after seed treatment, other plants from the same treated and control groups were used as sole food source for *S. littoralis.* Fourteen plants from the treatment were included in the feeding experiment. The leaves of each plant were placed in three petri dishes containing five larvae each, except for plants number 13 and 4, which were placed together in the same plates, and plants number 10 and 19, which were placed together in the same plates (plants 4 and 10 were analyzed for the second time, see Figure 11A). A total of 15 larvae per plant and 180 larvae overall were tested. Two plants from the control group were included in the feeding experiment. The leaves of each control plant were placed in three petri dishes containing five larvae each, summing to 15 larvae per plant and 30 larvae total. Four days after feeding begun, 29 larvae out of 180 were found dead in the ATPase treated group and 29 larvae out of 30 were found dead in the control group. Figure 11B shows the percentage of dead larvae four days after the beginning of experiment.

### EXAMPLE 18: SEED TREATMENT AGAINST SPODOPTERA LITTORALIS EF1α GENE

Corn seeds (var. Vivani) were treated with dsRNA molecules (SEQ ID Nos. 131 and 132) having a nucleotide sequence that is essentially identical or essentially complementary to at least 18 contiguous nucleotides of the *S. littoralis* EF1α gene according to the protocol described in Example 1. A mixture of 25 µg/ml from each of the two dsRNAs was used. The dsRNA was diluted either with 0.1mM EDTA alone, or additionally mixed with 40 µg/ml of PEG-modified carbon nanotubes (CNTP). Treatment was performed by gently shaking the seeds in the solution for 4 hours in a dark growth chamber at 15 °C. After treatment, seeds were planted in soil and grown at about 25 °C with 16 hours photoperiod. The plants were watered with tap water as necessary. Seeds that were treated with 50 µg/ml dsRNA derived from GFP sequence (SEQ ID No. 124), or with a similar solution not containing dsRNA, with or without 40 µg/ml of PEG-modified carbon nanotubes, were germinated and grown alongside the treated plants as a control.

24 days after seed treatment, leaves of treated and control plants were used as sole food source for *S. littoralis.* Ten plants from the EF1α treatment group, two plants from the GFP control, one plant from the EDTA control and one plant from the EDTA/CNTP control were included in the feeding experiment. The leaves of each plant were placed in three petri dishes containing five larvae each, summing to 15 larvae per plant, 150 larvae for EF1α treatment, 30 larvae for GFP control and 15 for both of the EDTA controls. Figure 12A shows average weight of *S*. *littoralis* larvae after ten days of feeding.

25 days after seed treatment, leaves of treated and control plants were used as sole food source for *S. littoralis.* Thirteen plants from the EF1α/CNTP treatment group, two plants from the GFP/CNTP control, one plant from the EDTA/CNTP control and one plant from the EDTA control were included in the feeding experiment. The leaves of each plant were placed in three petri dishes containing five larvae each, summing to 15 larvae per plant, except for plant 9 in the EF1α/CNTP group, where only two plates were analyzed. A total of 190 larvae for EF1α/CNTP treatment, 30 larvae for GFP/CNTP control and 15 larvae for both of the EDTA controls were tested. Seven days into the feeding experiment, plant 1 from the EF1α/CNTP group was replaced by plant 6 from the same group. Figure 12B shows average weight of *S. littoralis* larvae after ten days of feeding. To determine the expression levels of EF1α in the larvae ten days after feeding on treated plants, each repeat (plate) of five larvae was pooled together, and total RNA was extracted. cDNA was prepared using oligo-dT primers (SEQ ID Nos. 136-143) and the expression level of *S. littoralis* EF1α mRNA was determined in treated and control larvae by real-time PCR with SYBR Green (Quanta BioSciences), using Actin and ATPase as normalizers. No significant change in EF1α expression levels (Wilcoxon rank-sum test, p-value>0.05) was observed.

61 days after seed treatment, leaves of treated and control plants were used again as sole food source for *S. littoralis.* Thirteen plants from the EF1α/CNTP treatment group and three plants from the GFP/CNTP control were included in the feeding experiment. Some of the plants from the EF1α/CNTP group were tested for the first time and some were tested for the second time (see Figure 12B). The three plants from the GFP/CNTP control were tested for the first time. The leaves of each plant were placed in three petri dishes containing five larvae each, summing to 15 larvae per plant, except for plant 8 in the EF1α/CNTP group, where only two plates were analyzed. A total of 190 larvae for EF1α/CNTP treatment and 45 larvae for GFP/CNTP control were tested. Twelve days into the experiment, an unusually large number of larvae were found dead in both treatment and control groups. Therefore, this time point was not further analyzed.

### EXAMPLE 19: SEED TREATMENT AGAINST SPODOPTERA LITTORALIS EF1α GENE

Corn seeds (var. 01DKD2) were treated with dsRNA molecules (SEQ ID Nos. 131 and 132) having a nucleotide sequence that is essentially identical or essentially complementary to at least 18 contiguous nucleotides of the *S. littoralis* EF1α gene according to the protocol described in Example 1. A mixture of 25 µg/ml from each of the two dsRNAs was used. The dsRNA was diluted either with 0.1mM EDTA alone, or additionally mixed with 40 µg/ml of PEG-modified carbon nanotubes (CNTP). Treatment was performed by gently shaking the seeds in the solution for 4 hours in a dark growth chamber at 15 °C. After treatment, seeds were planted in soil and grown at about 25 °C with 16 hours photoperiod. The plants were watered with tap water as necessary. Seeds that were treated with 50 µg/ml dsRNA (SEQ ID No. 20) derived from GUS sequence, with or without 40 µg/ml of PEG-modified carbon nanotubes, were germinated and grown alongside the treated plants as a control.

Eight days after seed treatment, the leaves of treated and control plants were used as sole food source for *S. littoralis.* Thirteen plants from the EF1α treatment, thirteen plants from the EF1α/CNTP treatment, ten plants from the GUS control and four plants from the GUS/CNTP control were included in the feeding experiment. The leaves of each plant were placed in two petri dishes covered with 1% agar. Each plate contained three larvae, summing to six larvae per plant, 78 larvae for both the EF1α and EF1α/CNTP treatments, 60 larvae for the GFP control and 24 for the GUS/CNTP control. Body weight of the larvae was recorded four days after feeding. Figures 13A-B shows average weight of *S*. *littoralis* larvae in control and treatment groups.

### EXAMPLE 20: SEED TREATMENT AGAINST SPODOPTERA LITTORALIS IAP, ATPase AND NADPH GENES

Tomato plants grown from the tomato seeds described in Example 7, which were treated with dsRNA molecules (SEQ ID Nos. 34, 35, 25 and 26) having a nucleotide sequence that is essentially identical or essentially complementary to at least 18 contiguous nucleotides of the *S. littoralis* IAP gene, ATPase gene or NADPH gene were examined further for control of *S*. *littoralis.*

48 days after seed treatment, the leaves of treated and control plants were used as sole food source for *S. littoralis.* One plant from each treatment served as a food source for seven larvae placed in a petri dish. The surface of all plates was covered with vermiculite. Mortality and body weight of the larvae were tracked throughout the experiment. Three days into the experiment, one larva was found dead in the IAP treated group, and two larvae were found dead in the MIX treated group. No further death occurred in the following days up to day 7. Figure 14 shows the average weight of live *S. littoralis* larvae after three and seven days of feeding.

### EXAMPLE 21: SEED TREATMENT AGAINST SPODOPTERA LITTORALIS BETA ACTIN, ATPase AND NADPH GENES

Tomato seeds were treated with dsRNA molecules (SEQ ID Nos. 133, 31, 25, and 26) having a nucleotide sequence that is essentially identical or essentially complementary to at least 18 contiguous nucleotides of the *S. littoralis* Beta actin gene (see Table ), ATPase gene or NADPH gene according to the protocol described in Example 1. A final concentration of 96 µg/ml dsRNA for Beta actin, 73 µg/ml dsRNA for ATPase and 164 µg/ml dsRNA for NADPH, diluted with 0.1mM EDTA was used. Treatment was performed by gently shaking the seeds in the solution for 26 hours in a dark growth chamber at 15 °C. After treatment, seeds were germinated in soil and grown at about 25 °C with 16 hours photoperiod. The plants were watered with tap water as necessary. Seeds that were treated with a similar solution (EDTA) not containing dsRNA were germinated and grown alongside the treated plants as a control.

42 days after seed treatment, the leaves of treated and control plants were used as sole food source for *S. littoralis.* Plants number 1 and 2 from the Beta actin and ATPase treatments and plants number 21 and 23 from the NADPH treatment were used. The plants from each treatment served as a food source for five larvae placed in a petri dish. The surface of all plates was covered with vermiculite. Body weight of the larvae was tracked throughout the experiment. Figure 15 shows average weight of *S. littoralis* larvae after four days of feeding.

### EXAMPLE 22: SEED TREATMENT AGAINST SPODOPTERA LITTORALIS ATPase GENE

The tomato plants described in this Example originate from the seed treatment with ATPase dsRNA described in Example 21 above.

85 days after seed treatment, the leaves of treated and control plants described in Example 21 were used again as sole food source for *S. littoralis.* One plant from the ATPase treatment and one plant from the control were used. Leaves from these plants were placed in three petri dishes contain five larvae each. Three days into the experiment, another plant from the treatment and another plant from the control were added to their respective plates. Body weight of the larvae was tracked throughout the experiment. Since at the onset of the feeding experiment the larvae fed from the control group were 30 % smaller when the larvae fed from the treatment, the weight of the larvae relative to their initial weight was recorded. Figure 16A shows relative weight of *S. littoralis* larvae after six days of feeding.

88 days after seed treatment, other plants from the same seed treatment were used as sole food source for *S. littoralis.* Three plants from the ATPase treatment and two plants from the control were used. Leaves from these plants were placed in three petri dishes contain five larvae each. Mortality and body weight of the larvae were tracked throughout the experiment. After feeding for five days, 4 out of 15 and 1 out of 15 worms were found dead in the ATPase and control group, respectively. Figure 16B shows average weight of live *S. littoralis* larvae after five days of feeding.

### EXAMPLE 23: SEED TREATMENT AGAINST SPODOPTERA LITTORALIS NADPH GENE

The tomato plants described in this Example originate from the seed treatment with NADPH dsRNA described in Example 21 above.

95 days after seed treatment, leaves of treated and control plants were used as sole food source for *S. littoralis.* Two plants from the NADPH treatment (not tested previously) and a pool of plants from the control were used. Leaves from these plants were placed in three petri dishes contain five larvae each. Body weight of the caterpillars was tracked throughout the experiment. Figure 17A shows average weight of *S. littoralis* larvae after four days of feeding. On the fourth day, the control plants were replaced by plants that were germinated from seeds treated against the tomato gene AFR8. These seeds were treated with a mixture of two dsRNA sequences (SEQ ID Nos. 25 and 26) at a final concentration of 200 µg/ml (100 µg/ml from each dsRNA) for 24 hours. On the sixth day, another plant was added to each of the two NADPH treated plants. Figure 17B shows average weight of *S. littoralis* larvae seven days after the feeding experiment begun.

### EXAMPLE 24: SEED TREATMENT AGAINST NON-SPODOPTERA LITTORALIS GENES

The corn plants described in this Example originate from the seed treatments described in Example 16 (DWF1 dsRNA#2, SEQ ID NO 135) and in Example 17 (GUS, SEQ ID NO 20).

69 days after seed treatment, the leaves of the germinated plants were used as sole food source for *S. littoralis.* Two plants from the DWF1 dsRNA#2 treatment and five plants from the GUS treatment were included in the feeding experiment. The leaves of each plant were placed in three petri dishes containing five larvae each, summing to 15 larvae per plant, 30 larvae for the DWF1 dsRNA#2 treatment and 75 larvae for the GUS treatment. Ten days into the experiment, an unusually large number of larvae were found dead in both treatments. Due to the large number of death in both treatment groups, this experimental time point was not further analyzed.

70 days after seed treatment, other plants from the same treatments were used as sole food source for *S. littoralis.* Two plants from the DWF1 dsRNA#2 treatment and 16 plants from the GUS treatment were included in the feeding experiment. The leaves of each plant were placed in three petri dishes containing five larvae each, summing to 15 larvae per plant, 30 larvae for the DWF1 dsRNA #2 treatment and 240 larvae for the GUS treatment. Nine days into the experiment, an unusually large number of larvae were found dead in both treatments. Due to the large number of death in both treatment groups, this experimental time point was not further analyzed.

### EXAMPLE 25: SEED TREATMENT AGAINST SPODOPTERA LITTORALIS ATPase, EF1α AND NADPH GENES

Corn seeds (var. Vivani) were treated with dsRNA molecules (SEQ ID Nos. 131, 132, 31, 25 and 26) having a nucleotide sequence that is essentially identical or essentially complementary to at least 18 contiguous nucleotides of the *S. littoralis* EF1α gene, ATPase gene or NADPH gene according to the protocol described in Example 1, without pre-treatment wash. The two EFIα dsRNAs were used separately. A final concentration of 160 µg/ml dsRNA, diluted with 0.1mM EDTA, was used. Treatment was performed by gently shaking the seeds in the solution for 2 hours in a dark growth chamber at 15 °C. After treatment, seeds were washed briefly with DDW, planted in soil and grown at about 25 °C with 16 hours photoperiod. The plants were watered with tap water as necessary. Seeds that were treated with 160 µg/ml dsRNA (SEQ ID No. 124) derived from GFP sequence, or with a similar solution not containing dsRNA (EDTA) were germinated and grown alongside the treated plants as a control.

31 days after seed treatment, the leaves of germinated plants were used as sole food source for *S. littoralis.* The larvae taken for this experiment were up to five hours old (*i.e.* up to five hours after hatching). Six plants from each treatment were included in the feeding experiment. The leaves of each plant were placed in 16 wells of 24-well plate containing one larva each, summing to 16 larvae per plant and 96 larvae per treatment. The surface of the wells were covered with 1 % agarose. Eight days after feeding had begun, 57 larvae were found dead in the ATPase treated group and 42 larvae were found dead in the NADPH treated group. The number of dead larvae in other groups ranged between 13 and 23. The average number of dead larvae in the six ATPase treated plants was significantly higher than the average number of dead larvae in the six GFP control plants, with a p-value of 0.03 (t-test). Similarly, the average number of dead larvae in the NADPH treated plants was higher compared to the average number of dead larvae in the GFP control plants (t-test, p-value=0.07). Figure 18A and B shows the percentage of dead larvae eight days after the beginning of feeding. Figure 18C and D shows the percentage of dead larvae ten days after the beginning of feeding. Figure 18E shows average weight of live *S. littoralis* larvae 11 days after the feeding experiment had begun.

32 days after seed treatment, other plants from the same seed treatment were used as sole food source for *S. littoralis.* The larvae taken for this experiment were up to 24 hours old. Five to seven plants from each treatment were included in the feeding experiment. The leaves of each plant were placed in 16 wells of 24-well plate containing one larva each, summing to 16 larvae per plant, 80 larvae for EDTA, 96 larvae for GFP and ATPase and 112 larvae for NADPH and for the two EF1α treatments. The surface of the wells were covered with 1 % agarose. Body weight of the larvae was recorded eight and nine days after the start of feeding; some of the larvae, feeding on a subset of the plants, were recorded in the eighth day and the remaining larvae were recorded in the ninth day. Figure 25F shows the average weight of live *S. littoralis* larvae per plant.

### EXAMPLE 26: SEED TREATMENTS TARGETING COLEOPTERAN PESTS

This Example illustrates non-limiting embodiments of a method of providing a plant having improved resistance to an coleopteran pest, including the step of growing a plant from a seed that has been contacted with a exogenous non-transcribable dsRNA, wherein said plant exhibits improved resistance to said coleopteran pest, relative to a plant grown from a seed not contacted with said dsRNA. More specifically this Example illustrates a method of providing a maize plant having improved resistance to a corn rootworm *(Diabrotica sp.),* including the step of growing a maize plant from a maize seed that has been contacted with at least one dsRNA designed to silence a target gene endogenous to a corn rootworm, wherein the maize plant germinated from the maize seed exhibits improved resistance to the corn rootworm, relative to a maize plant grown from a maize seed not contacted with the dsRNA.

A 228 bp dsRNA trigger with the sense strand sequence of GGCTGATAGCACTTAAGGAGCTTCCTAATCACGAAAGAATTCTGCAGGATT 
TAGTTATGGACATACTGAGAGTACTCTCTGCTCCTGACTTAGAAGTCCGCAA
GAAGACTTTAAGTCTAGCCCTTGAATTAGTCTCTTCACGGAACATAGAAGA
AATGGTATTAGTATTAACAAAGGAAGTGAGTAAAACGGTAGACAGTGAACA
TGAGGATACAGGAAAGTACAGGC (MON104454, SEQ ID No.:144) was tested in
 a corn rootworm infestation assay in maize plants grown from maize seeds contacted prior to germination with the dsRNA trigger. Maize seeds (70 seeds, variety LH244) were placed in a 50-milliliter Falcon tube with 35 milliliters of a solution of the dsRNA trigger in buffer (0.1 millimolar EDTA, diluted from a 0.5 molar pH 8 stock) or 35 milliliters of buffer alone as a null control, and incubated in the dark at 15 °C with gentle agitation for 8 hours. Seeds of a transgenic maize plant that expresses an RNA suppression construct targeting DvSnf7 and that has resistance to corn rootworm were used in a transgenic positive control and were similarly incubated prior to germination in 35 milliliters of buffer alone. DvSnf7 is the Snf7 ortholog from *Diabrotica virgifera virgifera* (Western corn rootworm, WCR) and is a component of the ESCRT-III complex (endosomal sorting complex required for transport); see Bolognesi et al. (2012) PLoS ONE 7(10): e47534, doi:10.1371/joumal.pone.0047534. The following day, the seeds were washed 3 times (1 minute/wash with gentle agitation) in enough water to fill the Falcon tube. The washed seeds were planted at a depth of 0.5 inch in 6" closed-bottom polyethylene pots filled with Metromix 200 soil. Greater than 85 % of the seeds germinated in all treatments. At the V2/V3 stage (approximately 2 weeks after planting), 50 neonate *Diabrotica virgifera virgifera* larvae were added to each pot (12-15 replicates performed). As a transgenic positive control, maize plants expressing a recombinant Snf7 transgene and similarly challenged with *Diabrotica virgifera virgifera* larvae were used. After ~4 weeks, the larvae were isolated using a Berlese funnel, counted, and weighed. Larval recovery and weight were calculated. The results are shown in Figure 19. Larval recovery per plant did not differ significantly between larvae fed on maize plants grown from seed treated with the dsRNA trigger at 50 ppm (micrograms/milliliter) and larvae fed on control plants (Figure 19A), but total larval weight (Figure 19B) and average larval weight (Figure 19C) were significantly reduced in the larvae fed on maize plants grown from seed treated with the dsRNA trigger at 50 ppm (micrograms/milliliter), compared to larvae fed on control plants. The plants grown from seed treated with the dsRNA trigger at 500 ppm exhibited stunted plant growth and root growth, which may have affected the observed results; nonetheless, larval recovery per plant was significantly decreased (Figure 19A), and total larval weight (Figure 19B) and average larval weight (Figure 19C) were significantly reduced in the larvae fed on maize plants grown from seed treated with the dsRNA trigger at 500 ppm (micrograms/milliliter), compared to larvae fed on control plants. Quantigene assays did not detect a significant amount of MON104454 RNA in either leaf or root tissue of the maize plants grown from seed treated with the dsRNA trigger at 500 ppm.

A similar experiment was carried out in tomato plants grown from seeds treated prior to germination by incubating overnight in 100 ppm (micrograms/milliliter) of a 279 bp blunt-ended dsRNA trigger with the sense strand sequence of 
TACCTGTGGCTCTCACAGGCAGCGAAGATGGTACCGTTAGAGTTTGGCATA
CGAATACACACAGATTAGAGAATTGTTTGAATTATGGGTTCGAGAGAGTGT
GGACCATTTGTTGCTTGAAGGGTTCGAATAATGTTTCTCTGGGGTATGACGA
GGGCAGTATATTAGTGAAAGTTGGAAGAGAAGAACCGGCAGTTAGTATGGA
TGCCAGTGGCGGTAAAATAATTTGGGCAAGGCACTCGGATTACAACAAGCT
AATTTGAAGGCGCTGCCAGAAGG (T6593, SEQ ID No.:145) and subjected to a
 *Leptinotarsa decemlineata* (Colorado potato beetle, CPB) infestation assay. Control plants were treated with either buffer ("formulation") or a dsRNA trigger for green fluorescent protein (GFP). Germination rate was >90 % and no obvious effects on plant growth were observed for the treated plants, compared to the control plants. No significant effect on either the tomato plant defoliation rate (Figure 20A) or on recovery of viable larvae (Figure 20B) or average larval weight (Figure 20C) were observed for the plants treated with either T6593 dsRNA or GFP dsRNA, compared to the control plants. Quantigene assays did not detect a significant amount of T6593 RNA in analyzed tissues (young leaf, old leaf, cotyledon, root) of the tomato plants grown from seed treated with the T6593 dsRNA trigger at 500 ppm.

### EXAMPLE 27: SEED TREATMENTS TARGETING ESSENTIAL COLEOPTERAN GENES

This Example illustrates non-limiting embodiments of a method of providing a plant having improved resistance to an coleopteran pest, including the step of growing a plant from a seed that has been contacted with a exogenous non-transcribable dsRNA, wherein said plant exhibits improved resistance to said coleopteran pest, relative to a plant grown from a seed not contacted with said dsRNA. More specifically this Example illustrates a method of providing a maize plant having improved resistance to a corn rootworm (*Diabrotica sp*.)*,* including the step of growing a maize plant from a maize seed that has been contacted with at least one polynucleotide trigger designed to silence a target gene endogenous to a corn rootworm, wherein the maize plant germinated from the maize seed exhibits improved resistance to the corn rootworm, relative to a maize plant grown from a maize seed not contacted with the polynucleotide trigger.

Double-stranded RNA (dsRNA) triggers for the target genes identified in Table are produced. Suitable triggers are of 21-1,000 base pairs in length, in some embodiments, 21-50, 50-100, 100-200, 200-500, 500-700, or 700-1,000 base pairs in length. The triggers provided in Table are between 173-504 base pairs in length, but both shorter or longer triggers are useful in the methods disclosed herein. All of the dsRNA triggers provided in Table were determined to cause significant larval stunting and mortality at 10 ppm and at 0.1 ppm in a diet bioassay with *Diabrotica virgifera virgifera* (Western corn rootworm, WCR) as described in the working examples in US Patent Application Publication 2009/0307803, where the dsRNA trigger is delivered as an overlayer on the surface of a solid insect diet in a 96-well plate.

**Table 12: dsRNA triggers.**

| Trigger ID | Trigger Length (bp) | Target Gene | SEQ ID NO. OF TARGET GENE |
|---|---|---|---|
| T33514 | 501 | Croquemort | 146 |
| T33515 | 502 | predicted: similar to ENSANGP00000020392 | 147 |
| T33516 | 500 | Cathepsin L-like proteinase | 148 |
| T33519 | 501 | Uncharacterized conserved protein | 149 |
| T30147 | 502 | Eukaryotic translation initiation factor 3 subunit, putative | 150 |
| T30502 | 501 | Cleavage and polyadenylation specificity factor subunit 6 | 151 |
| T32275 | 502 | Cleavage and polyadenylation specificity factor subunit 6 | 152 |
| T32328 | 504 | Cleavage and polyadenylation specificity factor subunit 6 | 153 |
| T30501 | 501 | Lissencephaly-1 homolog | 154 |
| T30145 | 502 | Wd-repeat protein | 155 |
| T33520 | 501 | Sodium-dependent phosphate transporter | 156 |
| T30139 | 502 | T-complex protein 1 subunit delta | 157 |
| T30137 | 502 | Putative uncharacterized protein | 158 |
| T32250 | 502 | Solute carrier family 2, facilitated glucose transporter member 6 | 159 |
| T30133 | 501 | 26S proteasome non-ATPase regulatory subunit, putative | 160 |
| T30471 | 501 | WD repeat-containing protein 75 | 161 |
| T30132 | 501 | THO complex subunit 5-like protein | 162 |
| T30469 | 502 | Another transcription unit protein | 163 |
| T30467 | 502 | CG8315 | 164 |
| T30466 | 374 | Putative uncharacterized protein | 165 |
| T33522 | 500 | E3 ubiquitin-protein ligase UBR2 | 166 |
| T30463 | 502 | TMEM9 domain family member B | 167 |
| T30462 | 501 | Eukaryotic translation initiation factor 2 subunit 1 | 168 |
| T32319 | 500 | Eukaryotic translation initiation factor 2 subunit 1 | 169 |
| T30126 | 502 | Pre-mRNA-processing factor 6 | 170 |
| T32320 | 496 | Delta-aminolevulinic acid dehydratase | 171 |
| T30456 | 502 | StAR-related lipid transfer protein 7 | 172 |
| T32316 | 496 | 26S proteasome non-ATPase regulatory subunit, putative | 173 |
| T30117 | 502 | Putative uncharacterized protein | 174 |
| T30112 | 502 | General transcription factor IIF subunit 2 | 175 |
| T30423 | 501 | Proliferating cell nuclear antigen | 176 |
| T32201 | 502 | Proliferating cell nuclear antigen | 177 |
| T30420 | 501 | Cactin | 178 |
| T30417 | 501 | Vesicle-trafficking protein SEC22b | 179 |
| T30106 | 482 | Putative uncharacterized protein | 180 |
| T33528 | 501 | Anon-15Ab | 181 |
| T30411 | 502 | ATP-dependent RNA helicase SUV3, mitochondrial | 182 |
| T33531 | 501 | ATP binding cassette transporter | 183 |
| T30371 | 490 | Nuclear pore complex protein Nup107 | 184 |

| | | | |
|---|---|---|---|
| ^{∗∗} (+) significant stunting or mortality compared with water-treated control; (-) no significant stunting or mortality compared with water-treated control; NT = either (1) trigger was not tested, or (2) both of the following occurred: the sample did not provide significant stunting/mortality and the positive control did not provide significant stunting/mortality in that test. Positive control used in this assay was dsRNA with the sequence previously disclosed as SEQ ID NO. 880 in U. S. Patent No. 7,943,819. | | | |

Blunt-ended double-stranded RNA (dsRNA) triggers for each of the trigger sequences provided in Table are synthesized and tested in a corn rootworm infestation assay in maize plants grown from maize seeds contacted prior to germination with the individual dsRNA trigger as described above in Example 26 using *Diabrotica virgifera virgifera* larvae, wherein mortality or stunting of the larvae due to contact with or ingestion of the polynucleotide triggers is assayed. Triggers that are found to be effective in causing larval stunting or mortality or both are further tested.

It is anticipated that methods using a combination of certain polynucleotide triggers according to the present embodiments (*e*.*g*., the dsRNA triggers described herein) with one or more non-polynucleotide pesticidal agents will result in a synergetic improvement in prevention or control of insect infestations, when compared to the effect obtained with the polynucleotide triggers alone or the non-polynucleotide pesticidal agent alone. In one embodiment, maize plants having improved resistance to corn rootworm infestation are grown from seed having in their genome a recombinant DNA sequence encoding a non-polynucleotide pesticidal agent, wherein the seed are contacted prior to germination with an effective amount of a polynucleotide trigger. Bioassays such as the corn rootworm infestation assay described herein are useful for defining dose-responses for larval mortality or growth inhibition using combinations of the polynucleotide triggers of the present embodiments and one or more non-polynucleotide pesticidal agents (e.g., a patatin, a plant lectin, a phytoecdysteroid, a *Bacillus thuringiensis* insecticidal protein, a *Xenorhabdus* insecticidal protein, a Photorhabdus insecticidal protein, a *Bacillus laterosporous* insecticidal protein, and a *Bacillus sphearicus* insecticidal protein). One of skill in the art can test combinations of polynucleotide triggers and non-polynucleotide pesticidal agents in routine bioassays to identify combinations of bioactives that are synergistic and desirable for use in protecting plants from insect infestations.

### EXAMPLE 28: GENERATION OF dsRNA MOLECULES FOR SILENCING EF1α GENE OF S. LITTORALIS

dsRNA polynucleotide triggers derived from the *S. littoralis* EF1α gene were analyzed against the corn genome (*Zea mays* - taxid:4577, Figure 21) using the same BLAST parameters as described in Example 7. dsRNAs targeting *S. littoralis* EF1α and having homology to a corn gene are selected.

### EXAMPLE 29: ALTERED CORN EF1α EXPRESSION FOLLOWING SEED TREATMENT WITH S. LITTORALIS dsRNA (FOR REFERENCE ONLY)

Corn seeds (var. Vivani) were treated according to the protocol described in Example 1 with exogenous non-transcribable dsRNA trigger molecules (SEQ ID Nos. 131 and 132) derived from the *S. littoralis* EF1α gene sequence, with no pre- and posttreatment washes. A mixture of 25 µg/ml from each dsRNA was used. The dsRNA was diluted either with 0.1mM EDTA, or mixed with 40 µg/ml of PEG-modified carbon nanotubes. Treatment was performed by gently shaking the seeds in the solution for 4 hours in a dark growth chamber at 15 °C. After treatment, seeds were planted in soil and grown at about 25 °C with 16 hours photoperiod. The plants were watered with tap water as necessary. Seeds treated with 50 µg/ml dsRNA derived from GFP sequence were germinated and grown alongside the EF1α dsRNA treated plants as a control.

Total RNA was extracted from leaves of germinated seeds, 20 days post treatment. cDNA was prepared using oligo-dT primers and the expression level of corn EF1α mRNA was determined in treated and control plants by real-time PCR with SYBR Green (Quanta BioSciences). The house-keeping genes GPM120 and NFE101 were used as endogenous control genes to normalize for input amounts. Primers were designed so as to not amplify the dsRNA trigger and thus detect only corn-derived EF1α mRNA.

**Table 13: Primers Used for RT-PCR Analysis for Expression Level of EF1α.**

| Target Gene | Forward / Reverse | Primer Sequence | SEQ ID No. |
|---|---|---|---|
| EF1α | Forward | GCAACCACTCCCAAATACTC | 191 |
| EF1α | Reverse | CAGGGTTGTACCCAACTTTC | 192 |
| GPM120 | Forward | AGGCTTTCGCTGCGTGTT | 193 |
| GPM120 | Reverse | TGGCCCATCCAAACTCAGA | 194 |
| NFE101 | Forward | GCTCAAGTTCTTCGGATGAC | 195 |
| NFE101 | Reverse | ACTTCTTCCAGCAGACTAGC | 196 |

This analysis showed a significant (Wilcoxon rank-sum test, p-value<0.05) up-regulation of corn EF1α mRNA. The median expression level of EF1α in plants treated with *S. littoralis* dsRNA was 2.12 and 1.68-fold higher than in control plants treated with GFP dsRNA, with or without PEG-modified carbon nanotubes, respectively. See Figure 22A and B.

The plants treated with dsRNA/CNTP were analyzed again for EF1α expression level 48 days post treatment. This analysis showed an up-regulation of corn EF1α mRNA. The median expression level of EF1α in plants treated with *S. littoralis* dsRNA was 1.66-fold higher than in control plants treated with GFP dsRNA. See Figure 22C.

### EXAMPLE 30: CORN EF1α, BETA ACTIN, ATPase AND NADPH EXPRESSION FOLLOWING SEED TREATMENT WITH S. LITTORALIS dsRNAs (FOR REFERENCE ONLY)

Corn seeds were treated with exogenous non-transcribable dsRNA trigger molecules derived from *S. littoralis* genes according to the protocol described in Example 1. Seeds were washed with double distilled water (DDW) prior to treatment for four hours. Next, seeds were dried at 30°C overnight. Following the drying step, a final concentration of 132 µg/ml dsRNA for EF1α (a mixture of dsRNA#1 (SEQ ID No. 131) and dsRNA#2 (SEQ ID No. 132) at about equal concentrations), 53 µg/ml dsRNA for ATPase (SEQ ID No. 31), 76 µg/ml dsRNA for Beta actin (SEQ ID No. 133) and 154 µg/ml dsRNA for NADPH (SEQ ID Nos. 25 and 26), all diluted with 0.1mM EDTA, was used. Treatment was performed by gently shaking the seeds in the solution for 26 hours in a dark growth chamber at 15 °C. After treatment seeds were washed briefly with DDW and placed on wet paper for germination without a drying step. Seven days after germination the seedlings were planted in soil and grown at about 25 °C with 16 hours photoperiod. The plants were watered with tap water as necessary. Seeds that were treated with a similar solution (EDTA) not containing dsRNA were germinated and grown alongside the treated plants as a control.

Nine weeks after treatment, total RNA was extracted from leaves of germinated seeds. cDNA was prepared using oligo-dT and random primers and the expression level of corn EF1α, Beta actin, ATPase and NADPH was determined in treated and control plants. The numbers of plants analyzed were 3, 4, 3, 3 and 7 for EF1α, Beta actin, ATPase, NADPH and control respectively. The house-keeping gene FKBP was used as endogenous control gene to normalize for input amounts. Primers were designed so as to not amplify the dsRNA triggers and thus detect only corn-derived mRNAs.

**Table 14: Primers Used for RT-PCR Analysis for Expression Level of EF1α, BETA ACTIN, ATPase AND NADPH.**

| Target Gene | Forward / Reverse | Primer Sequence | SEQ ID No. |
|---|---|---|---|
| EF1α | Forward | GCAACCACTCCCAAATACTC | 197 |
| EF1α | Reverse | CAGGGTTGTACCCAACTTTC | 198 |
| Beta actin | Forward | TCTGGCATCACACCTTCTAC | 199 |
| Beta actin | Reverse | TTCTCACGGTTAGCCTTTGG | 200 |
| ATPase | Forward 1 | TGTCCTGCCATCTCTATCTC | 201 |
| ATPase | Reverse 1 | ACATCCGAATGGTCTCTACG | 202 |
| ATPase | Forward 2 | CACAACCGTGCAGTTTACAG | 203 |
| ATPase | Reverse 2 | AAATGCGCCCAAGCATATCG | 204 |
| NADPH | Forward | CAGAGGACGAGGAATATGAG | 205 |
| NADPH | Reverse | CTAGCAGCATTGTCAGTAGG | 206 |
| FKBP | Forward | CGGTGTTCGACAGCAGCTAC | 207 |
| FKBP | Reverse | CTTCGCCGCCAACAATACCC | 208 |

Due to the small group size, this analysis showed no significant difference in the expression of these genes (Wilcoxon rank-sum test, p-value>0.05), but the expression of EF1α showed an up-regulation trend. The median expression level of EF1α in plants treated with *S. littoralis* EF1α dsRNA was 2.28-fold higher than in control plants (Figure 23).

### EXAMPLE 31: CORN EF1α AND ATPase EXPRESSION FOLLOWING SEED TREATMENT WITH S. LITTORALIS dsRNAs (FOR REFERENCE ONLY)

Corn seeds were treated with exogenous non-transcribable dsRNA trigger molecules derived from the *S. littoralis* EF1α and ATPase genes according to the protocol described in Example 1. Seeds were washed with double distilled water (DDW) prior to treatment for four hours and dried at 30 °C overnight. Two EF1α dsRNA sequences (dsRNA#1 (SEQ ID No. 131) and #2 (SEQ ID No. 132)) were used separately in two different seed treatments; each at a final concentration of 67 µg/ml dsRNA. ATPase dsRNA (SEQ ID No. 31) was used at a final concentration of 145 µg/ml. All dsRNAs were diluted with 0.1mM EDTA. Treatment was performed by gently shaking the seeds in the solution for 24 hours in a dark growth chamber at 15 °C. After treatment, seeds were dried at 30 °C overnight and then planted in soil and grown at about 25 °C with 16 hours photoperiod. The plants were watered with tap water as necessary. Seeds that were treated with 67 µg/ml dsRNA (SEQ ID No. 20) derived from GUS sequence were germinated and grown alongside the treated plants as a control.

Seven days after treatment, total RNA was extracted from leaves of germinated seeds and the expression level of corn EF1α and ATPase was determined in treated and control plants as described in Example 2 above. The house-keeping gene GPM120 was used as endogenous control gene to normalize for input amounts. Primers were designed so as to not amplify the dsRNA triggers and thus detect only corn-derived mRNA.

**Table 15: Primers Used for RT-PCR Analysis for Expression Level of EF1α and ATPase.**

| Target Gene | Forward / Reverse | Primer Sequence | SEQ ID No. |
|---|---|---|---|
| EF1α | Forward | GCAACCACTCCCAAATACTC | 191 |
| EF1α | Reverse | CAGGGTTGTACCCAACTTTC | 192 |
| ATPase | Forward | GCGCAAGTTTTTCGTAGATGAC | 209 |
| ATPase | Reverse | ACCATAGTCCACAGATGACAC | 210 |
| GMP120 | Forward | GCTGCGTGTTGTGCGTTCTG | 211 |
| GMP120 | Reverse | TCGTCGCGTGCTGTCTGTTC | 212 |

| | | | |
|---|---|---|---|
| No significant difference in the expression of these genes was observed. | | | |

### EXAMPLE 32: CORN NADPH EXPRESSION FOLLOWING SEED TREATMENT WITH S. LITTORALIS dsRNA (FOR REFERENCE ONLY)

Corn seeds (var. 01DKD2) were treated according to the protocol described in Example 45 with exogenous non-transcribable dsRNA trigger molecules (SEQ ID No. 26) derived from the *S. littoralis* NADPH gene. A final concentration of 80 µg/ml dsRNA diluted with 0.1mM EDTA was used. Treatment was performed by gently shaking the seeds in the solution for 3.5 hours in a dark growth chamber at 15 °C. After treatment, seeds were planted in soil and grown at about 25 °C with 16 hours photoperiod. The plants were watered with tap water as necessary. Seeds that were treated with GFP dsRNA, or with a similar solution not containing dsRNA, were germinated and grown alongside the treated plants as a control.

20 days after treatment, total RNA was extracted from leaves of germinated seeds and the expression level of corn NADPH was determined in treated and control plants as described in Example 45 above. The house-keeping genes GPM120 and NFE101 were used as endogenous control genes to normalize for input amounts. Primers were designed so as to not amplify the dsRNA trigger and thus detect only corn-derived mRNA.

**Table 16: Primers Used for RT-PCR Analysis for Expression Level of NADPH.**

| Target Gene | Forward / Reverse | Primer Sequence | SEQ ID No. |
|---|---|---|---|
| NADPH | Forward | CAGAGGACGAGGAATATGAG | 205 |
| NADPH | Reverse | CTAGCAGCATTGTCAGTAGG | 206 |
| GPM120 | Forward | AGGCTTTCGCTGCGTGTT | 213 |
| GMP120 | Reverse | TGGCCCATCCAAACTCAGA | 214 |
| NFE101 | Forward | GCTCAAGTTCTTCGGATGAC | 215 |
| NFE101 | Reverse | ACTTCTTCCAGCAGACTAGC | 216 |

| | | | |
|---|---|---|---|
| No significant difference in the expression of NADPH was observed. | | | |

### EXAMPLE 33: CORN EF1α EXPRESSION FOLLOWING SEED TREATMENT WITH S. LITTORALIS dsRNAs (FOR REFERENCE ONLY)

Corn seeds (var. 01DKD2) were treated according to the protocol described in Example 29 with exogenous non-transcribable dsRNA trigger molecules (SEQ ID No. 131) derived from the *S. littoralis* EF1α gene. A mixture of 25 µg/ml from each of the two dsRNAs was used. The dsRNA was diluted either with 0.1mM EDTA alone, or additionally mixed with 40 µg/ml of PEG-modified carbon nanotubes (CNTP). Treatment was performed by gently shaking the seeds in the solution for 4 hours in a dark growth chamber at 15 °C. After treatment, seeds were planted in soil and grown at about 25 °C with 16 hours photoperiod. The plants were watered with tap water as necessary. Seeds that were treated with 50 µg/ml dsRNA derived from GUS sequence, with or without 40 µg/ml of PEG-modified carbon nanotubes, were germinated and grown alongside the treated plants as a control.

20 days after treatment, total RNA was extracted from leaves of germinated seeds and the expression level of corn EF1α was determined in treated and control plants as described in Example 29. The house-keeping genes GPM120, NFE101 and Expressed were used as endogenous control genes to normalize for input amounts. Primers were designed so as to not amplify the dsRNA trigger and thus detect only corn-derived mRNA.

**Table 17: Primers Used for RT-PCR Analysis for Expression Level of EF1α.**

| Target Gene | Forward / Reverse | Primer Sequence | SEQ ID No. |
|---|---|---|---|
| EF1α | Forward | GCAACCACTCCCAAATACTC | 198 |
| EF1α | Reverse | CAGGGTTGTACCCAACTTTC | 199 |
| GPM120 | Forward | AGGCTTTCGCTGCGTGTT | 193 |
| GMP120 | Reverse | TGGCCCATCCAAACTCAGA | 194 |
| NFE101 | Forward | GCTCAAGTTCTTCGGATGAC | 215 |
| Expressed | Forward | GGATGCTACTCGCCAGACA | 217 |
| Expressed | Reverse | GTGGTCAGCCTGCTTCAAC | 218 |

| | | | |
|---|---|---|---|
| No significant difference in the expression of EF1α was observed. | | | |

### EXAMPLE 34: CORN EF1α EXPRESSION FOLLOWING SEED TREATMENT WITH S. LITTORALIS dsRNAs (FOR REFERENCE ONLY)

Corn seeds (var. Vivani) were treated according to the protocol described in Example 1, with exogenous non-transcribable dsRNA trigger molecules (SEQ ID Nos. 131 and 132) derived from the *S. littoralis* EF1α gene, without pre-treatment wash. A mixture of 25 µg/ml from each of the two dsRNAs was used. The dsRNA was diluted either with 0.1mM EDTA alone, or additionally mixed with 40 µg/ml of PEG-modified carbon nanotubes (CNTP). Treatment was performed by gently shaking the seeds in the solution for 4 hours in a dark growth chamber at 15 °C. After treatment seeds were washed briefly with DDW and directly germinated in soil without a drying step. Plants were grown at about 25 °C with 16 hours photoperiod and watered with tap water as necessary. Seeds that were treated with a similar solution not containing dsRNA, or with 50 µg/ml dsRNA derived from GFP sequence, with or without 40 µg/ml of PEG-modified carbon nanotubes, were germinated and grown alongside the treated plants as a control.

14 days after treatment, total RNA was extracted from leaves of germinated seeds and the expression level of corn EF1α was determined in treated and control plants as described in Example 29 above. The house-keeping genes NFE101 and Expressed were used as endogenous control genes to normalize for input amounts. Primers were designed so as to not amplify the dsRNA trigger and thus detect only corn-derived mRNA.

**Table 18: Primers Used for RT-PCR Analysis for Expression Level of EF1α.**

| Target Gene | Forward / Reverse | Primer Sequence | SEQ ID No. |
|---|---|---|---|
| EF1α | Forward | GCAACCACTCCCAAATACTC | 197 |
| EF1α | Reverse | CAGGGTTGTACCCAACTTTC | 198 |
| NFE101 | Forward | GCTCAAGTTCTTCGGATGAC | 215 |
| Expressed | Forward | GGATGCTACTCGCCAGACA | 217 |
| Expressed | Reverse | GTGGTCAGCCTGCTTCAAC | 218 |

| | | | |
|---|---|---|---|
| No significant difference in the expression of EF1α was observed. | | | |

### EXAMPLE 35: CORN EF1α EXPRESSION FOLLOWING SEED TREATMENT WITH S. LITTORALIS dsRNAs (FOR REFERENCE ONLY)

Corn seeds (var. Vivani) were treated according to the protocol described in Example 1, with exogenous non-transcribable dsRNA trigger molecules (SEQ ID Nos. 131 and 132) derived from the *S. littoralis* EF1α gene, without pre-treatment wash. The two dsRNAs were used separately, each at a final concentration of 160 µg/ml. The dsRNAs were diluted either with IDT buffer alone (30 mM HEPES, pH 7.5, 100 mM Potassium Acetate), or additionally mixed with 40 µg/ml of PEG-modified carbon nanotubes (CNTP). Treatment was performed by gently shaking the seeds in the solution for 7 hours in a dark growth chamber at 25 °C. After treatment, seeds were washed briefly with DDW and directly germinated in soil without a drying step. Plants were grown at about 25 °C with 16 hours photoperiod and watered with tap water as necessary. Seeds that were treated with a similar solution not containing dsRNA, or with 160 µg/ml dsRNA derived from GFP sequence, with or without 40 µg/ml of PEG-modified carbon nanotubes, were germinated and grown alongside the treated plants as a control.

Six days after treatment, total RNA was extracted from leaves of germinated seeds and the expression level of corn EF1α was determined in treated and control plants as described in Example 29 above. The house-keeping genes GPM120 and Expressed were used as endogenous control genes to normalize for input amounts. Primers were designed so as to not amplify the dsRNA trigger and thus detect only corn-derived mRNA.

**Table 19: Primers Used for RT-PCR Analysis for Expression Level of EF1α.**

| Target Gene | Forward / Reverse | Primer Sequence | SEQ ID No. |
|---|---|---|---|
| EF1α | Forward 1 | GCAACCACTCCCAAATACTC | 197 |
| EF1α | Reverse 1 | CAGGGTTGTACCCAACTTTC | 198 |
| EF1α | Forward 2 | CCCAGGTCATCATCATGAAC | 191 |
| EF1α | Reverse 2 | GAGCTCAGCAAACTTGACAG | 192 |
| GPM120 | Forward | GCTGCGTGTTGTGCGTTCTG | 211 |
| Expressed | Forward | GGATGCTACTCGCCAGACA | 217 |
| Expressed | Reverse | GTGGTCAGCCTGCTTCAAC | 218 |

The results of this analysis are shown in Figure 24. A significant up-regulation of corn EF1α mRNA was observed in plants following treatment with EF1α dsRNA #1 (t-test, p-value=0.004). The average expression level of EF1α in plants treated with this dsRNA was 1.8 higher than in control plants treated with GUS dsRNA. When grouping all the plants treated with EF1α dsRNAs (both dsRNA #1 and #2, with and without CNTP) and comparing to all plants treated with GUS dsRNA (with and without CNTP) a significant up-regulation of corn EF1α mRNA was observed. See Figure 24B. The average expression level of EF1α in plants treated with EF1α dsRNAs was 1.73 higher than in control plants treated with GUS dsRNA (t-test, p-value=0.005).

### EXAMPLE 36: CORN ATPase AND NADPH EXPRESSION FOLLOWING SEED TREATMENT WITH S. LITTORALIS dsRNAs (FOR REFERENCE ONLY)

Corn seeds (var. Vivani) were treated according to the protocol described in Example 1 with exogenous non-transcribable dsRNA trigger molecules (SEQ ID Nos. 31 and 26) derived from the *S. littoralis* ATPase and NADPH genes, without pre-treatment wash. A final concentration of 160 µg/ml dsRNA, diluted with 0.1mM EDTA, was used. Treatment was performed by gently shaking the seeds in the solution for 2 hours in a dark growth chamber at 15°C. After treatment, seeds were washed briefly with DDW, planted in soil and grown at about 25 °C with 16 hours photoperiod. The plants were watered with tap water as necessary. Seeds that were treated with 160 µg/ml dsRNA (SEQ ID No. 124) derived from GFP sequence, or with a similar solution not containing dsRNA (EDTA) were germinated and grown alongside the treated plants as a control.

27 days after treatment, total RNA was extracted from leaves of germinated seeds and the expression levels of corn ATPase and NADPH were determined in treated and control plants as described in Example 29 above. The house-keeping gene, Expressed, was used as endogenous control genes to normalize for input amounts. Primers were designed so as to not amplify the dsRNA trigger and thus detect only corn-derived mRNA.

**Table 20: Primers Used for RT-PCR Analysis for Expression Level of ATPase and NADPH.**

| Target Gene | Forward / Reverse | Primer Sequence | SEQ ID No. |
|---|---|---|---|
| ATPase | Forward | GCGCAAGTTTTTCGTAGATGAC | 219 |
| ATPase | Reverse | ACCATAGTCCACAGATGACAC | 220 |
| NADPH | Forward | CAGAGGACGAGGAATATGAG | 205 |
| NADPH | Reverse | CTAGCAGCATTGTCAGTAGG | 206 |
| Expressed | Forward | GGATGCTACTCGCCAGACA | 217 |
| Expressed | Reverse | GTGGTCAGCCTGCTTCAAC | 218 |

The results of this analysis are shown in Figure 25A (ATPase expression) and 25B and C (NADPH expression). No difference in corn ATPase expression levels was detected following treatment with *S. littoralis* ATPase dsRNA. However, a trend of down-regulation of corn NADPH mRNA was observed in plants following treatment with NADPH dsRNA triggers. The average expression level of NADPH in plants treated with this dsRNA trigger was 1.37 fold lower than in control plants treated with GFP dsRNA trigger (t-test, p-value=0.11). When grouping all control plants (those treated with GFP dsRNA and those treated with EDTA) and comparing to plants treated with NADPH dsRNA trigger, a significant down-regulation of corn NADPH mRNA was observed, with an average decrease of 1.67 fold in NADPH expression levels following treatment with NADPH dsRNAs (t-test, p-value=0.02).

## Claims

1. A method of treating a seed to improve insect resistance of a plant grown from the seed, the method comprising:
(a) priming the seed prior to introducing a naked exogenous double-stranded RNA (dsRNA) into the seed, wherein said priming is effected by:
(i) washing the seed prior to said contacting; and
(ii) drying the seed following step (i);
(b) wherein said introducing is effected by directly contacting said seed with said naked exogenous dsRNA, wherein said naked exogenous dsRNA molecule comprises at least one polynucleotide strand comprising at least one segment of 18 or more contiguous nucleotides of an insect pest gene in either anti-sense or sense orientation, wherein said contacting is effected by soaking the seed in a solution comprising a final concentration of 0.005 µg/µL to 1.5 µg/µL of said naked exogenous dsRNA and shaking the seed in the solution for 4 to 24 hours, wherein the dsRNA molecule is introduced into the seed but is not expressed from the plant genome, thereby not being an integral part of the genome; and wherein the plant grown from the seed exhibits improved insect resistance relative to a control plant, wherein the control plant is grown from a seed not contacted with the exogenous dsRNA molecule.

2. The method of Claim 1, wherein the insect pest gene is selected from the group consisting of ATPase, NADPH Cytochrome P450 Oxidoreductase, IAP, Chitin Synthase, EF1α, and β-actin.

3. The method of any one of Claims 1-2, wherein the plant is resistant to *Spodoptera littoralis, Diabrotica virgifera virgifera* or *Leptinotarsa decemlineata* infestation.

4. The method of any one of claims 1-3, wherein said washing is effected for 2 to 6 hours.

5. The method of any one of claims 1-4, wherein said washing is effected at 4°C to 28°C.

6. The method of claim 1, wherein said drying is effected at 25°C to 30°C for 10 to 16 hours.

7. The method of claim 1, wherein said washing is effected in the presence of double deionized water.

## Patentansprüche

1. Verfahren zur Behandlung eines Samens zur Verbesserung der Insektenresistenz einer aus dem Samen gezogenen Pflanze, wobei das Verfahren Folgendes umfasst: (a) Vorbereiten des Samens vor Einführen einer nackten exogenen Doppelstrang-RNA (dsRNA) in den Samen, wobei das Vorbereiten bewirkt wird durch:
(i) Waschen des Samens vor dem In-Kontakt-Bringen; und
(ii) Trocknen des Samens im Anschluss an Schritt (i);
wobei das Einführen durch direktes In-Kontakt-Bringen des Samens mit der nackten exogenen dsRNA bewirkt wird, wobei das nackte exogene dsRNA-Molekül wenigstens einen Polynukleotidstrang umfasst, der wenigstens ein Segment von 18 oder mehr zusammenhängenden Nukleotiden eines Insektenschädling-Gens in entweder Antisense oder Sense-Orientierung umfasst, wobei das In-Kontakt-Bringen durch Einweichen des Samens in einer eine Endkonzentration von 0,005 µg/µl bis 1,5 µg/µl der nackten exogenen dsRNA umfassenden Lösung und 4 bis 24 Stunden Schütteln des Samens in der Lösung bewirkt wird, wobei das dsRNA-Molekül in den Samen eingeführt, aber nicht vom Pflanzengenom exprimiert wird, womit es kein integraler Teil des Genoms ist; und wobei die aus dem Samen gezogene Pflanze verbesserte Insektenresistenz relativ zu einer Kontrollpflanze zeigt, wobei die Kontrollpflanze aus einem nicht mit dem exogenen dsRNA-Molekül in Kontakt gebrachten Samen gezogen wird.

2. Verfahren nach Anspruch 1, wobei das Insektenschädling-Gen ausgewählt ist aus der Gruppe bestehend aus ATPase, NADPH-Cytochrom-P450-Oxidoreduktase, IAP, Chitin-Synthase, EF1α und β-Actin.

3. Verfahren nach einem der Ansprüche 1-2, wobei die Pflanze resistent gegen Befall mit *Spodoptera littoralis, Diabrotica virgifera virgifera* oder *Leptinotarsa decemlineata* ist.

4. Verfahren nach einem der Ansprüche 1-3, wobei das Waschen über 2 bis 6 Stunden erfolgt.

5. Verfahren nach einem der Ansprüche 1-4, wobei das Waschen bei 4°C bis 28°C erfolgt.

6. Verfahren nach Anspruch 1, wobei das Trocknen bei 25°C bis 30°C über 10 bis 16 Stunden erfolgt.

7. Verfahren nach Anspruch 1, wobei das Waschen in Gegenwart von doppelt deionisiertem Wasser erfolgt.

## Revendications

1. Procédé de traitement d'une graine pour améliorer la résistance aux insectes d'un végétal ayant poussé à partir de la graine, le procédé comprenant :
(a) l'amorçage de la graine avant l'introduction d'un ARN double brin (ARNds) exogène nu dans la graine, ledit amorçage étant effectué par :
(i) lavage de la graine avant ladite mise en contact ; et
(ii) séchage de la graine à la suite de l'étape (i) ;
(b) ladite introduction étant effectuée par mise en contact de manière directe de ladite graine avec ledit ARNds exogène nu, ladite molécule d'ARNds exogène nu comprenant au moins un brin de polynucléotide comprenant au moins un segment de 18 nucléotides contigus ou plus d'un gène d'organisme nuisible de type insectes dans l'orientation soit antisens, soit sens, ladite mise en contact étant effectuée par trempage de la graine dans une solution comprenant une concentration finale de 0,005 pg/pL à 1,5 pg/pL dudit ARNds exogène nu et agitation de la graine dans la solution pendant 4 à 24 heures, la molécule d'ARNds étant introduite dans la graine mais n'étant pas exprimée par le génome du végétal, n'étant ainsi pas une partie intégrante du génome ; et le végétal ayant poussé à partir de la graine présentant une résistance aux insectes améliorée par rapport à un végétal témoin, le végétal témoin ayant été fait pousser à partir d'une graine non mise en contact avec la molécule d'ARNds exogène.

2. Procédé selon la revendication 1, le gène d'organisme nuisible de type insectes étant choisi dans le groupe constitué par ATPase, NADPH cytochrome P450 oxydoréductase, IAP, chitine synthase, EF1α et β-actine.

3. Procédé selon l'une quelconque des revendications 1 et 2, le végétal étant résistant à une infestation par *Spodoptera littoralis, Diabrotica virgifera virgifera* ou *Leptinotarsa decemlineata.*

4. Procédé selon l'une quelconque des revendications 1 à 3, ledit lavage étant effectué pendant 2 à 6 heures.

5. Procédé selon l'une quelconque des revendications 1 à 4, ledit lavage étant effectué à une température de 4 °C à 28 °C.

6. Procédé selon la revendication 1, ledit séchage étant effectué à une température de 25 °C à 30 °C pendant 10 à 16 heures.

7. Procédé selon la revendication 1, ledit lavage étant effectué en la présence d'eau doublement désionisée.
